# EUROPEAN PATENT SPECIFICATION

(11) **EP 3 880 216 B1**
(45) Date of publication and mention of the grant of the patent: **09.04.2025**
(21) Application number: 19884558.8
(22) Date of filing: 15.11.2019
(51) Int. Cl.: A61K 35/17, C12N 5/00

(54) **METHODS FOR TREATING CANCER WITH MANUFACTURED T CELLS**
VERFAHREN ZUR BEHANDLUNG VON KREBS MIT HERGESTELLTEN T-ZELLEN
MÉTHODES DE TRAITEMENT D'UN CANCER AVEC DES LYMPHOCYTES T FABRIQUÉS

(30) Priority: 16.11.2018 US 201862768145 P; 28.10.2019 US 201962927034 P; 28.10.2019 US 201962927079 P
(43) Date of publication of application: 22.09.2021
(73) Proprietor: Rapa Therapeutics, LLC, Rockville, MD 20852 (US)
(72) Inventor: FOWLER, Daniel, Harding, Bethesda, MD 20814 (US)
(74) Representative: Gill Jennings & Every LLP
(86) International application number: PCT/US2019/061777
(87) International publication number: WO 2020/102708

(56) References cited:
- EP-A1- 2 532 740
- WO-A1-2017/177137
- WO-A1-2018/085690
- WO-A1-2018/106958
- WO-A1-2018/106958
- WO-A2-2010/151517
- WO-A2-2016/090034
- US-A1- 2012 114 623
- US-A1- 2017 274 058
- US-A1- 2017 356 917
- STANZANI MARTA ET AL: "CD25 expression on donor CD4+ or CD8+ T cells is associated with an increased risk for graft-versus-host disease after HLA-identical stem cell transplantation in humans", BLOOD, vol. 103, no. 3, 1 February 2004 (2004-02-01), US, pages 1140 - 1146, XP093046776, ISSN: 0006-4971, Retrieved from the Internet <URL:http://ashpublications.org/blood/article-pdf/103/3/1140/1694552/zh800304001140.pdf> DOI: 10.1182/blood-2003-06-2085
- CHATTOPADHYAY SUBHASIS ET AL: "Effect of CD4+CD25+ and CD4+CD25- T Regulatory Cells on the Generation of Cytolytic T Cell Response to a Self but Human Tumor-Associated Epitope In Vitro", THE JOURNAL OF IMMUNOLOGY, vol. 176, no. 2, 15 January 2006 (2006-01-15), US, pages 984 - 990, XP093046779, ISSN: 0022-1767, Retrieved from the Internet <URL:https://journals.aai.org/jimmunol/article-pdf/176/2/984/1214356/984.pdf> DOI: 10.4049/jimmunol.176.2.984
- JACOBSOHN ET AL.: "Phase II study of pentostatin in patients with corticosteroid-refractory chronic graft-versus-host disease", JOURNAL OF CLINICAL ONCOLOGY, vol. 25, no. 27, 20 September 2007 (2007-09-20), pages 4255 - 4261, XP055707857

## Description

### BACKGROUND

CD4⁺ and CD8⁺ T cells of type I cytokine profile (Th1 and Tc1 cells, respectively) are candidate T cell populations for adoptive T cell therapy efforts. This Th1-type T cell is promoted by polarizing cytokines such as IL-12 and IFN-α, which stimulate STAT1 and STAT4 transcription factors, which in-turn promote TBET transcription factor that defines in part Th1-type differentiation status.

The success of adoptive T cell therapy is dependent in-part upon the in vivo persistence of the T cell population in the host. T cell persistence is a balance determined by both an increase in T cell ability to proliferate and maintain T cell memory and by reduction in T cell propensity to apoptotic cell death. In previous research, it has been demonstrated that ex vivo manufacture of T cells in the pharmacologic agent rapamycin (which inhibits the mammalian target of rapamycin, mTOR) yielded T cells that manifested these characteristics, namely: an increased ability to undergo antigen-driven clonal expansion in vivo after adoptive transfer; an improved memory status, as exemplified by a T central memory (T_{CM}) differentiation state; and a multifaceted anti-apoptotic phenotype characterized by induction of autophagy, including mitochondrial autophagy, and by preferential expression of anti-apoptotic members of the bcl-2 family of genes relative to the pro-apoptotic members. Taken together, these properties of the ex vivo manufactured, rapamycin-resistant cells was associated with enhanced in vivo modulation of transplantation responses, including the prevention of graft-versus-host disease (GVHD) and graft rejection and the mediation of human-into-mouse xenogeneic GVHD; of note, these cells were successfully translated into clinical trials in the autologous and allogeneic settings for the treatment of multiple myeloma and were shown to be safe and effective for relapsed, refractory multiple myeloma. For these clinical trial efforts, the manufacturing process included the following elements: co-stimulation with anti-CD3, anti-CD28 coated magnetic beads (3/28 beads) at the relatively high ratio of 3 beads: 1 T cell; simultaneous addition of T cells and 3/28 beads to ex vivo culture; addition of a high-dose of the orally-administered mTOR inhibitor rapamycin (1 µM); and use of IL-2 addition to culture during cytokine polarization (IFN-α addition). The cells produced by this method are referred to herein as T-Rapa cells which are more specifically defined later in the present disclosure.

Cancer development can be associated with alterations in immune functions. Such alterations include suppressed cell mediated immunity (CMI) associated with failure to reject tumors, as well as enhanced humoral immunity that can potentiate tumor promotion and progression. CD4⁺ T cell subsets, Th1 and Th2 T cells, have distinctive function and regulate each other. Th1 cells produce interleukin (IL-2) and interferon (IFN-γ) and direct CMI responses, whereas Th2 cells produce IL-4 and IL-10, and facilitate local humoral immune responses.

There is evidence of Th1/Th2 imbalance in certain cancers, where the proportion of Th2 cells is significantly elevated at the expense of Th1 cell number. Chronic Th1/Th2 imbalance in favor of Th2 potentially leads to suppressed cell mediated immunity, thereby providing a propitious environment for decreased effective immuno-surveillance and development of malignancy.

Idiotype specific T cell responses are found in most patients with early stage multiple myeloma. These include Th1 responses with IL-2 and IFN-γ production. For example, Th1-type immunity is found preferentially in cases of indolent disease and Th2-type responses predominate in cases of advanced multiple myeloma. Defective Th1 immune responses (mediated by IL-6) as well as dysregulated cytokine network are found in multiple myeloma patients. Myeloma idiotype-specific T helper cells derived from MM patients are consistently of non-Th1 phenotype.

Despite advances in the treatment of multiple myeloma and the recent FDA-approval of new pharmaceutical agents and monoclonal antibodies, multiple myeloma is nearly universally fatal. As such, patients with relapsed, refractory multiple myeloma (RRMM) who are refractory to the top five drugs against multiple myeloma ("penta-refractory") have limited survival of a few months and few therapeutic options. Multiple myeloma is a disease that is susceptible to immune therapy, as evidenced by the long-observed curative role of allogeneic stem cell transplantation and by numerous other approaches, including monoclonal antibody therapy, vaccines, and T cell receptor (TCR-) and CAR-modified T cell therapy. As such, this penta-refractory patient population is suitable for a novel T cell therapy. In addition, patients with less refractory disease are also in great need of novel therapy; that is, even at the second or third relapse of disease, the median progression-free survival is typically less than two years.

For certain cancers, there is a need for novel and innovative immune therapy approaches.

EP2532740 A1 (12 December 2012) discloses antigen-specific CD4+ and CD8+ central memory T cell preparations for adoptive T cell therapy. WO 2018/106958 A1 (14 June 2018) discloses methods and compositions for vaccinating and boosting cancer patients. WO 2010/151517 A2 (29 December 2010) discloses antigen specific long-term memory T cells. Stanzani Marta et al., (Blood, vol.103, no.3, 1 February 2004, pages 1140-1146) discloses CD25 expression on donor CD4+ or CD8+ T cells being associated with an increased risk for graft-versus-host disease after HLA-identical stem cell transplantation in humans. Chattopadhyay Subhasis et al., (The Journal of Immunology, vol. 176, no.2, 15 January 2006, pages 984-990) discloses the effect of CD4+CD25+ and CD4+CD25- T regulatory cells on the generation of cytolytic T cell responses to a self but human tumor-associated epitope *in vitro.*

### SUMMARY

The present invention discloses a method for producing manufactured T cells, comprising: inoculating a culture input population of cells comprising T cells from a subject in a culture medium comprising temsirolimus and an IL-2 signaling inhibitor, wherein said culture medium does not contain IL-2 and no IL-2 is added to said culture medium and wherein said temsirolimus is present in said culture medium at a concentration of at least 1 µM, and wherein said IL-2 signaling inhibitor is an anti-IL-2 receptor antibody or fragment thereof; adding IFN-α to said culture medium; incubating said T cells and culture medium to yield manufactured T cells; and harvesting said manufactured T cells.

Further embodiments of the present invention are set out in the appended claims.

### BRIEF DESCRIPTION OF THE DRAWINGS

FIGURE 1A depicts the percentage of CD4⁺ T cells expressing FoxP3 at day 0 and after various culture conditions.
FIGURE 1B depicts the percentage of CD4⁺ T cells expressing TBET at day 0 and after various culture conditions.
FIGURE 2A depicts T cell yield after culturing CD4⁺ and CD8⁺ T cells under various conditions.
FIGURE 2B depicts T cell yield after culturing CD4⁺ and CD8⁺ T cells under various conditions.
FIGURE 3A depicts IFN-gamma secretion after culturing CD4⁺ and CD8⁺ T cells under various conditions.
FIGURE 3B depicts TNF-alpha secretion after culturing CD4⁺ and CD8⁺ T cells under various conditions.
FIGURE 4 depicts a Western blot of p-4EBP1 and actin proteins from CD4⁺ and CD8⁺ T cells cultured using T-Rapa methodology and a method of the present disclosure (Rapa-T) (top panel). The p-4EBP1 level was normalized by actin expression in in CD4⁺ and CD8⁺ T cells cultured using T-Rapa methodology and a method of the present disclosure (Rapa-T) (bottom panel).
FIGURE 5 depicts a Western blot of P70S6K and actin proteins from CD4⁺ and CD8⁺ T cells cultured using T-Rapa methodology and a method of the present disclosure (Rapa-T) (top panel) and P70S6K level normalized by actin expression in CD4⁺ and CD8⁺ T cells cultured using T-Rapa methodology and a method of the present disclosure (bottom panel).
FIGURE 6 depicts a Western blot of P-STAT5 and actin proteins from CD4⁺ and CD8⁺ T cells cultured using T-Rapa methodology and a method of the present disclosure (Rapa-T) (top panel) and P-STAT5 level normalized by actin expression in CD4⁺ and CD8⁺ T cells cultured using T-Rapa methodology and a method of the present disclosure (bottom panel).
FIGURE 7A depicts day 6 IFN-gamma secretion after culturing CD4⁺ and CD8⁺ T cells under various conditions.
FIGURE 7B depicts day 13 IFN-gamma secretion after culturing CD4⁺ and CD8⁺ T cells under various conditions.
FIGURE 8A depicts day 6 TNF-alpha secretion after culturing CD4⁺ and CD8⁺ T cells under various conditions.
FIGURE 8B depicts day 13 TNF-alpha secretion after culturing CD4⁺ and CD8⁺ T cells under various conditions.
FIGURE 9A depicts day 6 GM-CSF secretion after culturing CD4⁺ and CD8⁺ T cells under various conditions.
FIGURE 9B depicts day 13 GM-CSF secretion after culturing CD4⁺ and CD8⁺ T cells under various conditions.
FIGURE 10A depicts day 6 IL-2 secretion after culturing CD4⁺ and CD8⁺ T cells under various conditions.
FIGURE 10B depicts day 13 IL-2 secretion after culturing CD4⁺ and CD8⁺ T cells under various conditions.
FIGURE 11 depicts a Western blot of P62 and actin proteins from CD4⁺ and CD8⁺ T cells cultured under various conditions (top panel) and P62 protein expression normalized by actin expression in CD4⁺ and CD8⁺ T cells under various conditions (bottom panel).
FIGURE 12 depicts a Western blot of p-RAPTOR and actin proteins from CD4⁺ and CD8⁺ T cells cultured under various conditions (top panel) and p-RAPTOR level normalized by actin expression in CD4⁺ and CD8⁺ T cells cultured under various conditions (bottom panel).
FIGURE 13 depicts a Western blot of BIM and actin proteins from CD4⁺ and CD8⁺ T cells cultured under various conditions (top panel) and BIM protein expression normalized by actin expression in CD4⁺ and CD8⁺ T cells cultured under various conditions (bottom panel).
FIGURE 14A depicts flow cytometry expression analysis of CD45RA on the CD4+ cell subset after culturing the T cells under various conditions.
FIGURE 14B depicts flow cytometry expression analysis of CD45RA on the CD4+ cell subset after culturing the T cells under various conditions.
FIGURE 14C depicts flow cytometry expression analysis of CD45RA on the CD4+ cell subset after culturing the T cells under various conditions.
FIGURE 14D depicts flow cytometry expression analysis of CD45RA on the CD4⁺ cell subset after culturing the T cells under various conditions.
FIGURE 15A depicts flow cytometry expression analysis of CD62L, CCR7, and CD127 on the CD4+ cell subset after culturing the T cells under various conditions.
FIGURE 15B depicts flow cytometry expression analysis of CD62L, CCR7, and CD127 on the CD4+ cell subset after culturing the T cells under various conditions
FIGURE 15C depicts flow cytometry expression analysis of CD62L, CCR7, and CD127 on the CD4+ cell subset after culturing the T cells under various conditions
FIGURE 15D depicts flow cytometry expression analysis of CD62L, CCR7, and CD127 on the CD4+ cell subset after culturing the T cells under various conditions
FIGURE 16 depicts fold increase in culture yield of T cells cultured under various conditions.
FIGURE 17A depicts IFN-gamma secretion on day 6 after culturing T cells under various conditions.
FIGURE 17B depicts IFN-gamma secretion on day 13 after culturing T cells under various conditions.
FIGURE 18A depicts TNF-alpha secretion on day 6 after culturing T cells under various conditions.
FIGURE 18B depicts TNF-alpha secretion on day 13 after culturing T cells under various conditions.
FIGURE 19 depicts flow cytometry expression analysis of CD25 on the CD4⁺ T cell subset on day 6 and day 13 after culturing T cells under various conditions.
FIGURE 20 depicts flow cytometry expression analysis of CD62L, CCR7, and CD127 on the CD4⁺ T cell subset on day 6 and day 13 after culturing T cells under various conditions.
FIGURE 21 depicts flow cytometry expression analysis comparing the new Rapa-T method that incorporates no bead co-stimulation, the new Rapa-T method that incorporates bead co-stimulation (bead-to-T cell ratio, 1:3), and the old T-Rapa method (bead-to-T cell ratio, 3:1) for the naïve and T central memory panels: CD45RA expression; co-expression of CD62L and CCR7; and co-expression of CD62L, CCR7, and CD127.
FIGURE 22 depicts flow cytometry expression analysis comparing the new Rapa-T method that incorporates no bead co-stimulation, the new Rapa-T method that incorporates bead co-stimulation (bead-to-T cell ratio, 1:3), and the old T-Rapa method (bead-to-T cell ratio, 3:1) for: expression of the IL-2 receptor, CD25; and for expression of the immune suppressive molecules CTLA4 and TIM3.
FIGURE 23 depicts cytokine secretion results at the end of manufacturing and after an additional 6 days in culture without use of inhibitors comparing the new Rapa-T method that incorporates no bead co-stimulation, the new Rapa-T method that incorporates bead co-stimulation (bead-to-T cell ratio, 1:3), and the old T-Rapa method (bead-to-T cell ratio, 3:1) for: secretion of the type II cytokine IL-4; and secretion of the type I cytokine IFN-γ.
FIGURE 24 depicts type I cytokine secretion (IL-2 and IFN-y) results at the end of manufacturing and after an additional 6 days in culture without inhibitors in n=11 various culture conditions to further identify the role of no bead co-stimulation in the manufacture of the new Rapa-T cell population.
FIGURE 25A depicts flow cytometry data measurements for CD45RA+ for T cells under various culture conditions.
FIGURE 25B depicts flow cytometry data measurements for CD25+ for T cells under various culture conditions.
FIGURE 25C depicts flow cytometry data measurements for CD28+ for T cells under various culture conditions.
FIGURE 25D depicts flow cytometry data measurements for ICOS+ for T cells under various culture conditions.
FIGURE 25E depicts flow cytometry data measurements for CD39+ for T cells under various culture conditions.
FIGURE 25F depicts flow cytometry data measurements for CD73+ for T cells under various culture conditions.
FIGURE 25G depicts flow cytometry data measurements for GITR+ for T cells under various culture conditions.
FIGURE 25H depicts flow cytometry data measurements for LAG3+ for T cells under various culture conditions.
FIGURE 25I depicts flow cytometry data measurements for PD1+ for T cells under various culture conditions.
FIGURE 25J depicts flow cytometry data measurements for 2B4+ for T cells under various culture conditions.
FIGURE 25K depicts flow cytometry data measurements for LAIR1+ for T cells under various culture conditions.
FIGURE 25L depicts flow cytometry data measurements for CTLA4+ for T cells under various culture conditions.
FIGURE 25M depicts flow cytometry data measurements for KLRG1+ for T cells under various culture conditions.
FIGURE 25N depicts flow cytometry data measurements for TIGIT+ for T cells under various culture conditions.
FIGURE 25O depicts flow cytometry data measurements for TIM3+ for T cells under various culture conditions.
FIGURE 26 depicts Western Blot results for p-STAT5, p-STAT1, STAT1, p70S6K, p-SGK1, SGK1, Raptor, Rictor, Cytochrome C and actin for cells under various culture conditions.
FIGURE 27A depicts IL-2 secretion measurements for RAPA-T cells and T-RAPA cells from 24 hour supernatants after co-stimulation with anti-CD3/anti-CD28 coated magnetic beads and exposure to different cytokines.
FIGURE 27B depicts TNF-α secretion measurements for RAPA-T cells and T-RAPA cells from 24 hour supernatants after co-stimulation with anti-CD3/anti-CD28 coated magnetic beads and exposure to different cytokines.
FIGURE 28 depicts IL-2, TNF-α and IL-13 secretion measurements for RAPA-T cells after co-stimulation with anti-CD3/anti-CD28 coated beads or dissolvable anti-CD3/anti-CD28 microparticles.
FIGURE 29 depicts CD4, CD8, CD25 and CTLA4 expression as measured by flow cytometry for RAPA-T cells after co-stimulation with anti-CD3/anti-CD28 coated beads or dissolvable anti-CD3/anti-CD28 microparticles.
FIGURE 30 depicts a manufactured T cell therapy schema.
FIGURE 31 depicts a Pentostatin/Cyclophosphamide regimen followed by manufactured T cell infusion.
FIGURES 32A-32C details the nature of the control arm, that is, subjects that are not randomized to the Rapa-T cell therapy will receive one of three FDA-approved triplet regimens suitable for subjects with MM in the second or third relapse, namely: the DPd regimen (A); the DRd regimen (B); or the KRd regimen (C).
FIGURE 33 depicts an exemplary workflow for generating manufactured T cells of the present disclosure.
FIGURE 34A depicts cytokine secretion by RAPA-T cells after exposure to pancreatic cancer cells.
FIGURE 34B depicts cytokine secretion by RAPA-T cells after exposure to lung cancer cells.
FIGURE 35 depicts cytokine secretion by RAPA-T cells with or without exposure to pancreatic or lung cancer cells.
FIGURE 36 depicts a correlation between checkpoint inhibitor therapy response and the number of tumor mutations in cancer.

### DETAILED DESCRIPTION

The present disclosure provides methods for producing manufactured T cells, manufactured T cells produced by the methods disclosed herein, populations and compositions comprising a population of manufactured T cells and method for treating cancer in a subject using said manufactured T cells or populations of manufactured T cells.

### Definitions

The following definitions are provided:
As used herein, the singular forms "a", "an" and "the" include plural referents unless the context clearly dictates otherwise. The use of the term "or" in the claims and the present disclosure is used to mean "and/or" unless explicitly indicated to refer to alternatives only or the alternatives are mutually exclusive.

Use of the term "about", when used with a numerical value, is intended to include +/-10%. By way of example but not limitation, if a number of amino acids is identified as about 200, this would include 180 to 220 (plus or minus 10%).

The terms "subject," "patient," and "individual" are used interchangeably herein, and refer to a mammalian subject to be treated, with human patients being preferred. In some cases, the methods of the disclosure find use in experimental animals, in veterinary application, and in the development of animal models for disease, including, but not limited to, rodents including mice, rats, and hamsters; and primates.

"Sample" is used herein in its broadest sense. A sample comprising cells, polynucleotides, polypeptides, peptides, antibodies and the like may comprise a bodily fluid; a soluble fraction of a cell preparation, or media in which cells were grown; a chromosome, an organelle, or membrane isolated or extracted from a cell; genomic DNA, RNA, or cDNA, polypeptides, or peptides in solution or bound to a substrate; a cell; a tissue; a tissue print; a fingerprint, skin or hair; and the like.

"Treatment" is an intervention performed with the intention of preventing the development or altering the pathology or symptoms of a disorder. Accordingly, "treatment" refers to both therapeutic treatment and prophylactic or preventative measures. Those in need of treatment include those already with the disorder as well as those in which the disorder is to be prevented. For example, in tumor (e.g. cancer) treatment, a therapeutic agent may directly decrease the pathology of tumor cells, or render the tumor cells more susceptible to treatment by other therapeutic agents, e.g., radiation and/or chemotherapy. As used herein, "ameliorated" refers to a symptom which approaches a normalized value (by way of example but not limitation, a value obtained in a healthy patient or individual), e.g., is less than 50% different from a normalized value, preferably is less than about 25% different from a normalized value, more preferably, is less than 10% different from a normalized value, and still more preferably, is not significantly different from a normalized value as determined using routine statistical tests. By way of example but not limitation, amelioration or treatment of a patient suffering from an infectious disease organism, such as for example, Hepatitis B Virus, may be determined by a decrease of viral particles in a sample taken from a patient, as measured by, for example, a decrease in plaque forming units (p.f.u.).

"Treatment cycle" as used herein can generally refer to any of the primary treatment cycles, a first treatment cycle, a second treatment cycle or one or more additional treatment cycles.

As used herein, the term "therapeutically effective dose" or "therapeutically effective amount" is meant an amount of a compound of the present disclosure effective to yield the desired therapeutic response. By way of example but not limitation, a dose effective to delay the growth of or to cause the cancer to shrink or prevent metastasis can be a "therapeutically effective dose." The specific therapeutically effective dose will vary with such factors as the particular condition being treated, the physical condition of the patient, the type of mammal or animal being treated, the duration of the treatment, the nature of concurrent therapy (if any), and the specific formulations employed and the structure of the compounds or its derivatives.

"Immune cells" as used herein is meant to include any cells of the immune system that may be assayed, including, but not limited to, B lymphocytes, also called B cells, T lymphocytes, also called T cells, natural killer (NK) cells, natural killer T (NKT) cells, lymphokine-activated killer (LAK) cells, monocytes, macrophages, neutrophils, granulocytes, mast cells, platelets, Langerhans cells, stem cells, dendritic cells, peripheral blood mononuclear cells, tumor-infiltrating (TIL) cells, gene modified immune cells including hybridomas, drug modified immune cells, and derivatives, precursors or progenitors of the above cell types.

"T cells" are a subset of lymphocytes originating in the thymus and having heterodimeric receptors associated with proteins of the CD3 complex (e.g., a rearranged T cell receptor, the heterodimeric protein on the T cell surfaces responsible for antigen/MHC specificity of the cells). T cell responses may be detected by assays for their effects on other cells (e.g., target cell killing, activation of other immune cells, such as B-cells) or for the cytokines they produce.

As used herein, the term "anti-CD3/anti-CD28" should be understood to refer to anti-CD3/anti-CD28 antibodies. For example, "anti-CD3/anti-CD28 magnetic beads" should be understood to refer to magnetic beads having anti-CD3/anti-CD28 antibody moieties associated therewith. In instances where it is disclosed that no anti-CD3/anti-CD28 co-stimulation is provided even by a specific form such as anti-CD3/anti-CD28 magnetic beads, it should be understood that this can also exclude co-stimulation with other forms of anti-CD3/anti-CD28.

As used herein, the term "T-Rapa cell(s)" refers to T cell(s) produced by co-stimulation with anti-CD3/anti-CD28 coated magnetic beads at a ratio of 3:1 (bead:T cell ratio) without a delay between culture initiation and co-stimulation, where the cells are grown in X-Vivo or the equivalent media containing IFN-α (10,000 IU/mL), IL-2 (20 IU/mL) and rapamycin (1 µM), supplemented with 5% AB serum, but no IL-2 signaling inhibitor, where the cells are cultured for 6 days at 37°C and are initiated into culture at a concentration of 1.5 x 10⁶ cells/mL. As used in reference to methods "T-Rapa" refers to the method of producing T-Rapa cells as defined above unless otherwise noted.

As used herein, the term "manufactured T cells" and "Rapa-T cells" are used interchangeably to refer to T cells produced by the methods of the present disclosure. "Manufactured T cells" can include CD4⁺, CD8⁺ T cells or both. "Manufactured T cells" do not include T cells as collected from a patient, i.e. naturally occurring T cells.

It should be understood that as used herein "level of expression," "expression level" or an equivalent reference, when used to refer to results measured by flow cytometry refers to the frequency of positive cells of the specified type in the population. To the extent that the "level of expression" or equivalent refers to that of a specific cell type, it should be understood, that any reduction or increased referenced is relative to the corresponding specified cell type unless otherwise noted.

As will be recognized by those in the art, the term "autologous" cells means cells that are of the same or similar haplotype as that of the subject or "host" to which the cells are administered, such that no significant immune response against these cells occurs when they are transplanted into a host.

"CD4" is a cell surface protein important for recognition by the T cell receptor of antigenic peptides bound to MHC class II molecules on the surface of an APC. Upon activation, naive CD4 T cells differentiate into one of at least two cell types, Th1 cells and Th2 cells, each type being characterized by the cytokines it produces. "Th1 cells" are primarily involved in activating macrophages with respect to cellular immunity and the inflammatory response, whereas "Th2 cells" or "helper T cells" are primarily involved in stimulating B cells to produce antibodies (humoral immunity). CD4 is the receptor for the human immunodeficiency virus (HIV). Effector molecules for Th1 cells include, but are not limited to, IFN-γ, GM-CSF, TNF-α, CD40 ligand, Fas ligand, IL-3, TNF-β, and IL-2. Effector molecules for Th2 cells include, but are not limited to, IL-4, IL-5, IL-13, CD40 ligand, IL-3, G-CSF, IL-10, TGF-β, and eotaxin. Activation of the Th1 type cytokine response can suppress the Th2 type cytokine response, and reciprocally, activation of the Th2 type cytokine response can suppress the Th1 type response.

A "cytokine" is a protein made by a cell that affects the behavior of other cells through a "cytokine receptor" on the surface of the cells the cytokine effects. Cytokines manufactured by lymphocytes are sometimes termed "lymphokines." Cytokines are also characterized as Type I (e.g. IL-2 and IFN-gamma) and Type II (e.g. IL-4 and IL-10).

By the term "modulate," it is meant that any of the mentioned activities, are, e.g., increased, enhanced, increased, agonized (acts as an agonist), promoted, decreased, reduced, suppressed blocked, or antagonized (acts as an agonist). Modulation can increase activity more than 1-fold, 2-fold, 3-fold, 5-fold, 10-fold, 100-fold, etc., over baseline values. Modulation can also decrease its activity below baseline values.

"Substrate" refers to any rigid or semi-rigid support to which nucleic acid molecules or proteins are bound and includes membranes, filters, chips, slides, wafers, fibers, magnetic or nonmagnetic beads, gels, capillaries or other tubing, plates, polymers, and microparticles with a variety of surface forms including wells, trenches, pins, chamiels and pores.

### Methods for producing manufactured T cells, manufactured T cells produced by the methods disclosed herein, and compositions comprising a population of manufactured T cells

In methods of the present disclosure, IFN-α is utilized for the ex vivo polarization of a culture comprising T cells towards the Th1-type differentiation status phenotype. The Th1-type differentiation can be eroded by polarization towards a regulatory T (T_{REG}) cell phenotype, which is promoted by cytokines including IL-2, which signals through STAT5 to promote FoxP3 transcription factor that defines in part T_{REG} differentiation status. In the methods of the present disclosure, T_{REG} contamination is limited during Th1-type polarization by omitting exogenous use of IL-2 during cell culture and by preventing autocrine IL-2 signaling by culture addition of an IL-2 signaling inhibitor. In some aspects, the IL-2 signaling inhibitor is an anti-IL-2 receptor monoclonal antibody.

In the present disclosure, new methods for manufacturing are provided which incorporate the following interventions compared to other T cell manufacturing methods: (1) delayed or no addition of anti-CD3/CD28 beads (alternatively, one can provide any alternative source of anti-CD3/anti-CD28 co-stimulation such as nanoparticles or microparticles) to culture for improved T cell yield, where anti-CD3/CD28 beads or nanoparticles are used for co-stimulation; (2) use of a lower ratio of anti-CD3/CD28 beads for enhancement of the resistant T cell phenotype, or alternatively, no addition of bead, nanoparticle or microparticle artificial antibody-based co-stimulation; (3) use of the parenteral formulation of mTOR inhibition (temsirolimus) for increased manufacturing feasibility; and (4) avoidance of IL-2 signaling and resultant T_{REG} cell contamination of Th1-type differentiation by omitting the typical use of exogenous IL-2 during T cell culture and by abrogating endogenous, autocrine IL-2 signaling, for example, through use of an anti-IL-2 receptor monoclonal antibody (daclizumab or basiliximab or other reagents that inhibit IL-2 receptor signaling) during T cell culture. In side-by-side culture experiments, T cells generated by this new combinatorial method, termed "manufactured T cells," manifested a more optimal cellular phenotype relative to previously described T cells produced in ex vivo culture.

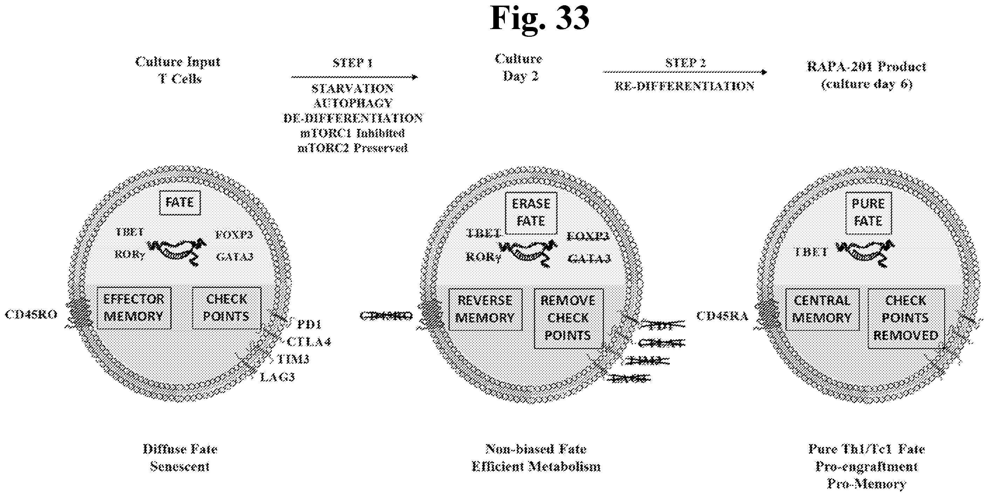

FIGURE 33 provides an exemplary workflow for generating manufactured T cells of the present disclosure.

The present invention discloses a method for producing manufactured T cells, which includes the steps of inoculating a culture input population of cells comprising T cells from a subject at a cell density in a culture medium comprising temsirolimus and an IL-2 signaling inhibitor. The culture medium does not already include temsirolimus and/or the IL-2 signaling inhibitor and these components can be added at or about the same time as the inoculation. The culture input population of cells is incubated for a first period of time without co-stimulation by anti-CD3/anti-CD28 antibodies, including, by way of example but not limitation, co-stimulation with anti-CD3/anti-CD28 coated magnetic beads, nanoparticles or microparticles. After the first period of time, the culture input population of cells can be stimulated by anti-CD3/anti-CD28 antibodies, for example, by adding anti-CD3/anti-CD28 coated magnetic beads, nanoparticles or microparticles. Where anti-CD3/anti-CD28 coated magnetic beads are used, these can be used at a bead ratio between 1:1 and 1:12. In addition, IFN-α is added to the culture medium. The culture input population of cells is then incubated for a second period of time to yield the manufactured T cells. In some aspects, there is no co-stimulation with anti-CD3/anti-CD28 coated magnetic beads, nanoparticles or microparticles. In some embodiments, no co-stimulation is performed.

In any of the foregoing embodiments, the method of producing manufactured T cells can further include after harvesting said manufactured T cells: packaging at least a portion of said manufactured T cells in a package; and freezing said package containing said portion of said manufactured T cells. Cryopreservation of said manufactured T cells can be performed by methods known in the art.

In any of the foregoing embodiments, the method can further include before inoculating T cells from said subject at a cell density in a culture medium: harvesting said culture input population of cells from said subject.

The culture medium can not contain IL-2 and no IL-2 can be added to said culture medium. In any of the foregoing embodiments, no serum can added to the culture, *e.g.* the culture is serum-free. In any of the foregoing embodiments, the culture medium can be substantially free of serum.

In any of the foregoing embodiments, said IFN-α can be added at or about the same time as the anti-CD3/anti-CD28 coated magnetic beads are added. If no co-stimulation of the culture is performed, IFN-α can be added, for example, at culture initiation or within 48 hours of culture initiation. By way of example, but not limitation, IFN-α can be added at 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48 hours after culture initiation.

In any of the foregoing embodiments, said cell density can be about 1 x 10⁶ T cells per mL to 50 x 10⁶ cells per mL. By way of example but not limitation, said cell density may be about 1 x 10⁶ cells per mL to 5 x 10⁶ cells per mL, 1 x 10⁶ cells per mL to 10 x 10⁶ cells per mL, 1 x 10⁶ cells per mL to 15 x 10⁶ cells per mL, 15 x 10⁶ cells per mL to 22.5 x 10⁶ cells per mL, 10 x 10⁶ cells per mL to 22.5 x 10⁶ cells per mL, 10 x 10⁶ cells per mL to 22.5 x 10⁶ cells per mL, 5 x 10⁶ cells per mL to 22.5 x 10⁶ cells per mL, 1 x 10⁶ cell per mL to 50 x 10⁶ cells per mL, 10 x 10⁶ cells per mL to 40 x 10⁶ cells per mL, 20 x 10⁶ cells per mL to 40 x 10⁶ cells per mL, 1 x 10⁶ cells per mL, 2.5 x 10⁶ cells per mL, 5 x 10⁶ cells per mL, 7.5 x 10⁶ cells per mL, 10 x 10⁶ cells per mL, 12.5 x 10⁶ cells per mL, 15 x 10⁶ cells per mL, 17.5 x 10⁶ cells per mL, 20 x 10⁶ cells per mL, 22.5 x 10⁶ cells per mL, 25 x 10⁶ T cells per mL, 30 x 10⁶ cells per mL, 35 x 10⁶ cells per mL, 40 x 10⁶ cells per mL, 45 x 10⁶ cells per mL, or 50 x 10⁶ cells per mL.

. In any of the foregoing embodiments, temsirolimus can be present in said culture medium at a concentration of 1 µM. By way of example, but not limitation, temsirolimus can be present in said culture medium at a concentration of at least 1.0 µM, 1.5 µM, 2.0 µM, 2.5 µM, 3.0 µM, 3.5 µM, 4.0 µM, 4.5 µM, 5.0 µM or more. By way of further example, but not limitation, temsirolimus can be present in said culture medium at a concentration of about 1-5 µM, 2-5 µM, 3-5 µM, 4-5 µM, 1.0 µM, 1.5 µM, 2.0 µM, 2.5 µM, 3.0 µM, 3.5 µM, 4.0 µM, 4.5 µM, 5.0 µM, or more.

In any of the foregoing embodiments, said temsirolimus can be added to said culture medium one or more times during the second period of time to maintain a desired concentration. In any of the foregoing embodiments, temsirolimus can be added once to the culture medium. By way of non-limiting example, said temsirolimus can be added to said culture medium every 2 days during said second period of time. The concentration of temsirolimus is at least 1 µM. By way of example but not limitation, the concentration of temsirolimus is at least 1.0 µM, 1.5 µM, 2.0 µM, 2.5 µM, 3.0 µM, 3.5 µM, 4.0 µM, 4.5 µM, 5.0 µM or more. By way of further example, but not limitation, the desired concentration of temsirolimus can be a concentration of about 1-5 µM, 2-5 µM, 3-5 µM, 4-5 µM, 1.0 µM, 1.5 µM, 2.0 µM, 2.5 µM, 3.0 µM, 3.5 µM, 4.0 µM, 4.5 µM, 5.0 µM, or more.

The IL-2 signaling inhibitor can be any substance that inhibits IL-2 signaling and can be added in an amount sufficient to inhibit IL-2 signaling. In any of the foregoing embodiments, said IL-2 signaling inhibitor can be an anti-IL-2 receptor antibody or fragment thereof, such as basiliximab or daclizumab. Said IL-2 signaling inhibitor can be present in said culture medium at a concentration of 5 to 50 µg/mL. By way of non-limiting example, IL-2 signaling inhibitor can be present at a concentration of about 5 to 50 µg/mL, 5 to 40 µg/mL, 5 to 30 µg/mL, 5 to 20 µg/mL, 5 to 10 µg/mL, 40 to 50 µg/mL, 30 to 50 µg/mL, 20 to 50 µg/mL, 20 to 40 µg/mL, 20 to 30 µg/mL, 5 µg/mL, 10 µg/mL, 15 µg/mL, 20 µg/mL, 25 µg/mL, 30 µg/mL, 35 µg/mL, 40 µg/mL, 45 µg/mL, or 50 µg/mL.

In any of the foregoing embodiments, said first period of time can be about 8 hours to about 24 hours. By way of non-limiting example, said first period of time can be about 8 hours to about 20 hours, 8 hours to about 16 hours, 8 hours to about 12 hours, 20 hours to about 24 hours, 16 hours to about 24 hours, 12 hours to about 24 hours, 8 hours, 10 hours, 12 hours, 14 hours, 16 hours, 18 hours, 20 hours, 22 hours, or 24 hours.

In any of the foregoing embodiments, said bead:T cell ratio can be 1:3. In some embodiments, the bead:T cell ratio can be between 1:12 and 1:1, or in the most extreme example, no bead addition. By way of example but not limitation, ratios of 1:12, 1:11, 1:10, 1:9, 1:8, 1:7, 1:6, 1:5, 1:4, 1:3, 1:2, 1:1 or any range therebetween can be used. In some embodiments, co-stimulation of the culture input population of cells can be achieved using anti-CD3/anti-CD28 containing nanoparticles which can be used at a reduced concentration than recommended. By way of example, but not limitation, such nanoparticles can be used at about 0.01X to about 0.1X, about 0.025X to about 0.1X, about 0.05X to about 0.1X, about 0.075X to about 0.1X, about 0.01X to about 0.075X, about 0.01X to about 0.05X, about 0.01X to about 0.025X, about 0.025X to about 0.075X, about 0.025X to about 0.05X, about 0.05X to about 0.075X, or about 0.01X, about 0.025X, about 0.05X, about 0.075X, or about 0.01X the recommended dose. By way of example but not limitation, a reagent such as Miltenyi^{®} T Cell TransAct^{™} could be used at a reduced dose compared to the recommended dose of 10 µL per 1 x 10⁶ T cells such as, by way of example but not limitation, 1.1 µL (a nine-fold decrease) or about 0.11X. Alternatively, if anti-CD3/anti-CD28 co-stimulation is to be used for producing manufactured T cells, the source of co-stimulation can be provided by dissolvable anti-CD3/anti-CD28 microparticles. By way of example, but not limitation, the dissolvable anti-CD3/anti-CD28 microparticles can be used at 20% of the strength recommended by the manufacturer (e.g. Cloudz^{®}; Bio-Techne). By way of further example, the dissolvable anti-CD3-anti-CD28 microparticles can be used at 5%, 10%, 15%, 20%, 25% or 30% of the manufacturer's recommended strength. The specific amount of anti-CD3/anti-CD28 reagent to be added can be titrated based on the desired functional characteristics of the final Rapa-T cell product. Specifically, a sufficient amount of reagent can be added to maintain T cell viability in vitro in the presence of the inhibitory molecules described in the present disclosure. However, any specific anti-CD3/anti-CD28 reagent should not be added in excess, as defined by: inappropriately high level of T cell activation (increase in CD25 expression by flow cytometry relative to the T cell culture with the optimal, minimal amount of co-stimulation); inappropriately high level of T cell checkpoint inhibitor receptor expression by flow cytometry; and inappropriately altered expression of molecules associated with T cell effector memory cells by flow cytometry (such as reduction in levels of CD62L and CCR7; such as increase in levels of CD45RO and KLRG).

In any of the foregoing embodiments, said IFN-α can be added to said culture medium to a concentration of about 1,000 IU/mL to about 10,000 IU/mL. By way of example but not limitation, concentrations of 2,500 IU/mL to 10,000 IU/mL, 5,000 IU/mL to 10,000 IU/mL, 7,500 IU/mL to 10,000 IU/mL, 1,000 IU/mL to 7,500 IU/mL, 1,000 IU/mL to 5,000 IU/mL, 1,000 IU/mL to 2,500 IU/mL, 2,500 IU/mL to 7,500 IU/mL, 2,500 IU/mL to 5,000 IU/mL, 5,000 IU/mL to 7,500 IU/mL, 5,000 IU/mL to 10,000 IU/mL, 7,500 IU/mL to 10,000 IU/mL, or 1,000 IU/mL, 2,500 IU/mL, 5,000 IU/mL, 7,500 IU/mL, or 10,000 IU/mL can be used. Lower concentrations of IFN-α such as 1000 IU/mL may result in less marked shift towards a Th1 phenotype.

In any of the foregoing embodiments, said second period of time can be about 4 days to about 8 days, 4 days to about 6 days, or 6 days to about 8 days. By way of non-limiting example, said second period of time can be about 4 days, 5 days, 6 days, 7 days, or 8 days. Where no co-stimulation is performed, the period of time for incubation can be about 4 days to about 8 days, 4 days to about 6 days, or 6 days to about 8 days. By way of non-limiting example, said second period of time can be about 4 days, 5 days, 6 days, 7 days, or 8 days.

In any of the foregoing embodiments, said culture medium can further comprise 5% human serum. In some embodiments, said culture medium can further comprise 1%-20% human serum. By way of example but not limitation, said culture medium may comprise about 1%, 2%, 3%, 4%, 5%, 6%, 7%, 8%, 9%, 10%, 11%, 12%, 13%, 14%, 15%, 16%, 17%, 18%, 19% or 20% human serum. In some embodiments, said culture medium can comprise at least 1%, 2%, 3%, 4%, 5%, 6%, 7%, 8%, 9%, 10%, 11%, 12%, 13%, 14%, 15%, 16%, 17%, 18%, 19% or 20% human serum. In any of the foregoing embodiments, no serum can be added to or present in the culture medium.

In any of the foregoing embodiments, said culture medium can further comprise X-Vivo 20 medium. In any of the foregoing embodiments, said culture medium can further comprise TexMACS (Miltenyi^{®}) medium. Any suitable culture medium can be used for culturing T cells.

In any of the foregoing embodiments, additional culture medium can be added to the culture. By way of example but not limitation, additional culture medium can be added at about 12 hours, 24 hours, 36 hours, 48 hours, 60 hours, 72 hours, 84 hours, 96 hours, 108 hours, 120 hours or any range or time therebetween after the initial inoculation of the culture input population of cells into culture. By way of example, but not limitation, the amount of culture medium added relative to the initial amount of culture medium can be in a ratio of about 0.5, 0.75, 1, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0 or more, and any range therebetween. By way of example, but not limitation, the amount of culture medium added after the initial inoculation of the culture input population of cells into culture can be an amount sufficient to reduce the cell density in the culture to a target cell density. By way of example but not limitation, this target cell density can be about 1 x 10⁶, 2 x 10⁶, 3 x 10⁶, 4 x 10⁶, 5 x 10⁶, 6 x 10⁶, 7 x 10⁶, 8 x 10⁶, 9 x 10⁶, 1 x 10⁷, 2 x 10⁷, 3 x 10⁷, or 4 x 10⁷ and any range therebetween, provided that the initial cell density is greater than the target cell density.

In any of the foregoing embodiments, said culture input population of cells can comprise about 5% to about 100%, about 10% to about 100%, about 20% to about 100%, about 30% to about 100%, about 40% to about 100%, about 50% to about 100%, about 60% to about 100%, about 70% to about 100%, about 80% to about 100%, about 90% to about 100%, about 5% to about 90%, about 5% to about 80%, about 5% to about 70%, about 5% to about 60%, about 5% to about 50%, about 5% to about 40%, about 5% to about 30%, about 5% to about 20%, or about 5% to about 10%, about T cells out of the total number of cells in the culture input population of cells. By way of non-limiting example, said culture input population of cells can comprise about 5%, 10%, 15%, 20%, 33%, 40%, 50%, 66%, 70%, 75%, 90%, 95%, 98%, or 99% or more T cells out of the total number of cells in said culture input population of cells.

In any of the foregoing embodiments, said culture input population of cells can further comprise monocytes. In any of the foregoing embodiments, the culture input population of cells can be enriched for T cells. By way of example, but not limitation, the culture input population of cells can be subjected to T cell enrichment using an automated Ficoll procedure. Methods to perform the Ficoll procedure are known in the art, and involve removing neutrophils and red blood cells from the sample. Any suitable method for enriching T cells in a population of cells can be used.

In any of the foregoing embodiments, the method can further comprise harvesting a sample comprising T cells from said subject; and isolating T cells from said sample to yield said culture input population of cells. Such samples can contain peripheral blood stem cells (PBSCs) and can be obtained, by way of example but not limitation, by mobilized collection, steady state apheresis or a simple blood draw. In any of the foregoing embodiments, the sample containing PBSCs and/or the culture input population of cells can be cryopreserved prior to manufactured T cell preparation. Steady state apheresis can be performed when the subject has a sufficient number of immune cells which, by way of example but not limitation can be characterized by a minimum absolute lymphocyte count (ALC). The minimum ALC can, for example, be 300 lymphocytes per microliter.

In any of the foregoing embodiments, said T cells can isolated by antibody-based purification.

In any of the foregoing embodiments, said enrichment of T cells can be performed by counter-flow centrifugal elutriation. Such a technique is well known in the art.

In any of the foregoing embodiments, said IFN-α can be added at or about the same time as the anti-CD3/anti-CD28 containing nanoparticles are added.

In any of the foregoing embodiments, the anti-CD3/anti-CD28 antibodies can be removed by any suitable method after culture. By way of example, but not limitation, anti-CD3/anti-CD28 magnetic beads can be removed by magnetic capture and dissolvable anti-CD3/anti-CD28 microparticles can be removed by adding release buffer and washing the manufactured T cells.

In some embodiments, a population of manufactured T cells exhibits increased IFN-γ secretion relative to T-Rapa cells after one week of incubation using stimulation with anti-CD3/anti-CD28 magnetic beads added at a bead:T cell ratio of 3:1.

In some embodiments, a population of manufactured T cells exhibits increased TNF-α secretion relative to T-Rapa cells after one week of incubation using stimulation with anti-CD3/anti-CD28 magnetic beads added at a bead:T cell ratio of 3:1.

In some embodiments, a population of manufactured T cells exhibits increased GM-CSF secretion relative to T-Rapa cells after one week of incubation using stimulation with anti-CD3/anti-CD28 magnetic beads added at a bead:T cell ratio of 3:1.

In some embodiments, a population of manufactured T cells exhibits increased IL-2 secretion relative to T-Rapa cells after one week of incubation using stimulation with anti-CD3/anti-CD28 magnetic beads added at a bead:T cell ratio of 3:1.

In some embodiments, a population of manufactured T cells comprises an increased percentage of cells positive for CD4, CD62L, CCR7 and CD127 relative to said control population of T cells and to T-Rapa cells.

In some embodiments, a population of manufactured T cells exhibits an increase in 4EBP1 phosphorylation relative to said control population of T cells. In some embodiments, a manufactured T cell exhibits increased 4EBP1 phosphorylation relative to a control T cell. By way of example but not limitation, the increase in phosphorylation of 4EBP1 relative to a control population of T cells characteristic of the T cells from which the T cell was produced, i.e. culture input T cells, or a control T cell is no more than 50%, no more than 45%, no more than 40%, no more than 35%, or no more than 30%. By way of further example but not limitation, the increase in 4EBP1 phosphorylation can be between 5-50%, 5-45%, 5-40%, 5-30%, 5-20%, 5-10%, 10-50%, 10-45%, 10-40%, 10-30%, 10-20%, 20-50%, 20-45%, 20-40%, 20-30%, 30-50%, 30-45%, 30-40%, 40-50%, or any value therebetween or ranges within these ranges. The phosphorylation of 4EBP1 is reduced (or blunted) as compared to T-Rapa cells. In some embodiments, the increase in 4EBP1 phosphorylation can be measured at 32 hours post-initiation of culture.

In some embodiments, a population of manufactured T cells exhibits reduced P70S6K expression relative to T-Rapa cells and increased relative to said control population of T cells characteristic of the cells form which the manufactured T cells were produced. By way of example, but not limitation, the increase can be by at least 10%, 20%, 30%, 40%, 50% or more and the reduction can be by 50%, 60%, 70%, 80% or more.

In some embodiments, a population of manufactured T cells exhibits reduced expression of the IL-2 receptor CD25 by flow cytometry relative to T-Rapa cells. By way of example, but not limitation, the reduction can be by at least 50%, 60%, 70%, 80%, 90% or more.

In some embodiments, a manufactured T cell can express a unique RNA expression profile relative to culture input T cells, characterized by a 50% or greater increase in RNA content of de-differentiation molecules such as KLF4, KLF10, Nanog and combinations thereof and a 50% or greater decrease in RNA content of differentiation molecules such as perforin, granzyme B, IFN-□, and combinations thereof.

In some embodiments, a population of manufactured T cells exhibits reduced levels of the following molecules associated with immune suppressive effects relative to T-Rapa cells: CTLA4; and TIM3.

In some embodiments, a population of manufactured T cells can be characterized by 10% or less of the CD4+ or CD8+ manufactured T cells expressing CTLA4 as measured by flow cytometry. In some embodiments, the population of manufactured T cells can be characterized by 5% or less of the CD4+ or CD8+ manufactured T cells expressing CTLA4 as measured by flow cytometry. By way of example, but not limitation, 10%, 9%, 8%, 7%, 6%, 5%, 4%, 3%, 2%, 1% or less of the CD4+ or CD8+ T cells in the population of manufactured T cells can express CTLA4 as measured by flow cytometry. In some embodiments, the population of manufactured T cells can exhibit a reduced frequency of CD4+ or CD8+ T cells expressing CTLA4 relative to the corresponding frequency of CD4+ or CD8+ T-Rapa cells expressing CTLA4 as measured by flow cytometry. In some embodiments, the reduced frequency of CD4+ or CD8+ T cells expressing CTLA4 can be at least 50% less than the corresponding frequency of CD4+ or CD8+ T-Rapa cells expressing CTLA4. By way of example, but not limitation, the reduced frequency can be at least 50%, 60%, 70%, 80%, 90%, 95% or 99% less than the corresponding frequency. In some embodiments, the reduced frequency is 6 days after the cells resulting in the manufactured T cells were inoculated into culture.

In some embodiments, a population of manufactured T cells can be characterized by 10% or less of the CD4+ or CD8+ manufactured T cells expressing TIM3 as measured by flow cytometry. In some embodiments, the population of manufactured T cells can be characterized by 5% or less of the CD4+ or CD8+ manufactured T cells expressing TIM3 as measured by flow cytometry. By way of example, but not limitation, 10%, 9%, 8%, 7%, 6%, 5%, 4%, 3%, 2% 1% or less of the CD4+ or CD8+ T cells in the population of manufactured T cells can express TIM3 as measured by flow cytometry. In some embodiments, the population of manufactured T cells can exhibit a reduced frequency of CD4+ or CD8+ T cells expressing TIM3 relative to a corresponding frequency of CD4+ or CD8+ T cells expressing TIM3 in a control population of T cells characteristic of T cells from which the manufactured T cells were produced as measured by flow cytometry. In some embodiments, the reduced frequency of CD4+ or CD8+ T cells expressing TIM3 can be at least 50% less than the corresponding frequency of CD4+ or CD8+ T cells expressing TIM3 in the control population. By way of example, but not limitation, the reduced frequency of CD4+ or CD8+ T cells expressing TIM3 can be at least 50%, 60%, 70%, 80%, 90%, 95% or 99% less than the corresponding frequency of CD4+ or CD8+ T cells expressing TIM3 in the control population. In some embodiments, the reduced frequency is 6 days after the cells resulting in the manufactured T cells were inoculated into culture. In some embodiments, the population of manufactured T cells can exhibit a reduced frequency of CD4+ or CD8+ T cells expressing TIM3 relative to a corresponding frequency of CD4+ or CD8+ T-Rapa cells expressing TIM3 as measured by flow cytometry. In some embodiments, the reduced frequency of CD4+ or CD8+ T cells expressing TIM3 can be at least 50% less than the corresponding frequency of CD4+ or CD8+ T-Rapa cells expressing TIM3. By way of example, but not limitation, the reduced frequency of CD4+ or CD8+ T cells expressing TIM3 can be at least 50%, 60%, 70%, 80%, 90%, 95% or 99% less than the corresponding frequency of CD4+ or CD8+ T-Rapa cells expressing TIM3. In some embodiments, the reduced frequency is 6 days after the cells resulting in the manufactured T cells were inoculated into culture.

In some embodiments, a population of manufactured T cells can be characterized by 5% or less of the CD4+ or CD8+ manufactured T cells expressing PD1 as measured by flow cytometry. By way of example, but not limitation, 5%, 4%, 3%, 2%, 1% or less of the CD4+ or CD8+ T cells in the population of manufactured T cells can express PD1 as measured by flow cytometry. In some embodiments, the population of manufactured T cells can exhibit a frequency of CD4+ or CD8+ T cells expressing PD1 relative to a corresponding frequency of CD4+ and CD8+ T-Rapa cells expressing PD1 as measured by flow cytometry. In some embodiments, the reduced frequency of CD4+ or CD8+ T cells expressing PD1 can be at least 50% less than the corresponding frequency of CD4+ or CD8+ T-Rapa cells expressing PD1. By way of example, but not limitation, the reduced frequency of CD4+ or CD8+ T cells expressing PD1 can be at least 50%, 60%, 70%, 80%, 90%, 95% or 99% less than the corresponding frequency of CD4+ or CD8+ T-Rapa cells expressing PD1. In some embodiments, the reduced frequency is 6 days after the cells resulting in the manufactured T cells were inoculated into culture.

In some embodiments, a population of manufactured T cells can be characterized by 5% or less of the CD4+ and CD8+ manufactured T cells expressing 2B4 as measured by flow cytometry. By way of example, but not limitation, 5%, 4%, 3%, 2%, 1% or less of the CD4+ or CD8+ T cells in the population of manufactured T cells can express 2B4 as measured by flow cytometry. In some embodiments, at least 0.1% of the CD4+ T cells in the population of manufactured T cells express 2B4 as measured by flow cytometry. By way of example, but not limitation, at least 0.1%, 0.2%, 0.3%, 0.5%, 0.6%, 0.7%, 0.8%, 0.9%, 1% or more of the CD4+ T cells in the population of manufactured T cells can express 2B4 as measured by flow cytometry. In some embodiments, the population of manufactured T cells can exhibit a reduced frequency of CD8+ T cells expressing 2B4 relative to a corresponding frequency of CD8+ T cells expressing 2B4 in control population of T cells characteristic of T cells from which the manufactured T cells were produced as measured by flow cytometry. By way of example, but not limitation, the reduced frequency of CD8+ T cells expressing 2B4 can be by at least 50%, 60%, 70%, or 80% less than the corresponding frequency of CD8+ T cells expressing 2B4 in the control population of T cells. In some embodiments, the reduced frequency is 6 days after the cells resulting in the manufactured T cells were inoculated into culture. In some embodiments, the population of manufactured T cells can exhibit a reduced frequency of CD4+ or CD8+ T cells expressing 2B4 relative to a corresponding frequency of CD4+ or CD8+ T-Rapa cells expressing 2B4 as measured by flow cytometry. By way of example, but not limitation, the reduced frequency of CD4+ or CD8+ T cells expressing 2B4 can be by at least 20%, 30%, 40%, 50%, 60%, 70% or 80% less than the corresponding frequency of CD4+ or CD8+ T-Rapa cells expressing 2B4. In some embodiments, the reduction is 6 days after the cells resulting in the manufactured T cells were inoculated into culture.

In some embodiments, a population of manufactured T cells can be characterized by 10% or less of the CD4+ or CD8+ manufactured T cells expressing LAIR1 as measured by flow cytometry. By way of example, but not limitation, 10%, 9%, 8%, 7%, 6%, 5%, 4%, 3%, 2%, 1% or less of the CD4+ or CD8+ T cells in the population of manufactured T cells can express LAIR1 as measured by flow cytometry. In some embodiments, the population of manufactured T cells can exhibit a reduced frequency of CD4+ or CD8+ T cells expressing LAIR1 relative to a corresponding frequency of CD4+ or CD8+ T cells expressing LAIR1 in a control population of T cells characteristic of T cells from which the manufactured T cells were produced as measured by flow cytometry. By way of example, but not limitation, the reduced frequency of CD4+ or CD8+ T cells expressing LAIR1 can be by at least 50%, 60%, 70%, 80%, 90%, 95% or 99% less than the corresponding frequency of CD4+ or CD8+ T cells expressing LAIR1 in the control population of T cells. In some embodiments, the reduced frequency is 6 days after the cells resulting in the manufactured T cells were inoculated into culture. In some embodiments, the CD4+ or CD8+ T cells of the population of manufactured T cells can exhibit a reduced level of expression of LAIR1 relative to T-Rapa cells as measured by flow cytometry. By way of example, but not limitation, the reduction can be by at least 30%, 40%, 50%, or more.

In some embodiments, a population of manufactured T cells can be characterized by 10% or less of the CD4+ or CD8+ manufactured T cells expressing TIGIT as measured by flow cytometry. By way of example, but not limitation, 10%, 9%, 8%, 7%, 6%, 5%, 4%, 3%, 2%, 1% or less of the CD4+ or CD8+ T cells in the population of manufactured T cells can express TIGIT as measured by flow cytometry. In some embodiments, at least 0.1% of the CD4+ T cells in the population of manufactured T cells express 2B4 as measured by flow cytometry. By way of example, but not limitation, at least 0.1%, 0.2%, 0.3%, 0.5%, 0.6%, 0.7%, 0.8%, 0.9%, 1% or more of the CD4+ T cells in the population of manufactured T cells can express TIGIT as measured by flow cytometry. In some embodiments, the population of manufactured T cells can exhibit a reduced frequency of CD4+ or CD8+ T cells expressing TIGIT relative to a corresponding frequency of CD4+ or CD8+ T-Rapa cells expressing TIGIT as measured by flow cytometry. By way of example, but not limitation, the reduced frequency of CD4+ or CD8+ T cells expressing TIGIT can be at least 40%, 50%, 60%, 70%, 80% or 90% less than the corresponding frequency of CD4+ or CD8+ T-Rapa cells expressing TIGIT. In some embodiments, the reduced frequency is 6 days after the cells resulting in the manufactured T cells were inoculated into culture.

In some embodiments, a population of manufactured T cells can be characterized by 10% or less of the CD4+ or CD8+ manufactured T cells expressing LAG3 as measured by flow cytometry. By way of example, but not limitation, 10%, 9%, 8%, 7%, 6%, 5%, 4%, 3%, 2%, 1% or less of the CD4+ or CD8+ T cells in the population of manufactured T cells can express LAG3 as measured by flow cytometry. In some embodiments, the population of manufactured T cells can exhibit a reduced frequency of CD4+ or CD8+ T cells expressing LAG3 relative to a corresponding frequency of CD4+ or CD8+ T-Rapa cells expressing LAG3 as measured by flow cytometry. In some embodiments, the reduced frequency of CD4+ or CD8+ T cells expressing LAG3 can be by at least 50% less than the corresponding frequency of CD4+ or CD8+ T-Rapa cells expressing LAG3. By way of example, but not limitation, the reduced frequency of CD4+ or CD8+ T cells expressing LAG3 can be at least 50%, 60%, 70%, 80%, 90%, 95% or 99% less than the corresponding frequency of CD4+ or CD8+ T-Rapa cells expressing LAG3. In some embodiments, the reduced frequency is 6 days after the cells resulting in the manufactured T cells were inoculated into culture

In some embodiments, a population of manufactured T cells can be characterized by a preserved level, i.e. substantially the same level, of positive co-stimulatory molecule CD28 relative to a control population of T cells characteristic of T cells from which the manufactured T cells were produced as measured by flow cytometry. In some embodiments, the frequency of CD28 expression in CD4+ or CD8+ T cells in the population of manufactured T cells can be within about 20% of the frequency of CD28 expression in CD4+ or CD8+ T cells in the control population, respectively. By way of example, but not limitation, the frequency of CD28 expression in CD4+ or CD8+ T cells in the population of manufactured T cells can be within 20%, 15%, 10%, 9%, 8%, 7%, 6%, 5%, 4%, 3%, 2%, or 1% of the frequency of CD28 expression in CD4+ or CD8+ T cells in the control population, respectively. In some embodiments, this preservation is 6 days after the cells resulting in the manufactured T cells were inoculated into culture.

In some embodiments, a population of manufactured T cells can be characterized by a preserved level, i.e. substantially the same level, of positive co-stimulatory molecule ICOS relative to a control population of T cells characteristic of T cells from which the manufactured T cells were produced as measured by flow cytometry. In some embodiments, the frequency of ICOS expression in CD4+ or CD8+ T cells in the population of manufactured T cells can be within about 20% of the frequency of ICOS expression in CD4+ or CD8+ T cells in the control population, respectively. By way of example, but not limitation, the frequency of ICOS expression in CD4+ or CD8+ T cells in the population of manufactured T cells can be within 20%, 15%, 10%, 9%, 8%, 7%, 6%, 5%, 4%, 3%, 2%, or 1% of the frequency of ICOS expression in CD4+ or CD8+ T cells in the control population, respectively. In some embodiments, this preservation is 6 days after the cells resulting in the manufactured T cells were inoculated into culture.

In some embodiments, a population of manufactured T cells can be characterized by a preserved level, i.e. substantially the same level, of CD45RA relative to a control population of T cells characteristic of T cells from which the manufactured T cells were produced as measured by flow cytometry. In some embodiments, the frequency of CD45RA expression in CD4+ or CD8+ T cells in the population of manufactured T cells can be within about 20% of the frequency of CD45RA expression in CD4+ or CD8+ T cells in the control population, respectively. By way of example, but not limitation, the frequency of CD45RA expression in CD4+ or CD8+ T cells in the population of manufactured T cells can be within 20%, 15%, 10%, 9%, 8%, 7%, 6%, 5%, 4%, 3%, 2%, or 1% of the frequency of CD45RA expression in CD4+ or CD8+ T cells in the control population, respectively. In some embodiments, this preservation is 6 days after the cells resulting in the manufactured T cells were inoculated into culture.

In some embodiments, a population of manufactured T cells can be characterized by 5% or less of the CD4+ or CD8+ manufactured T cells expressing CD25 as measured by flow cytometry. By way of example, but not limitation, 5%, 4%, 3%, 2%, 1% or less of the CD4+ or CD8+ T cells in the population of manufactured T cells can express CD25 as measured by flow cytometry. In some embodiments, the population of manufactured T cells can exhibit a reduced frequency of CD4+ or CD8+ T cells expressing CD25 relative to a corresponding frequency of CD4+ or CD8+ T-Rapa cells expressing CD25 as measured by flow cytometry. In some embodiments, the reduced frequency of CD4+ or CD8+ T cells expressing CD25 can be at least 50% less than the corresponding frequency of CD4+ or CD8+ T-Rapa cells expressing CD25. By way of example, but not limitation, the reduced frequency of CD4+ or CD8+ T cells expressing CD25 can be at least 50%, 60%, 70%, 80%, 90%, 95% or 99% less than the corresponding frequency of CD4+ or CD8+ T-Rapa cells expressing CD25. In some embodiments, the reduced frequency is 6 days after the cells resulting in the manufactured T cells were inoculated into culture.

In some embodiments, a population of manufactured T cells exhibits a quiescent and non-senescent phenotype characterized by a reduced level KLRG1, as measured by flow cytometry. In some embodiments, the reduction in the level of KLRG1 is 6 days after the cells resulting in the manufactured T cells were inoculated into culture. In some embodiments, a population of manufactured T cells can be characterized by 5% or less of the CD4+ or CD8+ manufactured T cells expressing KLRG1 as measured by flow cytometry. By way of example, but not limitation, 5%, 4%, 3%, 2%, 1% or less of the CD4+ or CD8+ T cells in the population of manufactured T cells can express KLRG1 as measured by flow cytometry. In some embodiments, the population of manufactured T cells can exhibit a reduced frequency of CD4+ or CD8+ T cells expressing KLRG1 relative to a corresponding frequency of CD4+ or CD8+ T-Rapa cells expressing KLRG1 as measured by flow cytometry. In some embodiments, the reduced frequency of CD4+ or CD8+ T cells expressing KLRG1 can be at least 50% less than the corresponding frequency of CD4+ or CD8+ T-Rapa cells expressing KLRG1. By way of example, but not limitation, the reduced frequency of CD4+ or CD8+ T cells expressing KLRG1 can be at least 50%, 60%, 70%, 80%, 90%, 95% or 99% less than the corresponding frequency of CD4+ or CD8+ T-Rapa cells expressing KLRG1. In some embodiments, the reduced frequency is 6 days after the cells resulting in the manufactured T cells were inoculated into culture. In some embodiments, the reduced frequency is 6 days after the cells resulting in the manufactured T cells were inoculated into culture.

In some embodiments, a population of manufactured T cells exhibits reduced expression of the immune suppression molecule CD39 relative to a control population of T cells characteristic of the T cells from which the manufactured T cells were produced. In some embodiments, a population of manufactured T cells can be characterized by 20% or less of the CD4+ or CD8+ manufactured T cells expressing CD39 as measured by flow cytometry. By way of example, but not limitation, 20%, 19%, 18%, 17%, 16%, 15%, 14%, 13%, 12%, 11%, 10%, 9%, 8%, 7%, 6%, 5% or less of the CD4+ or CD8+ T cells in the population of manufactured T cells can express CD39 as measured by flow cytometry. In some embodiments, the population of manufactured T cells can exhibit a reduced frequency of CD4+ or CD8+ T cells expressing CD39 relative to a corresponding frequency of CD4+ or CD8+ T-Rapa cells expressing CD39 as measured by flow cytometry. In some embodiments, the reduced frequency of CD4+ or CD8+ T cells expressing CD39 can be at least 50% less than the corresponding frequency of CD4+ or CD8+ T-Rapa cells expressing CD39. By way of example, but not limitation, the reduced frequency of CD4+ or CD8+ T cells expressing CD39 can be at least 50%, 60%, 70%, 80%, 90%, 95% or 99% less than the corresponding frequency of CD4+ or CD8+ T-Rapa cells expressing CD39. In some embodiments, the reduced frequency is 6 days after the cells resulting in the manufactured T cells were inoculated into culture.

In some embodiments, a population of manufactured T cells exhibits reduced expression of the immune suppression molecule CD73 relative to a control population of T cells characteristic of the T cells from which the manufactured T cells were produced. In some embodiments, a population of manufactured T cells can be characterized by 20% or less of the CD4+ or CD8+ manufactured T cells expressing CD73 as measured by flow cytometry. By way of example, but not limitation, 20%, 19%, 18%, 17%, 16%, 15%, 14%, 13%, 12%, 11%, 10%, 9%, 8%, 7%, 6%, 5% or less of the CD4+ or CD8+ T cells in the population of manufactured T cells can express CD73 as measured by flow cytometry. In some embodiments, the population of manufactured T cells can exhibit a reduced frequency of CD4+ or CD8+ T cells expressing CD73 relative to a corresponding frequency of CD4+ or CD8+ T-Rapa cells expressing CD73 as measured by flow cytometry. In some embodiments, the reduced frequency of CD4+ or CD8+ T cells expressing CD73 can be at least 50% less than the corresponding frequency of CD4+ or CD8+ T-Rapa cells expressing CD73. By way of example, but not limitation, the reduced frequency of CD4+ or CD8+ T cells expressing CD73 can be at least 50%, 60%, 70%, 80%, 90%, 95% or 99% less than the corresponding frequency of CD4+ or CD8+ T-Rapa cells expressing CD73. In some embodiments, the reduced frequency is 6 days after the cells resulting in the manufactured T cells were inoculated into culture.

In some embodiments, a population of manufactured T cells exhibits reduced expression of the immune suppression molecule GITR relative to a control population of T cells characteristic of the T cells from which the manufactured T cells were produced. In some embodiments, a population of manufactured T cells can be characterized by 5% or less of the CD4+ or CD8+ manufactured T cells expressing GITR as measured by flow cytometry. By way of example, but not limitation, 5%, 4%, 3%, 2%, 1% or less of the CD4+ or CD8+ T cells in the population of manufactured T cells can express GITR as measured by flow cytometry. In some embodiments, the population of manufactured T cells can exhibit a reduced frequency of CD4+ or CD8+ T cells expressing GITR relative to a corresponding frequency of CD4+ or CD8+ T-Rapa cells expressing GITR as measured by flow cytometry. In some embodiments, the reduced frequency of CD4+ or CD8+ T cells expressing GITR can be at least 20% less than relative to the corresponding frequency of CD4+ or CD8+ T-Rapa cells expressing GITR. By way of example, but not limitation, the reduced frequency of CD4+ or CD8+ T cells expressing GITR can be at least 20%, 30%, 40%, 50%, 60%, 70%, 80%, 90%, 95% or 99% less than the corresponding frequency of CD4+ or CD8+ T-Rapa cells expressing GITR. In some embodiments, the reduced frequency is 6 days after the cells resulting in the manufactured T cells were inoculated into culture.

In some embodiments, a manufactured T cell can exhibit an early differentiation state of cytokine biology relative to a control T cell culture, as evidenced by an increase in secretion of the precursor cytokine IL-2 and increased responsiveness to the homeostatic cytokines IL-7 and IL-15. In this manner, the manufacturing method of the present disclosure describes a process for the manufacture of helper-independent T cells with increased reactivity to homeostatic cytokines.

In some embodiments, a population of manufactured T cells exhibits increased IL-2 secretion relative to a T-Rapa culture incubated under the same conditions. In some embodiments, this increase in IL-2 secretion is at least 1.1-fold. By way of example, but not limitation, the increase can be at least 1.1-, 1.5-, 2.0-, 2.5-, 3.0-, 3.5-, 4.0-, 4.5-, 5.0-fold or more. In some embodiments, the population of manufactured T cells secretes at least 500 pg/mL/1 x 106 cells/day IL-2 after co-stimulation with anti-CD3/anti-CD28 coated magnetic beads at a ratio between 3:1 and 1:3 beads:T cells. By way of example, the population of manufactured T cells can secrete about 500, 600, 700, 800, 900, 1000 or more pg/mL/1 x 106 cells/day of IL-2 under these conditions.

In some embodiments, the population of manufactured T cells can secrete an increased amount of IL-2 when exposed to IL-7 or IL-15. In some embodiments, the population of manufactured T cells is characterized by an increase in IL-2 secretion of at least 1.1-fold when the population of manufactured T cells is incubated in the presence of IL-7 or IL-15 realtive to conditions without the presence of IL-7 or IL-15. By way of example, but not limitation, the increase can be at least 1.1-, 1.2-, 1.3-, 1.4-, 1.5-, 1.6-, 1.7-, 1.8-, 1.9-, 2.0-fold or more. In some embodiments, the population of manufactured T cells secretes at least 1000 pg/mL/1 x 10⁶ cells/day IL-2 after co-stimulation with anti-CD3/anti-CD28 coated magnetic beads at a ratio between 3;1 and 1:3 beads:T cells and exposure to IL-7, IL-15 or both IL-7 and IL-15 at a concentration of 10 ng/mL of IL-7 and 10 ng/mL IL-15 when present. By way of example, the population of manufactured T cells can secrete about 1000, 1100, 1200, 1300, 1400, 1500, 1600, 1700, 1800, 1900, 2000 or more pg/mL/1 x 10⁶ cells/day of IL-2 under these conditions. IL-2 secretion is associated with helper-independent T cells. The high IL-2 secretion of Rapa-T cells can be advantageous by obviating the need to administer exogenous IL-2 after T cell adoptive therapy. In some embodiments, the IL-7 or IL-15 is added at 10 ng/mL, if present.

In some embodiments, said population of manufactured T cells exhibits increased in vivo function relative to said control population T cells, said in vivo function characterized by increased human T cell engraftment in a model of human-into-mouse xenogeneic graft-versus-host-disease.

In some embodiments, said population of manufactured T cells exhibits a reduction in mTORC1 activation as measured by phosphor-P70S6K. Said reduction can be, by way of example but not limitation, at least 50%, at least 60%, at least 70%, at least 80%, at least 90%, at least 95%, or at least 99% relative to T-Rapa cells at 32 hours after culture initiation.

In some embodiments, said population of manufactured T cells exhibits decreased phosphor-STAT5 relative to T-Rapa cells. Said reduction can be, by way of example but not limitation, at least 50%, at least 60%, at least 70%, at least 80%, at least 90%, at least 95%, or at least 99% relative to T-Rapa cells at 32 hours after culture initiation.

In some embodiments, said population of manufactured T cells exhibits decreased phosphor-STAT5 relative to a control population of cultured T-Rapa cells. Said reduction can be, by way of example, but not limitation, at least 50% relative to the control population of cultured T-Rapa cells. In some embodiments, said reduction is at 48 hours after the manufactured T cells were inoculated into culture from an input population of cells comprising T cells. In some embodiments, the p-STAT5 level is as measured by Western blot.

In some embodiments, said population of manufactured T cells exhibits at least some detectable level of STAT1 and phosphor-STAT1. In some embodiments, said level is measured at 48 hours after the manufactured T cells were inoculated into culture from an input population of cells comprising T cells. In some embodiments, the population of manufactured T cells exhibits decreased phosphor-STAT5 relative to a control population of T cells and at least some level of STAT1 and phosphor-STAT1. In some embodiments, the level of STAT1 or p-STAT1 is as measured by Western blot.

In some embodiments, said population of manufactured T cells exhibits a reduction in mTORC1 activation as measured by p70S6K or Raptor expression relative to a control population of T cells characteristic of the T cells from which the population of manufactured T cells was obtained. Said reduction can be, by way of example, but not limitation, by 50%. In some embodiments, said reduction is at 48 hours after the manufactured T cells were inoculated into culture from an input population of cells comprising T cells. In some embodiments, the reduction is as measured by Western blot.

In some embodiments, said population of manufactured T cells exhibits approximately the same level of Rictor, SGK1 or phosphorylated SGK1 relative to a control population of T cells. By way of example, but not limitation, the level of Rictor, SGK1 or phosphorylated SGK1 can be within 50%, 40%, 30%, 20%, 10% or 5% or the corresponding level of Rictor, SGK1 or phosphorylated SGK1 in T-Rapa cells. In some embodiments, said level is at 48 hours after the manufactured T cells were inoculated into culture from an input population of cells comprising T cells. In some embodiments, the level of Rictor, SGK1 or pSGK1 is at least the level as measured in the control population of T cells. In some embodiments, the level is as measured by Western blot.

In some embodiments, a population of manufactured T cells exhibits an increase in secretion of at least one of IFN-γ, TNF-α, GM-CSF and IL-2 relative to T-Rapa cells after expansion in culture media for 6 days after manufacturing without inhibitors. Said increase can be, by way of example, but not limitation, at least 50%, at least 60%, at least 70%, at least 80%, at least 90%, at least 95%, at least 99% or more.

In some embodiments, a population of manufactured T cells at the end of manufacturing (day 6 in culture) can have an increased number of CD4+ T cells that express the T cell marker CD45RA relative to T-Rapa cells. Said increase can be, by way of example, but not limitation, at least 50%, at least 60%, at least 70%, at least 80%, at least 90%, at least 95%, at least 99% or more.

In some embodiments, a population of manufactured T cells can have reduced expression of one or more checkpoint inhibitor receptors selected from CD39, CD73, GITR, LAG3, PD1, 2B4, LAIR1, CTLA4, KLRG1, TIGIT, and TIM3. By way of example, but not limitation, the reduced expression can be at least 25% less than a corresponding level of expression in a T-Rapa cell. By way of further example, but not limitation, the reduced expression can be at least 25%, 50%, 75%, 80%, 85%, 90%, 95% or 99% less than a corresponding level of expression in a population of T-Rapa cells. In some embodiments, a population of manufactured T cells can have a level of expression of one or more checkpoint inhibitors selected from CD39, CD73, GITR, LAG3, PD1, 2B4, LAIR1, CTLA4, KLRG1, TIGIT and TIM3 that is within about 25% of a corresponding level of expression in a control T cell population characteristic of the T cells from which the population of manufactured T cells was produced. By way of example, but not limitation, the level of expression of the one or more checkpoint inhibitors selected from CD39, CD73, GITR, LAG3, PD1, 2B4, LAIR1, CTLA4, KLRG1, TIGIT and TIM3 can be within about 25%, 20%, 15%, 10%, or 5% of a corresponding level of expression in the control T cell population characteristic of the T cells from which the population of manufactured T cells was produced. It should be understood that the expression levels for the checkpoint inhibitors are compared between the same cell types, e.g. a CD4+ manufactured T cell would be compared to a CD4+ T-Rapa cell or CD4+ control T cell characteristic of the T cells from which the manufactured T cell was produced.

In some embodiments, a manufactured T cell can have reduced expression of one or more checkpoint inhibitor receptors selected from CD39, CD73, GITR, LAG3, PD1, 2B4, LAIR1, CTLA4, KLRG1, TIGIT and TIM3. By way of example, but not limitation, the reduced expression can be at least 25% less than a corresponding level of expression in a T-Rapa cell. By way of further example, but not limitation, the reduced expression can be at least 25%, 50%, 75%, 80%, 85%, 90%, 95% or 99% less than a corresponding level of expression in a T-Rapa cell. In some embodiments, a manufactured T cell can have a level of expression of one or more checkpoint inhibitors selected from CD39, CD73, GITR, LAG3, PD1, 2B4, LAIR1, CTLA4, KLRG1, TIGIT and TIM3 that is within about 25% of a corresponding level of expression in a control T cell characteristic of the T cells from which the manufactured T cell was produced. By way of example, but not limitation, the level of expression of the one or more checkpoint inhibitors selected from CD39, CD73, GITR, LAG3, PD1, 2B4, LAIR1, CTLA4, KLRG1, TIGIT and TIM3 can be within about 25%, 20%, 15%, 10%, or 5% of a corresponding level of expression in the control T cell characteristic of the T cells from which the manufactured T cell was produced. It should be understood that the expression levels for the checkpoint inhibitors are compared between the same cell types, e.g. a CD4+ manufactured T cell would be compared to a CD4+ T-Rapa cell or CD4+ control T cell characteristic of the T cells from which the manufactured T cell was produced.

In some embodiments, a manufactured T cell has increased expression of CD127 relative to a control T cell characteristic of the cells from which the manufactured T cell was produced. By way of example, but not limitation, this increase can be at least 10%, 20%, 30%, 40%, 50% or more.

In some embodiments a population of manufactured T cells can have at least 5% of CD4+ T cells that express CD127 as measured by flow cytometry. By way of example, the population of manufactured T cells can have at least 5%, 6%, 7%, 8%, 9%, 10% or more of CD4+ T cells that express CD127 as measured by flow cytometry. In some embodiments a population of manufactured T cells can have an increased frequency of CD4+ T cells that express CD127 relative to a control population of T cells characteristic of the cells from which the population of manufactured T cells was produced. By way of example, but not limitation, the increase can be at least 50%, 100%, 150%, 200%, 300% or more.

To the extent that any of the foregoing properties associated with populations of manufactured T cells can be associated with a single cell, a manufactured T cell can be characterized by said properties. In any of the foregoing embodiments, a manufactured T cell or population of manufactured T cells can have more than one of the recited properties.

### Method for treating cancer in a subject

Patients with relapsed multiple myeloma (MM) have limited survival and curative therapy has been elusive. As such, patients with relapsed MM are suitable for a novel T cell therapy.

The immune therapy differs from existing approaches of immune therapy in several important categories. First, the manufactured T cell product is manufactured to be inhibited at the level of the mammalian target of rapamycin (mTOR) pathway, which translates into resistance to apoptosis and into enrichment for central memory differentiation. Second, the manufactured T cell product is manufactured in high-dose IFN-alpha, which promotes a CD4⁺Th1 and CD8⁺Tc1 differentiation. Third, the manufactured T cell product is minimally co-stimulated with monoclonal antibodies or not co-stimulated and expresses a diverse T cell receptor (TCR) repertoire; as such, anti-tumor effects mediated by manufactured T cells are anticipated to occur primarily through in vivo clonal expansion to tumor antigens. Such a mechanism may be favorable in multiple myeloma, where tumor antigens are either not known or are variable over time due to a high tumor mutational rate. Characterization of in vivo emergent T cell responses will be critical to improving an understanding of potential mechanisms of manufactured T cell therapy and will be evaluated as a secondary objective on this study. And fourth, we will evaluate the manufactured T cell therapy on a novel immune-depleting and immune-suppressing regimen that consists of pentostatin combined with low-dose, dose-adjusted cyclophosphamide (PC regimen). This PC regimen is relatively sparing of myeloid cells, thereby permitting repeat therapeutic cycles without substantial neutropenia; this regimen is advantageous in terms of cost (can be administered in the outpatient setting) and safety (reduced rate of infection due to myeloid cell preservation). Multiple myeloma is a disease that is critically promoted by inflammatory signaling; as such, the inflammatory inhibition mediated by the PC regimen will be one component contributing to the regimen efficacy. Each of these factors was considered during the design of the clinical trial, which focuses on multiple infusions of manufactured T cells after PC conditioning.

The manufactured T cells express greatly reduced levels of the checkpoint inhibitors, and thus offers a novel ex vivo method to release the immune system from checkpoint inhibition, which is currently achieved through monoclonal antibody therapy. Along this line, it is anticipated that the manufactured T cell therapy will meet with success in cancers that are susceptible to checkpoint inhibitor therapy, including but not limited to: melanoma, renal cell carcinoma, bladder cancer, lung cancer, lymphoma, multiple myeloma, and colon cancer. It should also be noted that checkpoint inhibitor monoclonal antibody therapy has been relatively toxic in multiple myeloma patients, thereby creating a need for an alternative approach to avoid immune checkpoints, such as the manufactured T cell therapy.

In addition, the ability of manufactured T cells to undergo extensive clonal expansion to a wide variety of tumor antigens predicts that tumor cells with an increased mutational rate and tumors with micro-satellite instability will be particularly sensitive to manufactured T cell therapy.

The present disclosure provides for methods for treating cancer that include administering manufactured T cells of the present disclosure at a therapeutically effective dose.

In some embodiments, the method for treating cancer comprises administering to said subject a composition comprising manufactured T cells at a therapeutically effective dose. In some embodiments, administration of the composition comprising manufactured T cells can be repeated either at a therapeutically effective dose or to cumulatively achieve a therapeutically effective dose. In some embodiments, the method further comprises harvesting autologous cells from said subject, prior to subjecting said subject to said immune depletion regimen. In some embodiments, the method further comprises harvesting autologous cells from said subject prior to administering said composition comprising manufactured T cells to said subject.

In any of the foregoing embodiments, said immune depletion regimen can include administering to said subject at least one of pentostatin and cyclophosphamide. In some embodiments, pentostatin is administered to said subject, and wherein a dose of said pentostatin can be between 0.5-4 mg/m², 0.5-3 mg/m², 0.5-2 mg/m², 0.5-1 mg/m², 1-4 mg/m², 2-4 mg/m², or 3-4 mg/m². By way of non-limiting example, a dose of said pentostatin is about 0.5 mg/m², 1 mg/m², 1.5 mg/m², 2 mg/m², 2.5 mg/m², 3 mg/m², 3.5 mg/m², or 4 mg/m². In some embodiments, cyclophosphamide is administered to said subject, and wherein a dose of said cyclophosphamide can be between 50-400 mg, 50-300 mg, 50-200 mg, 50-100 mg, 100-400 mg, 200-400 mg, 300-400 mg, 200-300 mg, or 100-200 mg. By way of non-limiting example, a dose of said cyclophosphamide is about 50 mg, 100 mg, 150 mg, 200 mg, 250 mg, 300 mg, 350 mg, or 400 mg. In some embodiments, both pentostatin and cyclophosphamide are administered to said subject. In some embodiments, said pentostatin and cyclophosphamide are administered to said subject in a single composition. In some embodiments, said single composition is administered intravenously to said subject.

In any of the foregoing embodiments, said immune depletion regimen can include administering to said subject a first composition comprising pentostatin; and administering to said subject a second composition comprising cyclophosphamide. In some embodiments, said first composition is administered at a dose of 1-4 mg/m2 of pentostatin is between 0.5-4 mg/m², 0.5-3 mg/m², 0.5-2 mg/m², 0.5-1 mg/m², 1-4 mg/m², 1-3 mg/m², 1-2 mg/m², or 3-4 mg/m². By way of non-limiting example, a dose of said pentostatin is about 1 mg/m², 1.5 mg/m², 2 mg/m², 2.5 mg/m², 3 mg/m², 3.5 mg/m², or 4 mg/m². In some embodiments, said second composition comprises cyclophosphamide and is administered at a dose of said cyclophosphamide between 50-400 mg, 50-300 mg, 50-200 mg, 50-100 mg, 100-400 mg, 200-400 mg, 300-400 mg, 200-300 mg, or 100-200 mg. By way of non-limiting example, a dose of said cyclophosphamide is about 50 mg, 100 mg, 150 mg, 200 mg, 250 mg, 300 mg, 350 mg, or 400 mg.

In any of the foregoing embodiments, said therapeutically effective dose can be 1 x 10⁵ to 5 x 10⁶, 1 x 10⁶ to 2.5 x 10⁶ cells/kg, 2.5 x 10⁶ to 5 x 10⁶ cells/kg, 1 x 10⁵ to 2.5 x 10⁶ cells/kg, 2.5 x 10⁵ to 5 x 10⁶ cells/kg, 1 x 10⁵ to 2.5 x 10⁵ cells/kg, 2.5 x 10⁵ to 5 x 10⁵ cells/kg, 1 x 10⁵ cells/kg, 2 x 10⁵ cells/kg, 3 x 10⁵ cells/kg, 4 x 10⁵ cells/kg, 5 x 10⁵ cells/kg,1 x 10⁶ cells/kg, 2 x 10⁶ cells/kg, 3 x 10⁶ cells/kg, 4 x 10⁶ cells/kg, or 5 x 10⁶ cells/kg manufactured T cells per kg of the subject's body weight. In any of the foregoing embodiments, the composition comprising manufactured T cells is administered to said subject by infusion.

In any of the foregoing embodiments, the cancer can be, by way of example but not limitation, selected from the group consisting of multiple myeloma, renal cell carcinoma, bladder cancer, lung cancer, liver cancer, lymphoma, gastric cancer and colon cancer. In some embodiments, the cancer is multiple myeloma. In some embodiments, the multiple myeloma is relapsed multiple myeloma. By way of further example, but not limitation, the cancer can be sarcoma, pancreatic cancer, prostate cancer, ovarian cancer, breast cancer or colorectal cancer. In some embodiments, the cancer is a PDL1-negative cancer. In some embodiments, the cancer is susceptible to checkpoint inhibitor therapy. In some embodiments, the multiple myeloma is relapsed, refractory multiple myeloma. In some embodiments, the multiple myeloma is quad- or penta-refractory multiple myeloma. In some embodiments, the multiple myeloma is smoldering multiple myeloma. In some embodiments, said subject is suffering from multiple myeloma that has relapsed. In certain aspects, the subject is suffering from multiple myeloma that has relapsed, by way of example but not limitation, 1, 2, 3, 4, 5, 6, 7, 8, 9, 10 or more times. In some embodiments, said subject is suffering from smoldering multiple myeloma. In some embodiments, said subject is suffering from quad- or penta-refractory multiple myeloma. In some embodiments, said subject is suffering from multiple myeloma that is refractory to, by way of example but not limitation, 1, 2, 3, 4, 5 or more treatments.

In some embodiments, said subject has previously been treated and is now at the time of second or third relapse after having received different lines of treatment selected from the group consisting of administration of a proteasome inhibitor, administration of immune modulatory drugs, administration of alkylators, administration of CD38 monoclonal antibodies, and administration of glucocorticoids. This patient population is considered suitable for evaluation of a phase 3 randomized clinical trial, where randomization to a control cohort to receive standard chemotherapy for patients in second or third relapse is justifiable.

In a separate embodiment, said subject is highly-refractory to multiple standard drugs and therefore, a randomized clinical trial is not justified. Rather, such highly refractory patients will be treated with the Rapa-T therapy on a single-arm, phase II clinical trial. Highly refractory status can be quantified by the nomenclature quad- or penta-refractory, whereby such a subject is refractory to either 4 or 5 of the top drugs used to treat multiple myeloma, namely: bortezomib, carfilzomib, lenalidomide, pomalidomide, and daratumumab.

In the case of the embodiment relating to the phase 3 clinical trial of treating MM at the time of second or third relapse, the primary study objective will relate to progression-free survival as the study endpoint, with progression-free status being defined as said subject has less than a 25% increase in M-protein/free light chain difference when monitored on a monthly basis.

In some embodiments, the first treatment cycle is a minimum of 28 days in duration. In some embodiments, each of said one or more additional treatment cycles is a minimum of 35 days in duration.

In some embodiments, said step of administering pentostatin to said subject is repeated during the first treatment cycle. In some embodiments, said step of administering pentostatin to said subject is performed on days 1, 4, 8, and/or 11 of said first treatment cycle. In some embodiments, said step of administering cyclophosphamide to said subject is repeated during the first treatment cycle. In some embodiments, said step of administering cyclophosphamide to said subject is performed on days 1, 2, 3, 4, 5, 8, 9, 10, 11 and/or 12 of the first treatment cycle.

In any of the above embodiments, each of said one or more additional treatment cycles are separated by 0 to 4 weeks. In any of the above embodiments, said first treatment cycle and a first of said one or more additional treatment cycles are separated by 0 to 4 weeks. In any of the above embodiments, said step of administering to said subject a composition comprising manufactured T cells at a therapeutically effective dose is performed on day 15, 16, 17 or 18 of each of said one or more additional treatment cycles.

In some embodiments, said subject is in the second or third relapse of MM after having received regimens consisting of administration of a proteasome inhibitor, administration of immune modulatory drugs, administration of alkylators, administration of CD38 monoclonal antibodies, and administration of glucocorticoids.

In some embodiments, said subject is in a late stage of MM relapse and quad- or penta-refractory whereby standard therapies do not exist.

In some embodiments relating to a phase 3 clinical trial, the primary study objective will relate to progression-free survival, with progression-free being defined as less than a 25% increase in M-protein/free light chain difference between treatments.

In some embodiments, the method comprises subjecting said subject to an immune depletion regimen to reduce at least a portion of regulatory T cells and/or end-stage senescent effector T cells or to reduce at least a portion of the function of regulatory T cells and/or end-stage senescent effector T cells; and administering to said subject a composition comprising manufactured T cells at a therapeutically effective dose after said immune depletion regimen.

In some embodiments, said immune depletion regimen comprises: administering pentostatin to said subject; and administering cyclophosphamide to said subject; administering one or more additional doses of pentostatin to said subject if said subject's creatine clearance is ≥ 30 mL/min/1.73 m²; administering one or more additional doses of cyclophosphamide to said subject if said subject's absolute lymphocyte count is 50 per microliter or greater and said subject's absolute neutrophil count is 500 per microliter or greater.

In some embodiments, said step of measuring the CrCl of said subject and adjusting a dose of pentostatin to be administered is performed on days 1, 4, 8, and/or 11 of said immune depletion regimen.

In some embodiments, said step of measuring ALC and ANC and adjusting a dose of cyclophosphamide to be administered is performed on days 1, 2, 3, 4, 5, 8, 9, 10, 11 and/or 12 of the immune depletion regimen.

In some embodiments, said step of administering to said subject a composition comprising manufactured T cells at a therapeutically effective dose after said immune depletion regimen is performed 15-18 days after the start of the immune depletion regimen.

In any of the foregoing embodiments, the steps subjecting said subject to an immune depletion regimen to reduce at least a portion of regulatory T cells and/or end-stage senescent effector T cells or to reduce at least a portion of the function of regulatory T cells and/or end-stage senescent effector T cells and administering to said subject a composition comprising manufactured T cells at a therapeutically effective dose after said immune depletion regimen are repeated at least twice. In any of the foregoing embodiments, the steps subjecting said subject to an immune depletion regimen to reduce at least a portion of regulatory T cells and/or end-stage senescent effector T cells or to reduce at least a portion of the function of regulatory T cells and/or end-stage senescent effector T cells and administering to said subject a composition comprising manufactured T cells at a therapeutically effective dose after said immune depletion regimen can be repeated up to 8 times or more. In any of the foregoing embodiments, each step of administering to said subject a composition comprising manufactured T cells at a therapeutically effective dose after said immune depletion regimen can be separated by 0 to 9 weeks

It should also be understood that, in any of the foregoing embodiments, where co-stimulation by anti-CD3/anti-CD28 antibodies is performed, this co-stimulation can be provided in any form of anti-CD3/anti-CD28 antibodies. By way of example, but not limitation, where co-stimulation is indicated as being performed by using anti-CD3/anti-CD28 beads, anti-CD3/anti-CD28 nanoparticles or microparticles can be used

### EXAMPLES

The following examples are provided to better illustrate the methods of the present disclosure and the resultant manufactured T cells. These examples are not intended to be limiting or to otherwise alter the scope of the methods, cells and compositions disclosed in the present disclosure.

### Example 1

*Use of Anti-IL-2 Receptor Blockade and mTOR Blockade for Th1 Enrichment.* For adoptive T cell therapy, it is important to manufacture T cells that preferentially express a Th1 phenotype, with minimal contamination from cells with a regulatory T (T_{REG}) cell phenotype. Th1-type cells can be characterized in part by their expression of the cell fate transcription factor TBET, whereas T_{REG} cells express the FoxP3 transcription factor.

Methods for promoting TBET while limiting FoxP3 were evaluated. We evaluated the mTOR inhibitor temsirolimus, which is an FDA-approved medication administered intravenously for therapy of refractory renal cell carcinoma. The use of an mTOR inhibitor to manufacture T cells enriched for a Th1 phenotype may seem paradoxical, as inhibition of mTOR has, in general, been associated with the promotion of T cells of a T_{REG} phenotype. The current experiments differ from the prior research because temsirolimus is an intra-venous formulation, which thereby is advantageous relative to rapamycin, which is less feasible for cell culture due to limited solubility in media.

The current experiments also differ from the past research because we evaluated the combination of temsirolimus and the anti-IL-2 receptor monoclonal antibody, daclizumab. Daclizumab and basiliximab are both FDA-approved monoclonal antibodies that have a common mechanism of action and therefore can be used inter-changeably in the system we have developed.

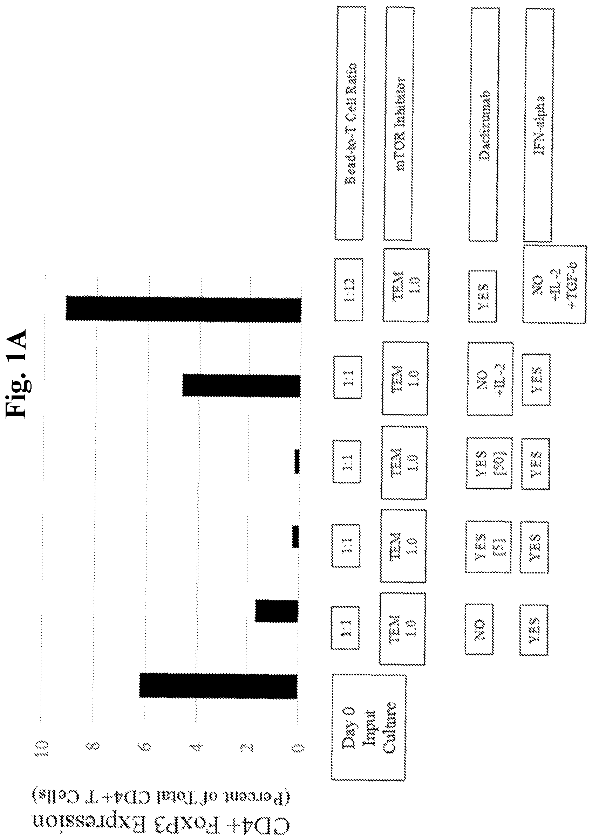

T cells were cultured ex vivo, as indicated, using various ratios of anti-CD3, anti-CD28 beads to T cells (1:1 or 1:12); the mTOR inhibitor temsirolimus (1 µM); without or with the anti-IL-2 receptor monoclonal antibody daclizumab (5 or 50 µg/ml); and either Th1 polarization cytokines (IFN-alpha; 10,000 IU/ml) or control regulatory T cell polarization (IL-2 plus TGF-β). At day 6 of culture, the T cells were evaluated for intra-cellular expression of the regulatory T cell transcription factor FoxP3 and the Th1 transcription factor TBET. (FIGURES 1A-1B).

It was found that addition of temsirolimus (1.0 µM concentration) in the setting of the type I polarizing cytokine IFN-α reduced the resultant T cell expression of FoxP3 relative to the day 0 input T cell population (see FIGURE 1A). Furthermore, we found that blockade of the IL-2 receptor further reduced FoxP3 expression; use of the antibody at 50 µg/ml was more effective than use of the antibody at 5 µg/ml, thereby indicated a dose-response relationship. Temsirolimus was not effective at reducing FoxP3 expression if exogenous IL-2 was added in combination with IFN-α (FIGURE 1A); furthermore, temsirolimus and daclizumab were not effective at limiting FoxP3 expression if the culture conditions were permissive for T_{REG} cell differentiation, namely: reduction in the bead ratio to 1:12; elimination of IFN-α from culture; and addition of exogenous IL-2 plus TGF-β (FIGURE 1A).

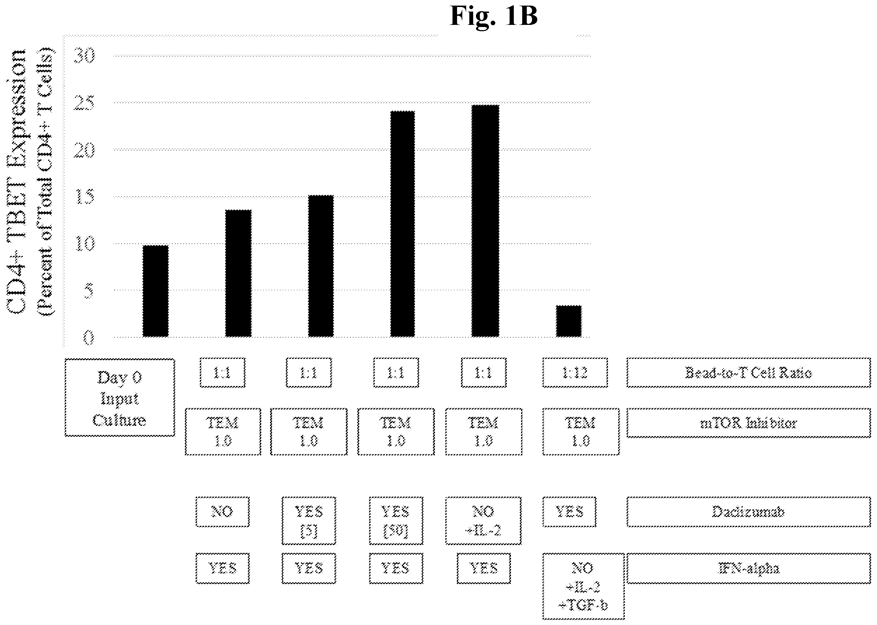

In addition to limiting FoxP3 expression, the combination of temsirolimus and an anti-IL-2 receptor monoclonal antibody was effective at promoting the Th1 phenotype, as indicated by increased expression of TBET (FIGURE 1B). Again, there was a dose response relationship, with 50 µg/ml daclizumab promoting TBET expression to a higher degree than 5 µg/ml (FIGURE 1B). Although addition of IL-2 to the IFN-α polarization increased TBET, this was also associated with an increase in FoxP3, thereby indicated a lack of Th1-purity through exogenous IL-2 addition. Of note, the T_{REG} control condition-IL-2 and TGF-β-as expected, had reduced expression of TBET (FIGURE 1B).

As such, the combination of mTOR inhibition and IL-2 receptor blockade represents a new method for Th1 cell generation.

Development of a Combination of Interventions to Inhibit T Cells During Ex Vivo Manufacturing: mTOR Inhibition; IL-2 Receptor Blockade; Reduced T Cell Co-stimulation; and Inhibition of T Cells Prior to Co-stimulation (Overnight Pre-Incubation). Given these results demonstrating that the combination of mTOR inhibition and IL-2 receptor blockade can improve the manufacturing of Th1-type cells (improvement in the TBET-to-FoxP3 ratio; limitation of T_{REG} cell contamination), we considered that additional interventions might also improve Th1 cell manufacturing.

One benefit to the use of mTOR inhibition for adoptive T cell therapy efforts is that this intervention can promote the manufacture of T cells with a more primitive differentiation status, such as the T central memory subset (T_{CM}) or the T stem cell memory subset (T_{SCM}). In prior research using ex vivo rapamycin, it was found that ex vivo rapamycin was effective for the manufacture of T cells of T_{CM} phenotype; this result is consistent with the known role of mTOR in the control of T cell memory status. It is important to promote the T_{CM} and/or T_{SCM} status during manufacturing because such T cells of more primitive differentiation status have increased long-term engraftment after adoptive transfer and mediate increased in vivo effects in experimental models. Several other methodologies have been described to promote the manufacture of T cells of limited differentiation status, including: use of a GSK3 inhibitor for promotion of WNT signaling; inhibition of AKT signaling; and inhibition of PI3 kinase signaling.

We developed a method whereby the T cells were plated and incubated in X-Vivo 20 media supplemented with 5% human AB serum which was devoid of exogenous cytokines and contained the mTOR inhibitor temsirolimus and the IL-2 receptor blockade via monoclonal antibody addition. This method incorporated an approximate 16-hour "pre-incubation" prior to co-stimulation with anti-CD3, anti-CD28 coated magnetic beads. To our knowledge, this method has not been previously reported.

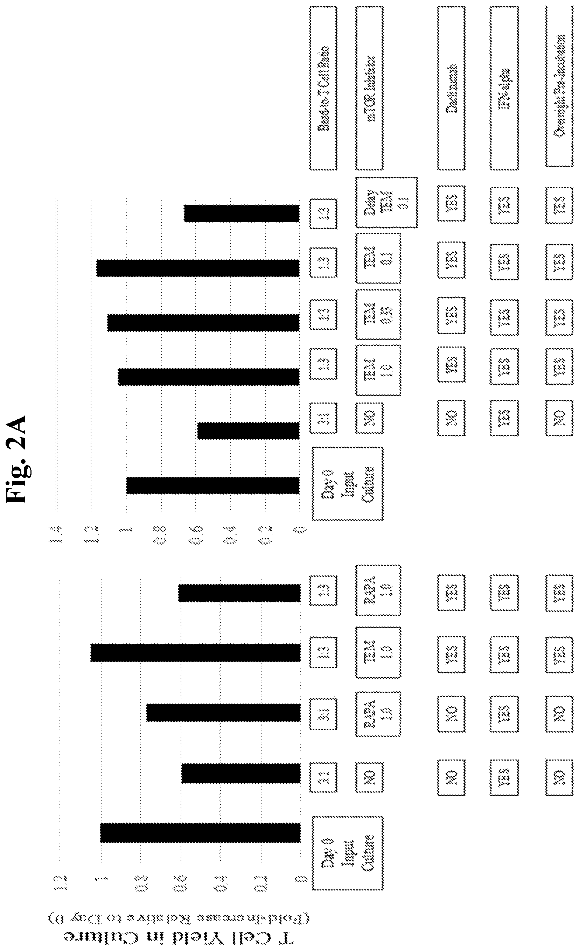

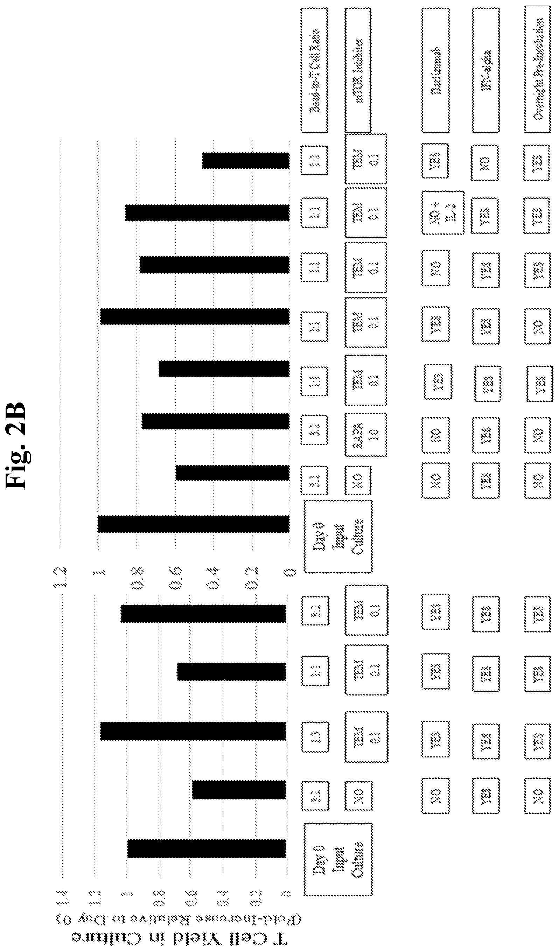

As a first step towards evaluating these increasingly stringent culture conditions, we evaluated whether the culture of CD4⁺ and CD8⁺ T cells under the various conditions would be feasible, as defined by the presence of viable cells at the end of the culture interval (for Th1 manufacturing, a 6-day culture interval). In FIGURES 2A-2B, CD4⁺ and CD8⁺ T cells were placed into culture under the various conditions, as indicated. The ratio of 3/28 beads to T cells was either 3:1, 1:1, or 1:3. The T cells were either co-stimulated at the same time as addition to culture ("No overnight pre-incubation") or after an overnight, 16-hour pre-incubation. In some conditions, the anti-IL-2 receptor monoclonal antibody daclizumab was added at a concentration of 50 µg/ml). For mTOR inhibition, temsirolimus was added either at 1.0 or 0.1 µM; alternatively, the control mTOR inhibitor rapamycin was added at a concentration of 1.0 µM. Most cultures also were supplemented with the type I cytokine promoting agent, IFN-α (10,000 IU/ml). As indicated, most of the culture conditions did not include the addition of exogenous IL-2. The T cell yield was calculated after 6-days in culture and compared to culture initiation ("Day 0 Input Culture").

As FIGURES 2A-2B illustrate, modification of the culture conditions to include not only mTOR inhibition (use of temsirolimus at either 1.0 µM or 0.1 µM; control use of rapamycin at 1.0 µM) and IL-2 receptor blockade (daclizumab) but also reduced co-stimulation (reduction from 3:1 beads-to-T cells to ratios of 1:1 and 1:3) and overnight pre-incubation resulted in similar numbers of viable T cells relative to control T cell cultures.

*Importance of Pre-Incubation and High-dose Temsirolimus in Th1*/*Tc1 Manufacturing.* At the time of cryopreservation of the Th1/Tc1 cell product (end of culture), it is important that the T cells have a relatively quiescent phenotype in addition to the T_{CM} phenotype. That is, we previously found that rapamycin-generated T cells secreted very small amounts of cytokines at the time of adoptive transfer but resulted in large amounts of cytokines in vivo; of note, others have identified a similar inverse correlation between cell product effector function (minimal) and in vivo effector function (maximal). T cell quiescence at the time of adoptive transfer can improve T cell survival post-transfer and may also be important for reducing the risk of cytokine release syndrome, which is a cause of morbidity and mortality after other forms of adoptive T cell therapy, particularly gene-modified chimeric-antigen-receptor (CAR) T cell therapy.

To evaluate this, we tested cytokine secretion potential of the manufactured T cells at the end of cell culture (day 6) and then again one week after ex vivo expansion after maximal co-stimulation (3/28 bead-to-T cell ratio, 3:1) and propagation in media devoid of any inhibitors.

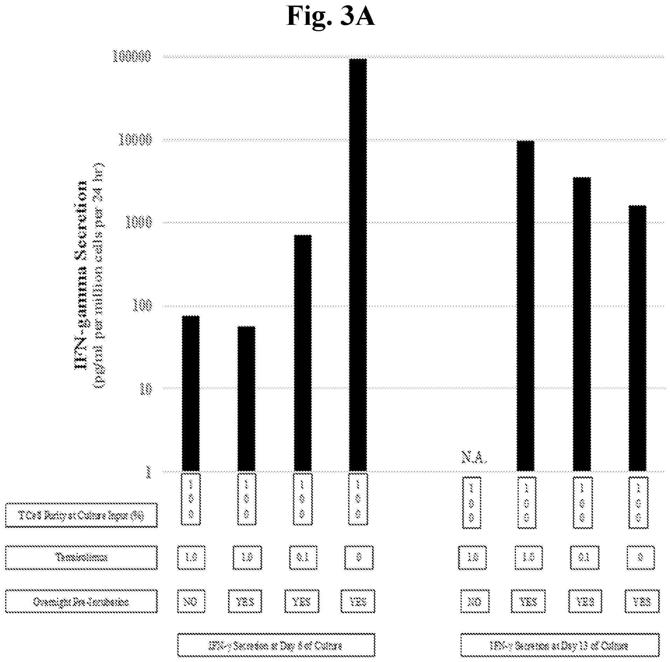

In FIGURES 3A-3B, CD4⁺ and CD8⁺ T cells were purified and cultured for 6-days either with or without a 16-hr interval of pre-incubation prior to co-stimulation (1:1 ratio of 3/28 beads-to-T cells). As indicated, temsirolimus was added at concentrations of either 1.0 or 0.1 µM; all cultures were supplemented with IFN-α (10,000 IU/ml) and daclizumab (50 µg/ml). On day 6 of culture, the resultant T cells were co-stimulated for 24 hr using a 3:1 ratio of 3/28 beads; the supernatant was tested for cytokine content by Luminex assay (results are described as pg per ml of cytokine secreted per million cells per 24 hr). In addition, the resultant T cells were co-stimulated with a 3:1 ratio of 3/28 beads and maintained for one week in culture using media that did not contain any exogenous cytokines or inhibitors; after this T cell culture, at day 13 of culture, the T cells were harvested, re-stimulated with 3/28 beads (3:1 ratio), and the 24 hr supernatant was evaluated as above for cytokine content. N.A, abbreviation indicates not applicable (insufficient T cell yield to perform assay).

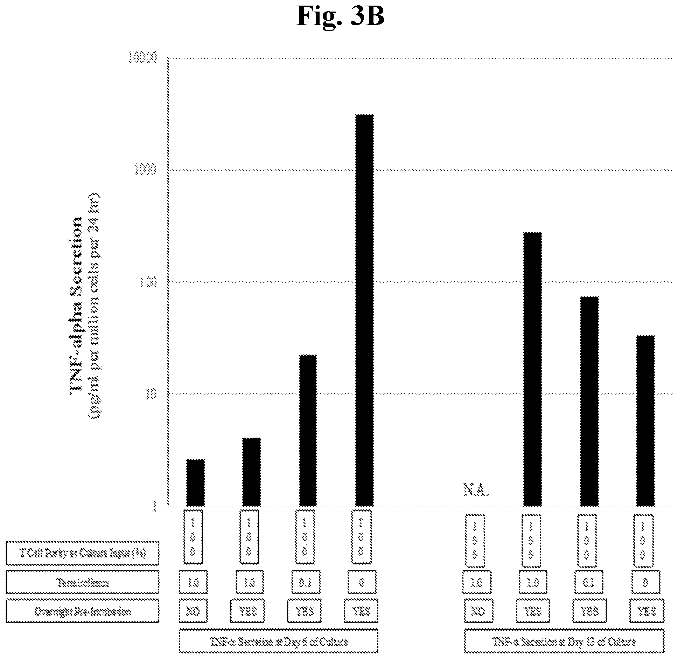

The results are shown in FIGURES 3A-3B, with IFN-γ secretion illustrated in FIGURE 3A and TNF-α secretion in FIGURE 3B. In each case, the day 6 T cell cytokine potential is shown in the left panels whereas the day 13 T cell cytokine potential is shown in the right panels.

These data indicate that high-dose temsirolimus (1.0 µM) resulted in the desired, very low levels of day 6 T cell IFN-γ and TNF-α secretion after 24 hr. of maximal co-stimulation in both the overnight pre-incubation condition and the condition without pre-incubation. Of note, use of temsirolimus at the concentration of 0.1 µM only partially reduced the day 6 T cell cytokine secretion potential. As such, temsirolimus in our method should be used at the higher concentration, that is, at least 1 µM. These data also indicate that the overnight pre-incubation intervention, when used alone, is not sufficient to yield the quiescent T cell phenotype. As such, the overnight pre-incubation step must be used in combination with high-dose temsirolimus to achieve the complete desired result.

In addition, this method results in the desired inverse relationship between initial T cell quiescence and resultant increased effector function after subsequent re-stimulation. That is, for both day 13 T cell values of IFN-γ and TNF-α secretion, the condition with the lowest level of day 6 cytokine potential (combination of pre-incubation plus high-dose temsirolimus) yielded the highest day 13 cytokine secretion values. Of note, the condition that consisted of high-dose temsirolimus without the overnight pre-incubation did not result in sufficient yield at day 13 of culture to evaluate cytokine secretion potential; as such, this result further confirms the value of the high-dose temsirolimus plus pre-incubation step for Th1/Tc1 cell generation.

*New Combinatorial Method Results in Enhanced mTOR Inhibition and Abrogation of STAT5 Phosphorylation.* The ability of the new combinatorial method (mTOR inhibition; IL-2 receptor blockade; pre-incubation for delay of co-stimulation; and use of a lower intensity of co-stimulation) to manufacture T cells of the desired phenotype resides in part upon its increased ability to control the molecular and cellular events that have been previously associated with the rapamycin-resistant T cell phenotype.

One component of this phenotype is the control of mTOR-dependent signaling events, such as that which occurs at the level of 4EBP1, which helps controls protein translation. To address this, we manufactured Th1/Tc1 cells using the T-Rapa method, which incorporated simultaneous T cell addition to culture with addition of high level co-stimulation (3/28 bead-to-T cell ratio, 3:1), high-dose rapamycin (1.0 µM), and cytokine addition (IL-2 plus IFN-α). In a side-by-side comparison, we manufactured T cells using a new combinatorial method of the present disclosure (16 hr pre-incubation step; co-stimulation at a reduced level (1:1 ratio); use of both temsirolimus (1.0 µM) and daclizumab (50 µg/ml); and addition of only IFN-α without IL-2.

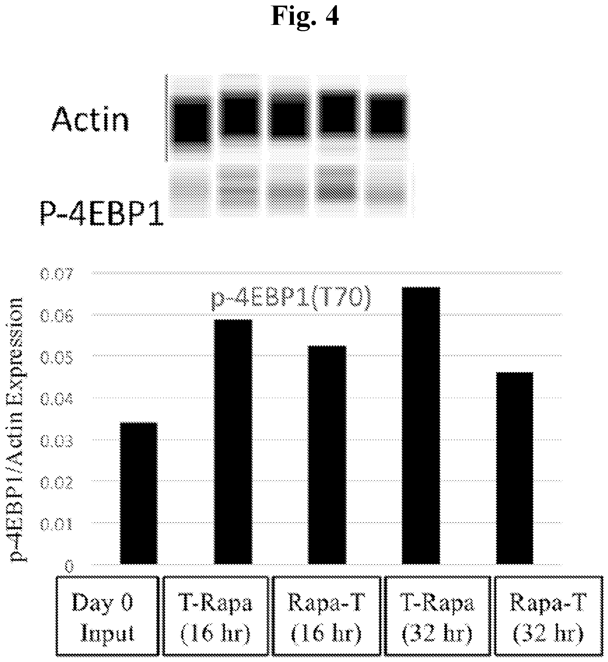

In FIGURE 4, CD4+ and CD8+ T cells were cultured using our previous methodology ["T-Rapa": simultaneous T cell addition to culture with addition of high level co-stimulation (3/28 bead-to-T cell ratio, 3:1), high-dose rapamycin (1.0 µM), and cytokine addition (IL-2 plus IFN-α)] or the new combinatorial methodology for manufactured T cells ["Rapa-T": 16 hr pre-incubation step; co-stimulation at a reduced level (1:1 ratio); use of both temsirolimus (1.0 µM) and daclizumab (50 µg/ml); and addition of only IFN-α without IL-2]. At 16-hr and 32-hr of T cell culture, a fraction of the T cells was harvested, protein was isolated, and western blot analysis of the housekeeping gene β-Actin and the mTOR pathway molecule phosphor-4EBP1 were quantified. Results were compared relative to protein obtained from the day 0 input T cells prior to any T cell activation.

As FIGURE 4 illustrates, the new combinatorial method for generating manufactured T cells ("Rapa-T" in FIGURE 4) resulted in reduced activation of the mTOR pathway (as measured by phosphorylation of 4EBP1) relative to T cells manufactured using our previously described method ("T-Rapa") at both 16-hr and 32-hour culture time points.

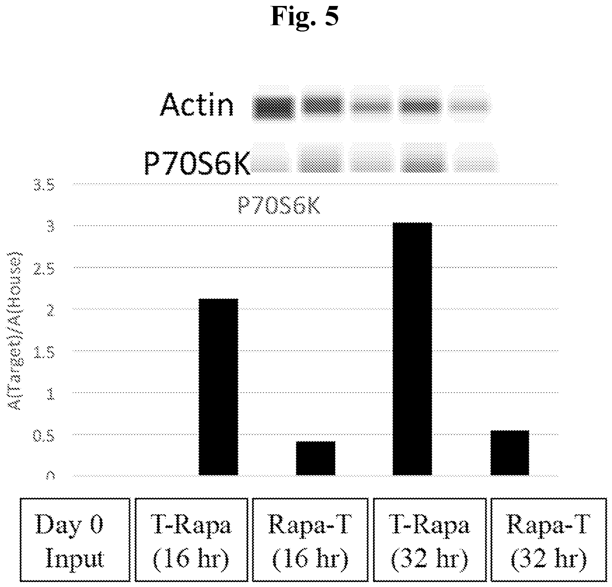

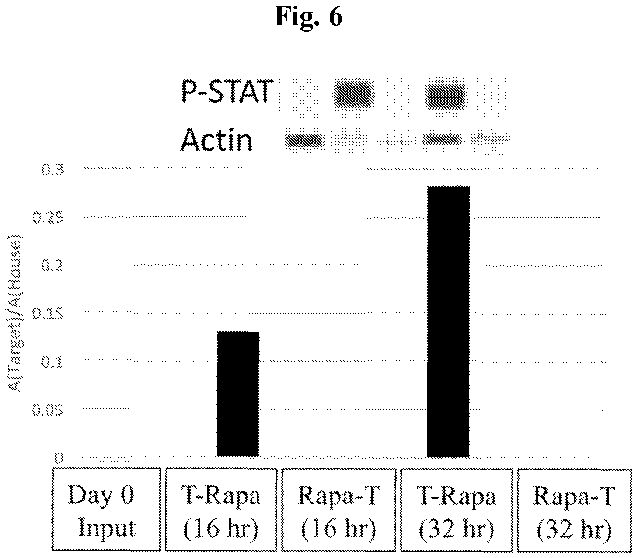

P70S6 kinase is an additional critical molecule in the mTOR pathway. In FIGURES 5 and 6, CD4⁺ and CD8⁺ T cells were cultured using our previous methodology ["T-Rapa": simultaneous T cell addition to culture with addition of high level co-stimulation (3/28 bead-to-T cell ratio, 3:1), high-dose rapamycin (1.0 µM), and cytokine addition (IL-2 plus IFN-α)] or the new combinatorial methodology for generating manufactured T cells ["Rapa-T": 16 hr pre-incubation step; co-stimulation at a reduced level (1:1 ratio); use of both temsirolimus (1.0 µM) and daclizumab (50 µg/ml); and addition of only IFN-α without IL-2]. At 16-hr and 32-hr of T cell culture, a fraction of the T cells was harvested, protein was isolated, and western blot analysis of the housekeeping gene β-Actin and the mTOR pathway molecule P70S6K (FIGURE 5) or phosphorylated STAT5 (FIGURE 6) were quantified. Results were compared relative to protein obtained from the day 0 input T cells prior to any T cell activation.

In marked contrast, manufacturing using the new combinatorial method for generating manufactured T cells resulted in greatly blunted levels of P70S6K (FIGURE 6, Rapa-T conditions). These data provide further evidence that the combinatorial method of Th1/Tc1 manufacturing provides improved control over mTOR activation. Using our previous methodology of manufacturing rapamycin-resistant T cells, we found substantial up-regulation of P70S6K at both 16-hr and 32-hr of T cell culture (FIGURE 5, T-Rapa conditions).

The combinatorial method was associated with improvement in the purity of Th1-type cells (reduction in contamination with FoxP3-expressing cells. The previous manufacturing method results in substantial STAT5 phosphorylation (FIGURE 6, T-Rapa results at 16-hr and 32-hr of culture); in marked contrast, the combinatorial approach abrogates STAT5 phosphorylation (FIGURE 6; Rapa-T results at 16-hr and 32-hr of culture).

As such, the new combinatorial method is also advantageous with respect to increased control of mTOR signaling during T cell manufacturing and abrogation of signaling events that promote contamination with T_{REG} cells (control of STAT5 phosphorylation).

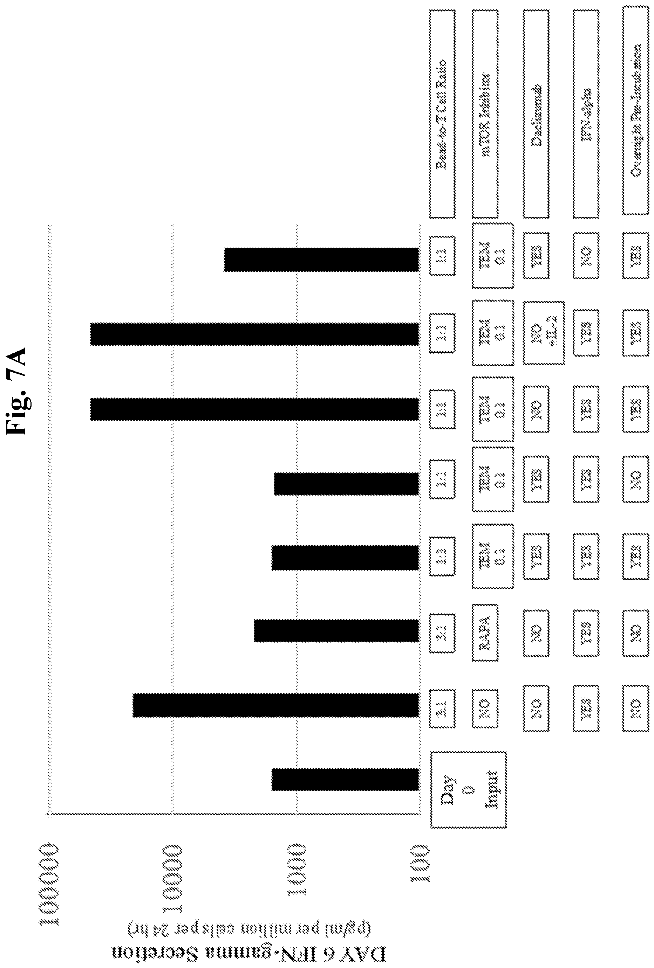

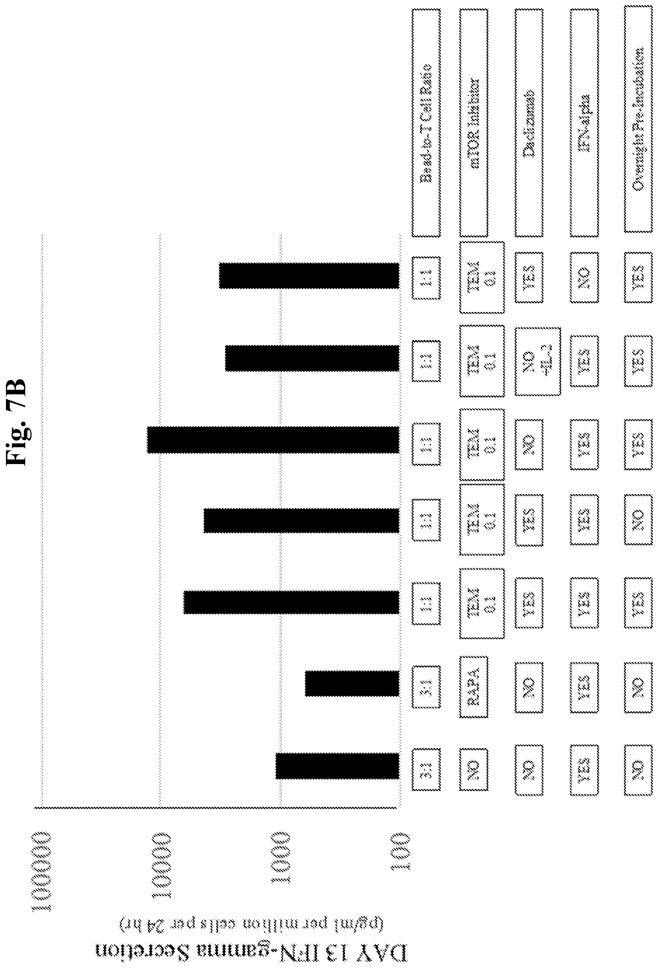

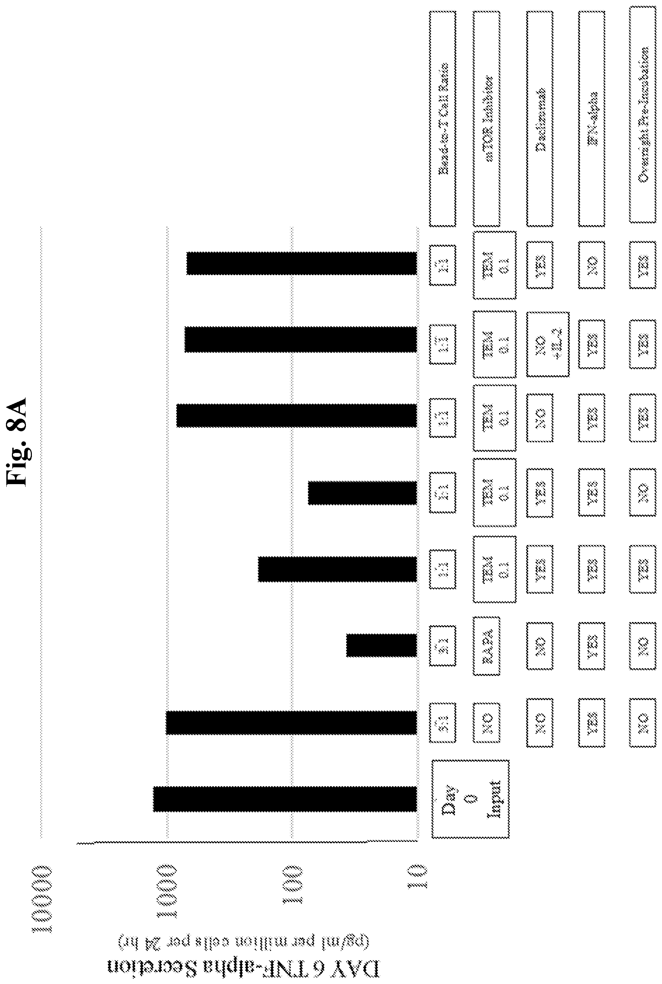

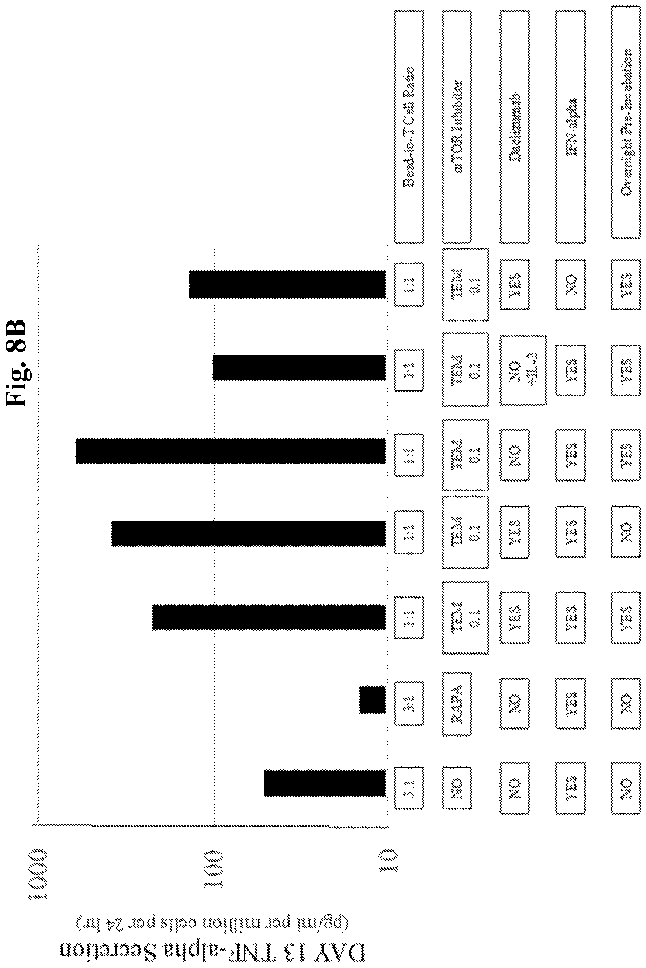

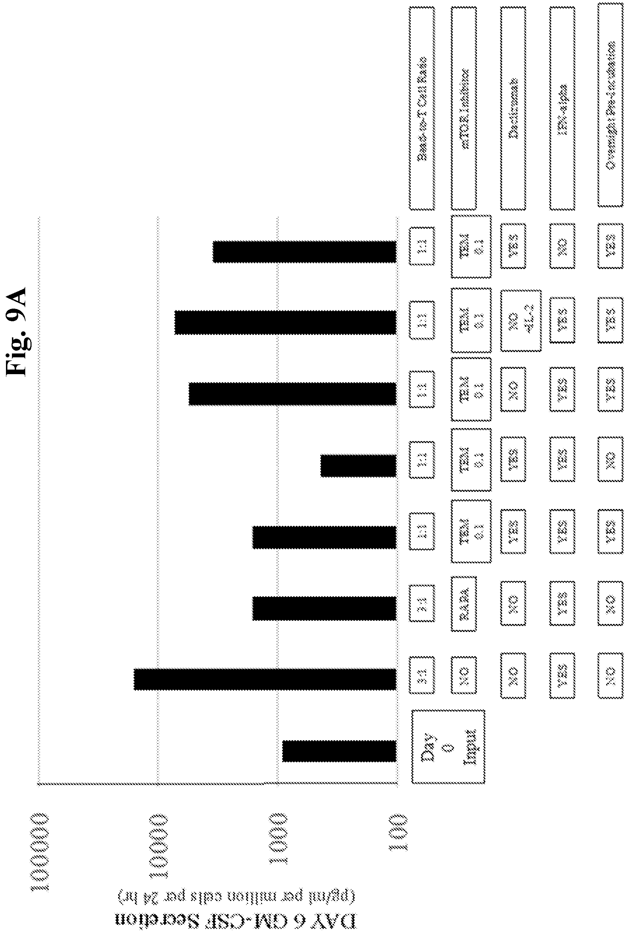

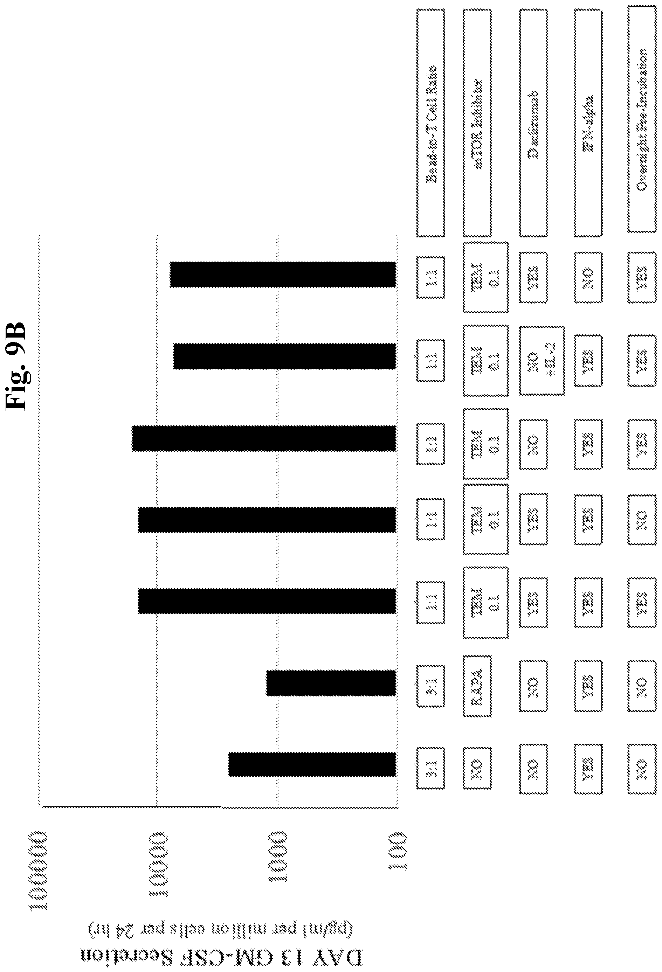

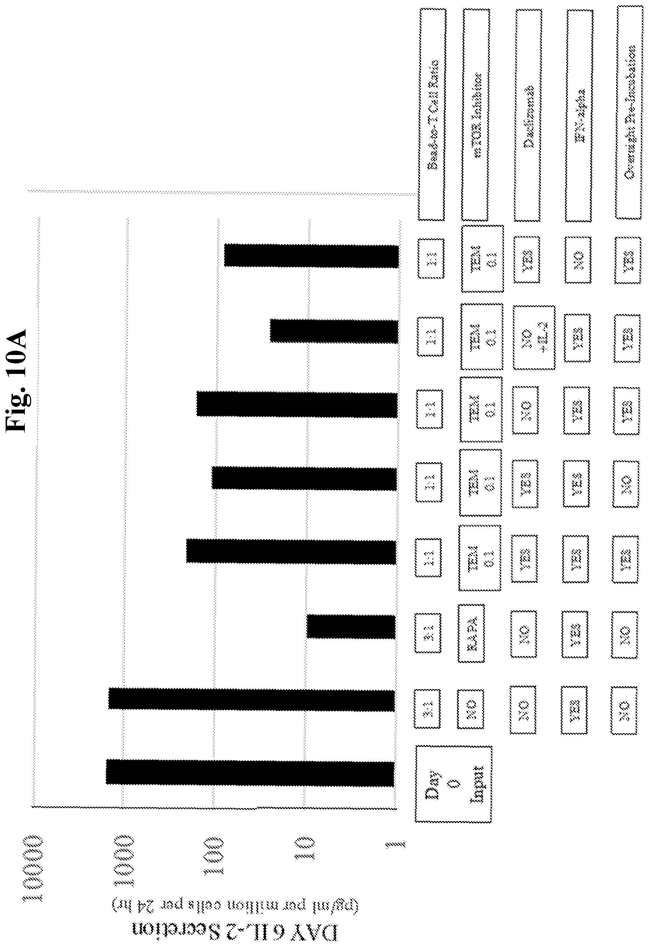

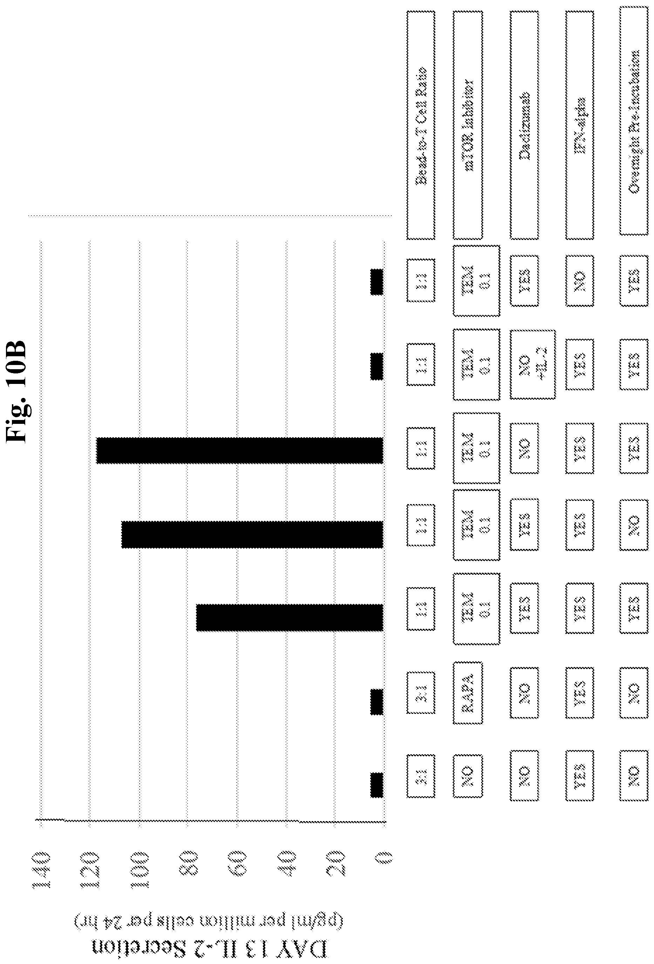

*Further Delineation of Individual Components of the Combinatorial Method of Th1*/*Tc1 Cell Generation.* Further cultures were established to gain additional information regarding the individual contribution of culture interventions to the resultant Th1/Tc1 phenotype. For FIGS. 7-10, CD4⁺ and CD8⁺ T cells were cultured, as indicated in FIGS. 7-10, using various 3/28 bead ratios, various methods of mTOR inhibition, variable addition of anti-IL-2 receptor blockade, variable addition of the type I polarizing cytokine IFN-α, and variable use of an initial overnight pre-incubation step. Supernatants generated by repeat co-stimulation (3:1 bead ratio) were collected at day 6 and day 13 of culture and tested for IFN-γ (FIGURES 7A-7B), TNF-α (FIGURES 8A-8B), GM-CSF (FIGURES 9A-9B), or IL-2 (FIGURES 10A-10B) content by Luminex assay (results expressed as pg per ml per million cells per 24 hr).

FIGURES 7A-7B show the IFN-γ secretion results at the end of culture (day 6) and one week after further culture in the absence of inhibitors (day 13). The desired phenotype is comprised of relatively low cytokine secretion values at day 6 with concomitant relatively high cytokine values at day 13. FIGURES 7A-7B demonstrate that the culture condition that includes each of the combined elements (low level of co-stimulation [1:1 ratio]; delayed co-stimulation after overnight pre-incubation; addition of the polarizing cytokine IFN-α; addition of the mTOR inhibitor [in this experiment, use of the sub-optimal concentration of 0.1 µM]; and inclusion of the anti-IL-2 receptor antibody daclizumab) had a desirable phenotype in that this condition was reduced in IFN-γ secretion at day 6 yet high in IFN-γ secretion at day 13. T cell culture conditions that omitted one or more of these elements tended to have higher cytokine values at day 6 and/or lower values at day 13. In addition, our previous method for manufacturing rapamycin-resistant T cells (T-Rapa; FIGURES 7A-7B) expressed a less favorable pattern of cytokine secretion (higher values at day 6; lower values at day 13).

The combinatorial method of Th1/Tc1 manufacturing also yielded a favorable cytokine phenotype (reduced values at day 6 combined with increased values at day 13) when the following cytokines were evaluated: TNF-α (FIGURES 8A-8B); GM-CSF (FIGURES 9A-9B); and IL-2 (FIGURES 10A-10B).

Taken together, these results provide further evidence that the new method of manufactured T cell manufacturing has important advantages relative to the prior T-Rapa method.

*Molecular Changes Associated with* Th1/Tc1 *Cell Manufacturing Using the Combinatorial Methodology.* We performed additional experiments to characterize molecules that are altered during Th1/Tc1 cell manufacturing using the combinatorial methodology. Such information is valuable not only because it can lead to an improved understanding of the T cell phenotype but also because such information can be utilized during manufacturing as a quality control element. In addition, such information can be used during the screening of additional combinatorial steps that might be used in future manufacturing efforts.

In previous efforts, we found that rapamycin-resistant T cells underwent autophagy during T cell manufacturing. It has long been known that autophagy is a direct result of mTOR inhibition: when mTOR is activated, T cells maintain a growth and proliferation state (autophagy signals are turned off); in contrast, when mTOR is inhibited, autophagy is promoted, thereby resulting in a reduction in T cell volume, including a reduction in mitochondrial volume (mitophagy). Indeed, autophagy is a necessary homeostatic process in T cell biology and is associated with T cell health, as energy requirements can be reduced and intra-cellular organelles and other cellular debris can be eliminated.

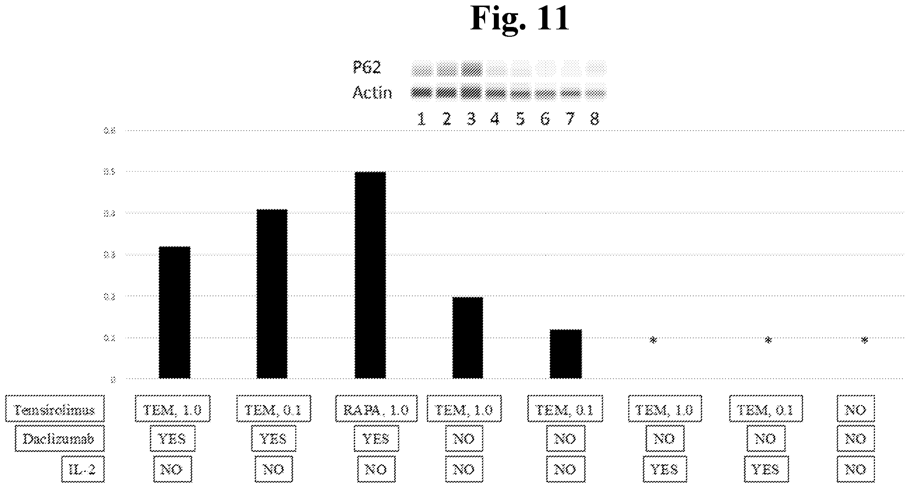

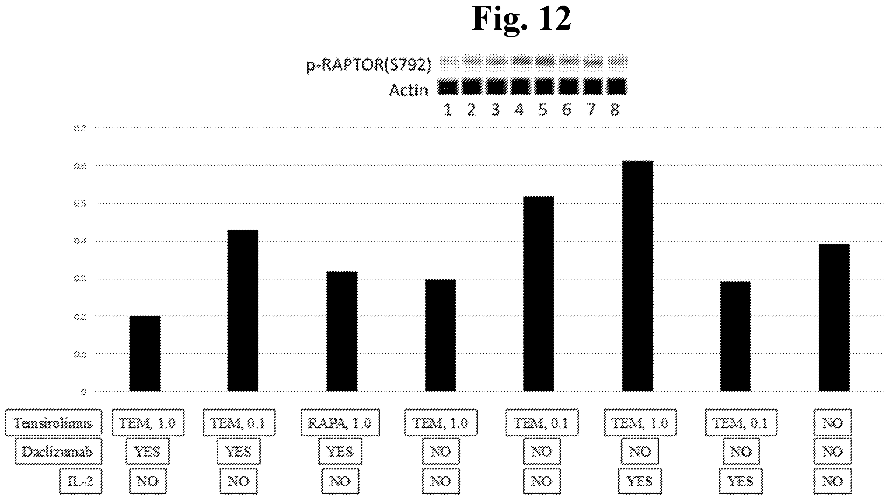

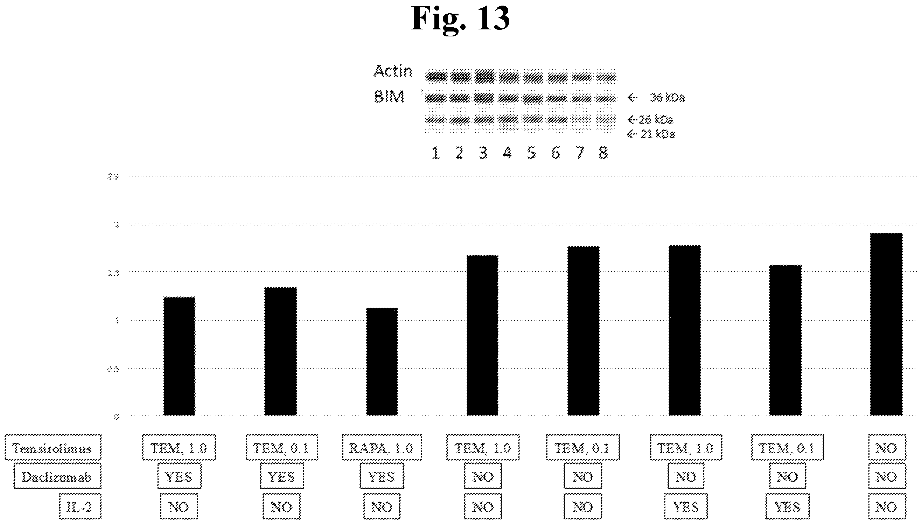

For FIGURES 11-13, human CD4⁺ and CD8⁺ T cells were cultured using a 16-hr pre-incubation interval prior to the addition of 3/28 beads at the reduced ratio of 1:3 beads-to-T cells. As indicated in FIGS. 11-13, the various T cell cultures received different methods of mTOR inhibition (rapamycin at 1.0 µM; temsirolimus at 1.0 or 0.1 µM), different conditions of exogenous IL-2 addition, and different conditions relative to addition of the anti-IL-2 receptor monoclonal antibody, daclizumab. After the 16-hr pre-incubation interval, cells were harvested from the T cell cultures, protein was isolated, and p62 (FIGURE 11), phospho-RAPTOR (FIGURE 12), or BIM (FIGURE 13) and the housekeeper gene β-actin were quantified by western blot.

We evaluated the ability of the combinatorial method to promote autophagy, as measured by T cell expression of the autophagy marker, p62. As FIGURE 11 indicates, the combinatorial method that included a pre-incubation step, a low level of co-stimulation (1:3 ratio of 3/28 beads to T cells), daclizumab blockade of the IL-2 receptor, and an mTOR inhibitor upregulated the autophagy marker p62. If each of these factors was present, autophagy could be realized by mTOR inhibition with rapamycin (1 µM) or temsirolimus (1.0 or 0.1 µM). Elimination of IL-2 receptor blockade from the regimen substantially blunted the induction of autophagy.

In addition, the combinatorial method also resulted in improved inhibition of the mTOR pathway, as indicated by reduced expression of the phosphorylated form of RAPTOR (FIGURE 12). Of note, the combinatorial method that incorporated high-dose temsirolimus yielded lower levels of phospho-RAPTOR compared to use of low-dose temsirolimus or high-dose rapamycin.

We also evaluated manufactured T cell expression of a pro-apoptotic member of the bcl-2 gene family, BIM. The bcl-2 family members operate primarily at the level of the mitochondria, and as such, mitochondrial autophagy (mitophagy) can influence the balance of bcl-2 family member genes, which help determine apoptosis threshold. Mitophagy has been shown to be beneficial in terms of reducing the apoptosis threshold, potentially be selectively eliminating mitochondria with unfavorable balances of the bcl-2 family of molecules. We found that the combinatorial method of manufacturing resulted in reduced expression of BIM (FIGURE 13).

In sum, these experiments indicate that enhanced autophagy, reduced mTOR signaling, and reduced pro-apoptotic molecule expression are associated with the combinatorial method of Th1/Tc1 cell manufacturing. These changes likely contribute to the increased in vivo function of the manufactured T cells, and as such, can be used as quality control steps or to screen future, next-generation methods of T cell manufacturing.

The Combinatorial Method Promotes T Cell Quiescence, T Cell De-differentiation. We performed additional experiments to characterize the surface phenotype of Th1/Tc1 cells manufactured by the combinatorial method, as defined by the incorporation of a pre-incubation step, a low level of co-stimulation (1:3 ratio of 3/28 beads to T cells), daclizumab blockade of the IL-2 receptor, and an mTOR inhibitor. First, we evaluated the effect of culture variables on T cell expression of the CD45 isoform RA, which is a marker of T cell naivety, including T cells of the stem cell memory subset. As such, development of a T cell manufacturing method that preserves or increases expression of CD45RA is desirable.

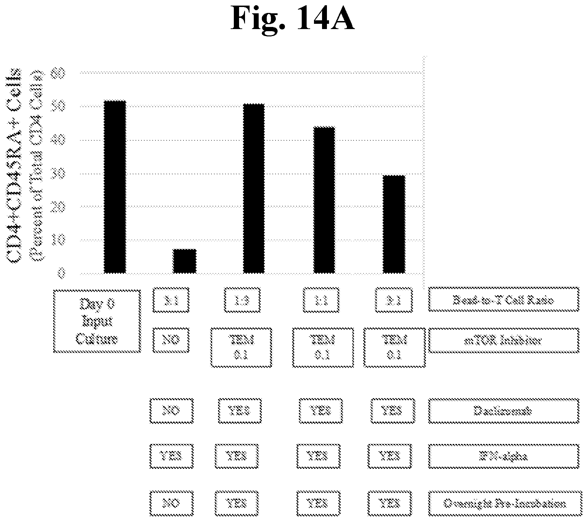

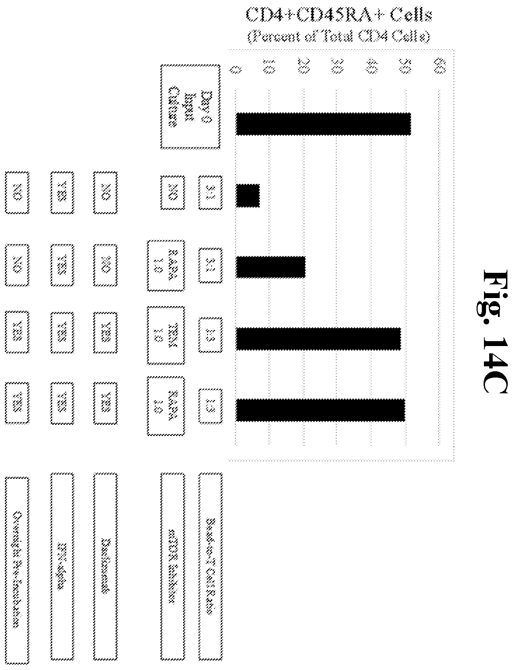

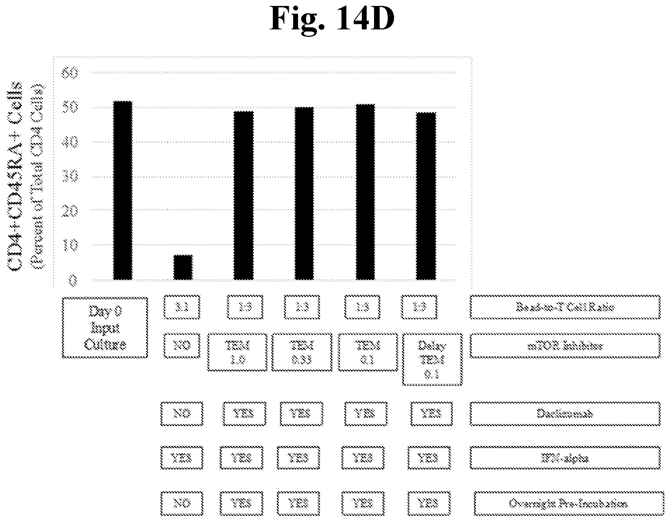

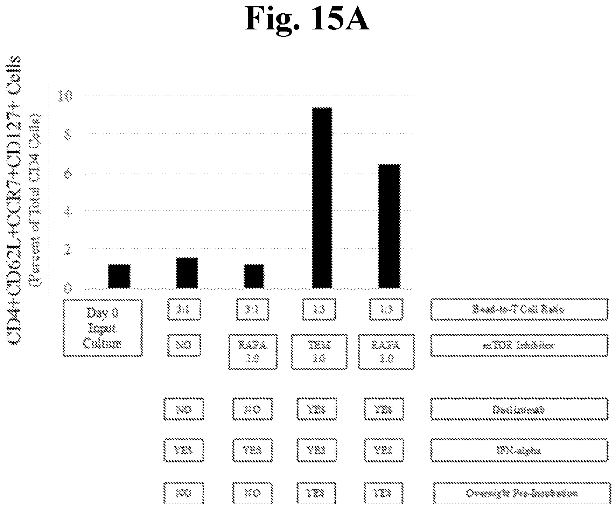

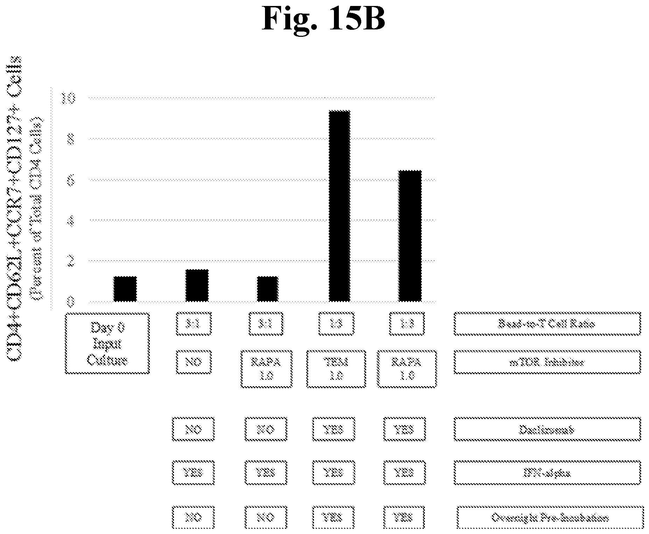

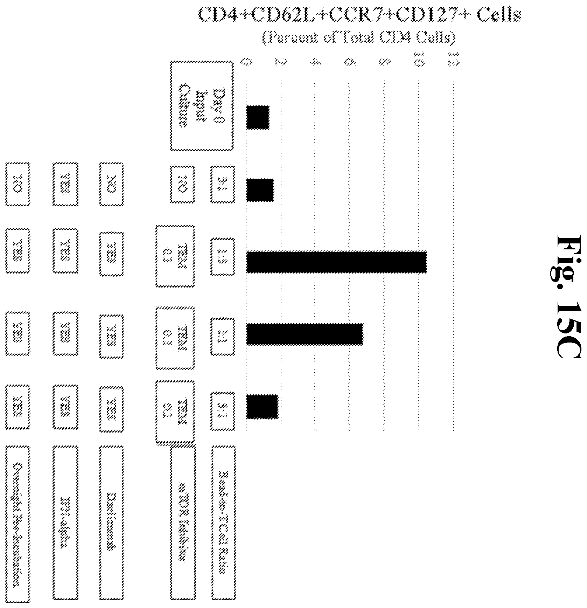

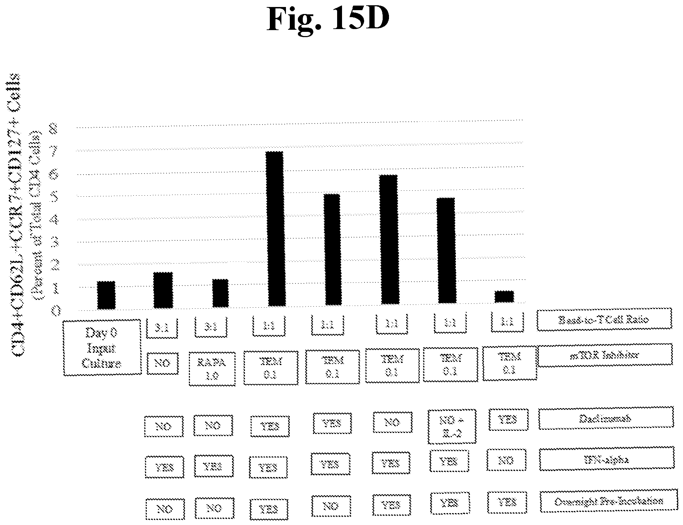

For FIGURES 14A-15D, CD4⁺ and CD8⁺ T cells were cultured, as now indicated in FIGURESS 14A-15D, using various 3/28 bead ratios, various methods of mTOR inhibition, variable addition of anti-IL-2 receptor blockade, variable addition of the type I polarizing cytokine IFN-α, and variable use of an initial overnight pre-incubation step. At day 6 of culture, T cells were harvested and evaluated for flow cytometric expression of CD45RA (FIGURES 14A-14D) or CD62L, CCR7, and CD127 (FIGURE 15A-15D) on the CD4 cell subset, with comparison of results to the expression level at day 0 of culture ("Day 0 Input Culture").

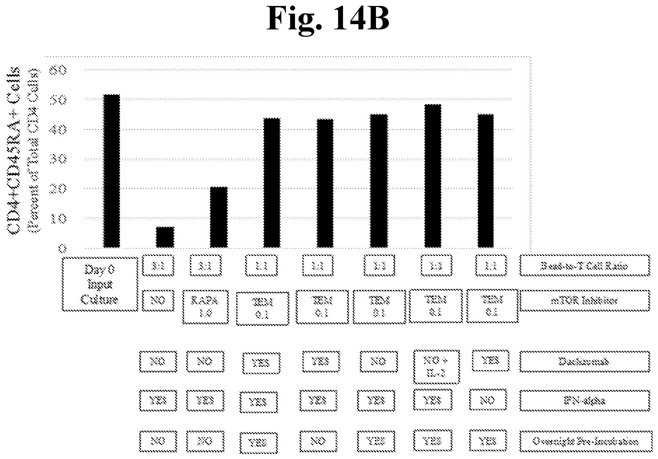

As FIGURES 14A-14D illustrate, T cell manufacturing without critical elements of the combinatorial method (use of high co-stimulation at a 3:1 bead-to-T cell ratio; no usage of daclizumab; no usage of an mTOR inhibitor) results in rapid deterioration of CD45RA expression (see column #2). In marked contrast, use of all of these components resulted in complete preservation of CD45RA expression (see column #4). Of note, T cells propagated using the prior manufacturing method that we identified did not have optimally preserved expression of CD45RA (T-Rapa cells; FIGURE 14B, column #3).

In addition, we evaluated whether the combinatorial method resulted in other markers of reduced T cell differentiation, including CD62L, CCR7, and CD127. FIGURE 15A (column #4) indicates that T cells manufactured using the pre-incubation step, low level co-stimulation, antibody blockade of the IL-2 receptor, and an mTOR inhibitor had increased co-expression of these T cell markers. Of note, T cells propagated using the prior manufacturing method that we identified did not have optimally increased expression of these three memory markers (T-Rapa cells; FIGURE 15A, column #3).

As such, the combinatorial method of Th1/Tc1 cell manufacturing is advantageous in terms of manufacturing T cells of limited differentiation status, which have been clearly and reproducibly shown to mediate increased in vivo effects.

The Combinatorial Method is Optimized by Reducing T Cell Purity at Culture Initiation. For T cell manufacturing, it is important to determine whether the culture input population must be purified to a high degree for T cell content or whether accessory cell populations such as monocytes might be tolerated. From a financial cost and labor standpoint, it is generally desirable to initiate cultures with populations that are not highly purified. However, contaminating populations of cells at culture initiation may be detrimental to T cell expansion or to generation of the desired T cell phenotype. To evaluate this parameter, we initiated cultures using the combinatorial method using input populations that were either 100%, 66%, 33%, or 10% pure for T cell content; the remaining populations of cells were primarily monocytes.

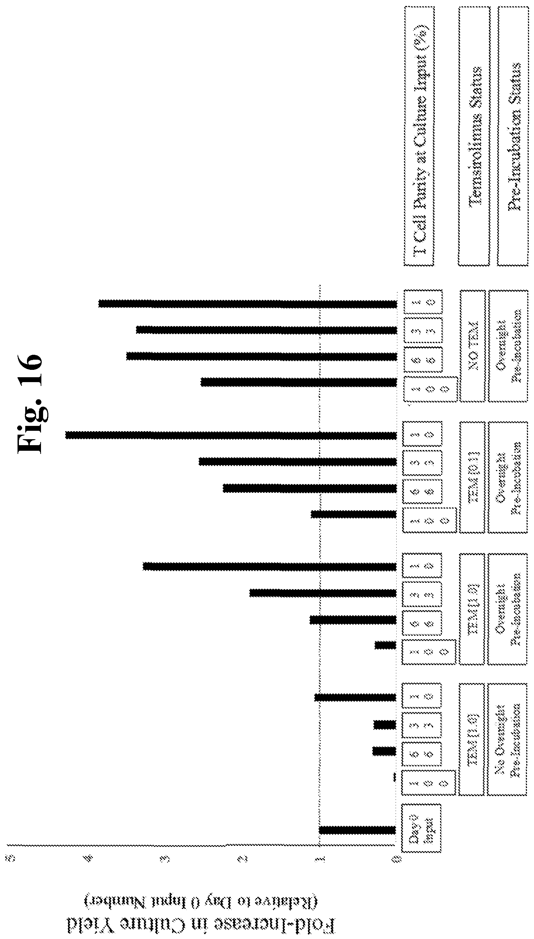

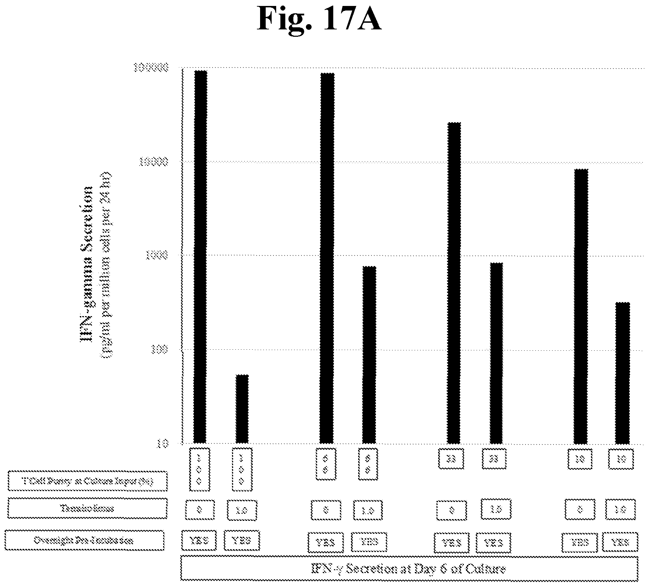

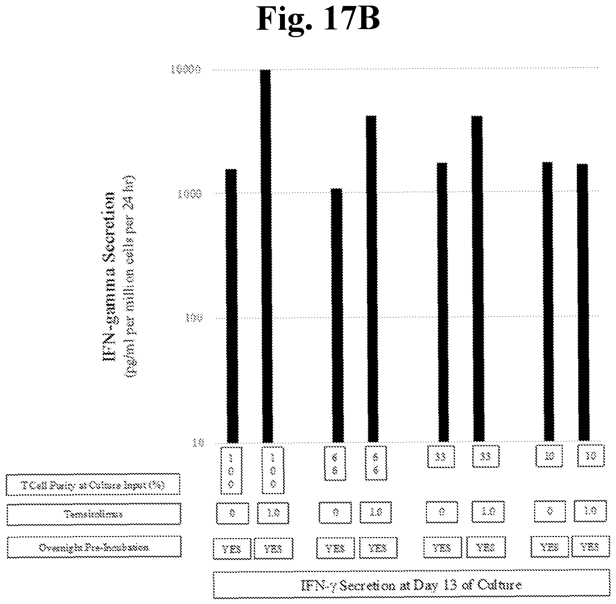

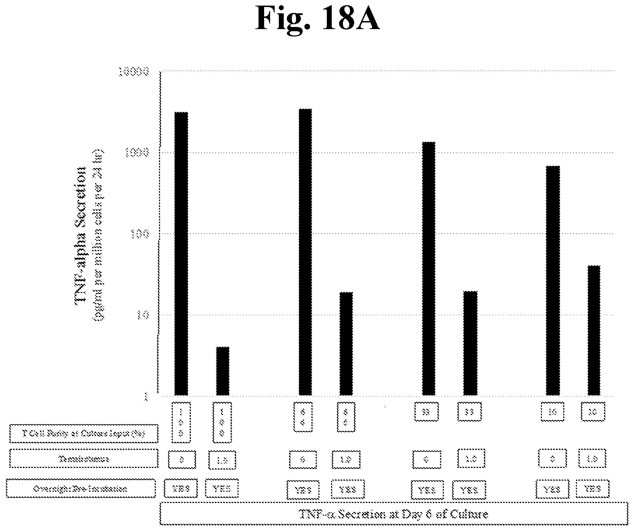

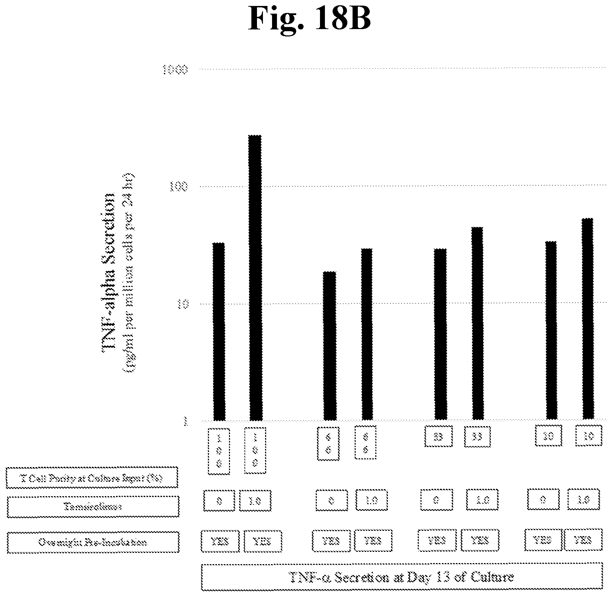

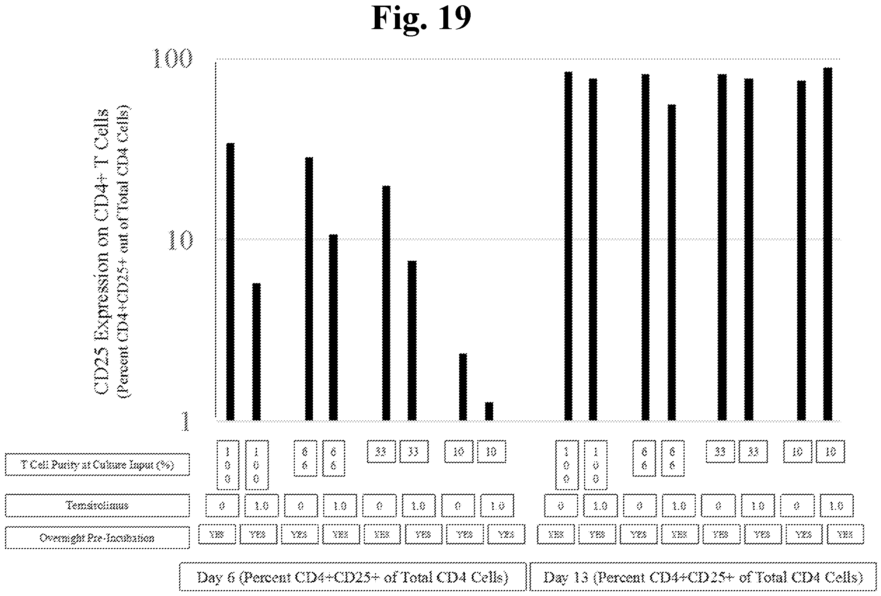

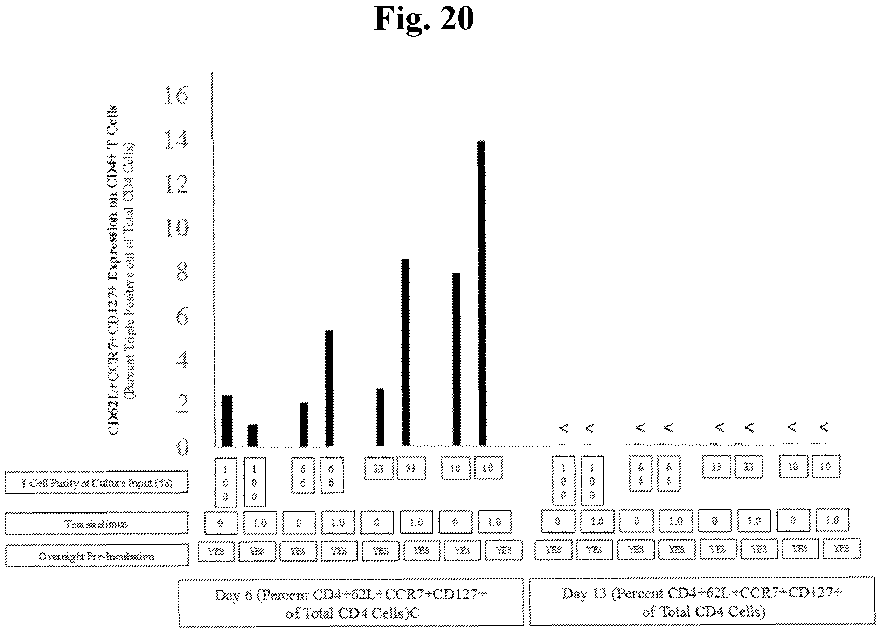

For FIGURES 16-20, prior to culture initiation, the input cell population was adjusted such that the purity of T cells was either 100%, 66%, 33%, or 10%; the remaining cell populations were comprised of the non-T cell populations contained in peripheral blood mononuclear cells (primarily monocytes). As indicated, T cells were expanded in media that variably contained or did not contain temsirolimus (concentration of either 1.0 or 0.1 µM); in addition, cultures variably included a 16-hr pre-incubation or no pre-incubation prior to anti-CD3, anti-CD28 bead co-stimulation (1:3 ratio). Each culture shown was propagated in media containing the anti-IL-2 receptor monoclonal antibody daclizumab (50 µg/ml) and IFN-α (10,000 IU/ml). At the end of the 6-day manufacturing interval, the T cells received a high-level of co-stimulation (3:1 bead-to-T cell ratio). For FIGURE 16, after high level of co-stimulation the T cells were then expanded for one week in media that did not contain inhibitors. At the end of this expansion interval, the T cells were enumerated and graphed relative to day 0 input number. For FIGS. 17-18, after high level of co-stimulation the T cells were expanded until day 13 of culture. At both day 6 and day 13, the T cells were co-stimulated and the 24-hr supernatant was evaluated for IFN-γ (FIGURES 17A-17B) or TNF-α (FIGURES 18A-18B) content (result shown in pg per ml per million cells per 24 hrs). For FIGURES 19-20, after high level of co-stimulation the T cells were expanded until day 13 of culture. At both day 6 and day 13, the T cells were evaluated by flow cytometry for CD25 expression (results shown are percent of CD4+ T cells that co-express CD25) (FIGURE 19) or for expression of CD62L, CCR7, and CD127 (FIGURE 20).

As detailed in FIGURE 16, cultures that were initiated with reduced T cell purity at culture input resulted in greater capacity for T cell expansion, with this relationship occurring in a dose-dependent manner. Of note, T cells that were propagated using the overnight pre-incubation step had a greater expansion relative to T cells that were co-stimulated at the time of culture initiation. These data provide further support to the combinatorial method and indicate that cell populations at culture initiation that may otherwise be considered contaminants appear to actually facilitate T cell expansion potential. As such, optimized use of the combinatorial method should also include the use of T cells that are not highly enriched at culture initiation; for quality assurance, it may be important to control the level of purity, by way of non-limiting example, initiating each culture with an inoculum that is either 66% or 33% pure for T cell content. As detailed in FIGURES 17A-17B and 18A-18B, T cells manufactured using the combinatorial method and input T cells that were not highly purified resulted in the desired cytokine secretion pattern, namely: (FIGURES 17A-17B) reduced IFN-γ secretion at the end of manufacturing (day 6) and increased IFN-γ secretion after one week expansion in media not containing inhibitors (day 13); and (FIGURES 18A-18B) reduced TNF-α secretion at the end of manufacturing (day 6) and increased TNF-α secretion after one week expansion in media not containing inhibitors (day 13).

In addition, we evaluated cell surface marker expression in T cells manufactured with the combinatorial method using input populations of reduced T cell purity. As shown in FIGURE 19, such T cells had reduced CD25 expression at the end of manufacturing (day 6), consistent with a quiescent phenotype; after one week of culture without inhibitors, the T cells greatly upregulated CD25. As shown in FIGURE 20, such T cells also had increased co-expression of memory markers CD62L, CCR7, and CD127; these markers were then reduced after one week T cell expansion.

In sum, these data indicate that manufacture of Th1/Tc1 cells using the combinatorial method is possible using input T cells having substantial contamination with non-T cell populations. Indeed, purposeful inclusion of such non-T cell populations can be utilized to improve T cell yield and improve the resultant T cell memory profile.

*Manufacturing from cryopreserved cell substrates.* In the case of previously collected PBSC products, such cryopreserved cells will be stored in the vapor phase of liquid nitrogen until thawing of cells and manufacture of Rapa-T cells. In the case of freshly isolated cells by apheresis or in the future by simple blood collection, the cells will undergo immediate processing, and then may either be placed directly into culture or may be cryopreserved by controlled rate freezing technology and stored in the vapor phase of liquid nitrogen for subsequent use later.

T cell culture from cryopreserved cell substrates for from freshly isolated cell populations requires some type of T cell enrichment, for example, by use of monoclonal antibody and column technology (positive or negative selection). Enrichment of the raw cellular material used in Rapa-T manufacturing does not require such antibody-based methodologies because T cells are efficiently enriched during the culture interval; as such, our method is consistent with recommendations for effective cell therapy on a global level. The initial processing steps for manufacture of Rapa-T cells focuses on removal of di-methyl sulfoxide (DMSO) used in the cryopreservation steps (when applicable), lysis of red blood cells (RBCs), and centrifugal removal of contaminating granulocytes and to some extent, monocytes. These steps are performed in a relatively automated method using primarily closed-system technology; this procedure is advantageous as it reduces human error, provides detailed manufacturing data for batch records, improves consistency across manufacturing runs, and reduces the risk for infectious agent contamination of the final product. Processing for the Rapa-T products incorporates the following steps: (1) thaw of cryopreserved products (when applicable) using solid-state, non-water based methods to reduce infectious agent contamination; (2) automated washing of the cellular product using the LOVO permeable membrane device; (3) integration of lysis of RBCs using ammonium-chloride-potassium (ACK) buffer during the LOVO washing steps; (4) volume reduction of cellular content using the LOVO method with subsequent plating of cells into the closed-system, counter-flow, centrifugal elutriation (CCE) device (Elutra; Terumo); and (5) pre-programmed operation of the Elutra device for efficient removal of granulocytes and monocytes by CCE.

After this lymphocyte enrichment and media purification, the cells are plated into specialized chambers that possess an enriched capacity for oxygen exchange (G-Rex vessels; Wilson-Wolf). In addition to having enhanced gas permeability characteristics, the G-Rex vessels are closed system units and have the additional advantage of automated, closed system media volume reduction (GatheRex Liquid Handling Pump). The lymphocyte-enriched cells are maintained in the G-Rex vessels for 6-days.

Several specific culture conditions can be utilized to promote the manufacture of a mixture of CD4+ and CD8+ T cells in the G-Rex vessels with functional attributes of a manufactured T cell. These specific conditions include: (1) use of enriched media (including but not limited to X-Vivo 20; Lonza) that is further supplemented with 5% human serum; (2) incorporation of a 16-hour rest interval of cell plating into the G-Rex prior to co-stimulation (cells are plated at a relatively high density of 1.5 x 106 cells per ml); (3) during this initial rest interval, cells are optimally rested by addition of the monoclonal antibody basiliximab (which blocks the IL-2 receptor and thereby prevents autonomous T cell activation by endogenously produced IL-2) and temsirolimus (which is a pharmacologic inhibitor of mTORC1); (4) after this 16-hour rest interval, cells are either not co-stimulated or are co-stimulated with anti-CD3/anti-CD28 coated magnetic beads (3/28 beads) under sub-optimal conditions, as defined by a bead-toT cell ratio of 1:3 (typically, most T cell expansion conditions utilize a 9-fold higher level of co-stimulation, a 3:1 bead-to-T cell ratio); (5) importantly, it is essential that the T cells are not washed after the initial rest interval; (6) after the rest interval, in addition to addition of 3/28 beads, it is essential to add the polarizing cytokine IFN-α at a high dose (10,000 IU/ml) to promote differentiation to CD4+ Th1 and CD8+ Tc1 phenotypes; (7) importantly, it is critical to avoid the addition of IL-2, which is a common additive to T cell cultures; and (8) after addition of the beads and IFN-α, it is important to leave the cells undisturbed until harvesting at day 6 of culture (no cell washing, no further culture additives).

*Cryopreservation of Manufactured T Cells.* 1) After the 6-day cell culture in the G-Rex vessels, the volume of the culture can be reduced in a closed system manner by the GatheRex instrument. Subsequently, the cells can be harvested, 3/28 beads can be removed by hand-held magnet, and cells can be placed into the LOVO device for serial washing of the cells to remove > 99% of culture additives (Temsirolimus, Basiliximab, IFN-α).

Washed cells can be reconstituted into cryopreservation media, which contains 5% DMSO and 5% pentastarch. The cryopreservation is performed in multiple single use aliquots in 50 ml freezer bags. Rapa-T cells are cryopreserved by GMP-compliant controlled rate freezing methodology and shipped in the vapor phase of liquid nitrogen by certified cryo-shipper after the Rapa-T cells have passed the specified release criteria testing.

Release criteria testing of the Rapa-T cells includes standard tests such as content of CD3+, CD4+ and CD8+ T cell purity (final product can be > 70% CD3+ T cell content by flow cytometry; CD4+ and CD8+ subsets can each be present at the 5% level). Cells can be > 70% viable, as determined by flow cytometry annexin and 7-AAD assays. Furthermore, cells should be free of bacterial and fungal contamination upon a minimum of a 3-day culture interval (ideally, a 14-day culture interval); furthermore, the cell product should be below detection limit for bacterial LPS endotoxin.

In addition to these standard tests, specialized functional tests will constitute release criteria for the Rapa-T cell products. Prior to release of product and cell therapy, a Rapa-T cell can possess the following attributes relative to culture input T cells: (1) an enhanced T central memory phenotype, as defined by increased flow cytometric co-expression of CD62 ligand and CCR7; (2) low level expression of checkpoint inhibitory molecules, such as programmed death-1 (PD1); (3) a resting state, as defined by reduced levels of Th1/Tc1-type cytokine secretion upon maximal co-stimulation; (4) an autophagy signature, as evidenced by reduced mitochondrial mass by flow cytometry MitoTracker assay; (5) a resistant phenotype, as evidenced by at least 50% inhibition of mTORC1 and mTORC2 downstream targets; and (6) a multi-faceted differential gene expression profile of n=80 key transcription factor and differentiation molecules.

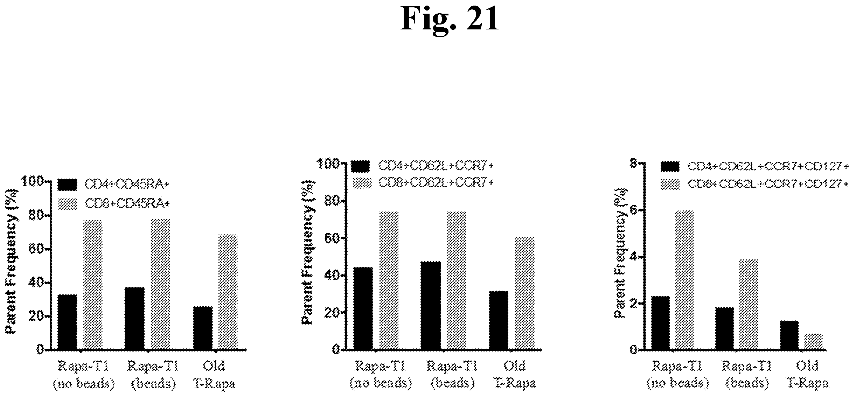

FIGURE 21 illustrates that the new Rapa-T method generates T cells with increased expression of the naive or T central-memory markers relative to T-Rapa cells, independent of whether the new Rapa-T method uses no bead co-stimulation or a low level of bead co-stimulation (1:3 ratio of beads-to-T cells). In FIGURE 21, Rapa-T1 cells were generated by culture in IFN-α, temsirolimus, and basiliximab, as previously described, either without bead co-stimulation (first two columns in each panel) or with 1:3 bead-to-T cell co-stimulation (third and fourth column in each panel); the results were compared to culture using the previous T-Rapa method (use of rapamycin and 3:1 bead-to-T cells; fifth and sixth column in each panel). Flow cytometry was performed at the end of culture, with results detailed for both the CD4⁺ T cell subset (black columns) and the CD8⁺ T cell subset (gray columns). Results shown are for the naive T cell subset, as defined by CD45RA+ expression (left panel); for the T central memory subset, as defined by co-expression of CD62L and CCR7); and for the more primitive T cell subset that co-expresses CD62L, CCR7, and CD127.

As detailed in FIGURE 21, the CD4⁺ and CD8⁺ T cells manufactured according to the methods described in this disclosure have increased levels of expression of naive and T central memory markers by flow cytometry relative to T-Rapa cells, including in conditions where the methods involve no bead co-stimulation or co-stimulation at a reduced level compared to the T-Rapa method.

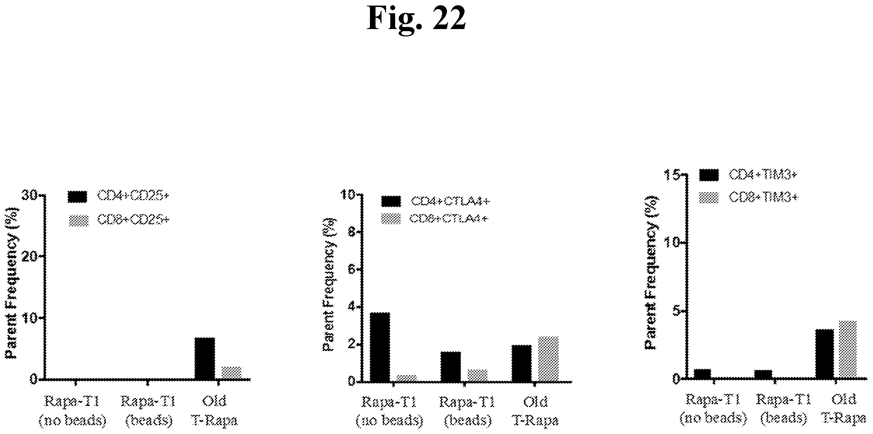

FIGURE 22 illustrates that the new Rapa-T method generates T cells with reduced expression of CD25, CTLA4, and TIM3 relative to T-Rapa cells, independent of whether the new Rapa-T method uses no bead co-stimulation or a low level of bead co-stimulation (1:3 ratio of beads-to-T cells). In FIGURE 22, Rapa-T1 cells were generated by culture in IFN-α, temsirolimus, and basiliximab, as previously described, either without bead co-stimulation (first two columns in each panel) or with 1:3 bead-to-T cell co-stimulation (third and fourth column in each panel); the results were compared to culture using the previous T-Rapa method (use of rapamycin and 3:1 bead-to-T cells; fifth and sixth column in each panel). Flow cytometry was performed at the end of culture, with results detailed for both the CD4⁺ T cell subset (black columns) and the CD8⁺ T cell subset (gray columns). Results shown are for expression of the IL-2 receptor CD25 (left panel); for the immune suppressive and T_{REG}-associated molecule CTLA4; and for the immune checkpoint molecule TIM3.

As detailed in FIGURE 22, the CD4⁺ and CD8⁺ T cells manufactured according to the methods described in this disclosure have reduced levels of expression of the IL-2 receptor CD25², which is associated with T cell activation and T_{REG} cell function, and reduced levels of the immune suppressive molecule CTLA4³ and the immune checkpoint inhibitory molecule TIM3⁴ by flow cytometry relative to T-Rapa cells, including in conditions where the methods involve no bead co-stimulation or co-stimulation at a reduced level compared to the T-Rapa method.

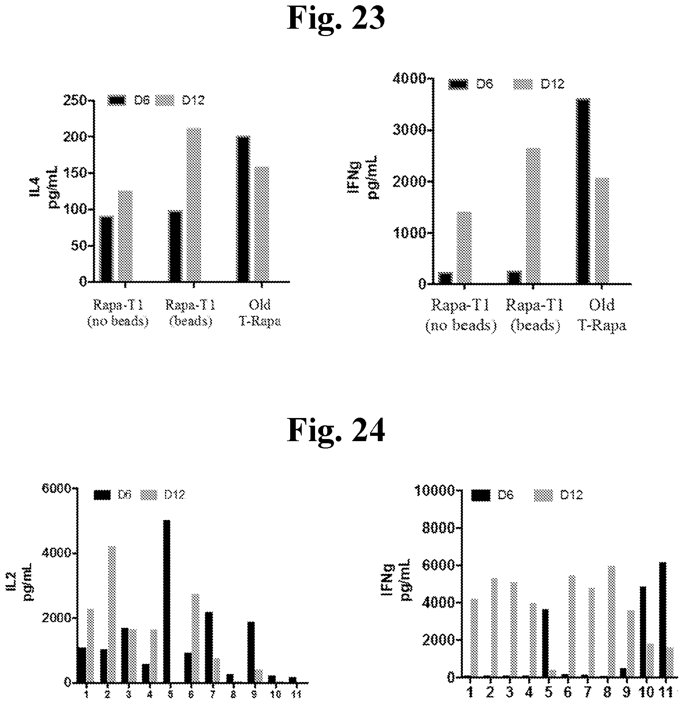

FIGURE 23 illustrates that the new Rapa-T method generates T cells with a similar pattern of Th2 vs. Th1 polarization but increased quiescence relative to T-Rapa cells, independent of whether the new Rapa-T method uses no bead co-stimulation or a low level of bead co-stimulation (1:3 ratio of beads-to-T cells). In FIGURE 23, Rapa-T1 cells were generated by culture in IFN-α, temsirolimus, and basiliximab, as previously described, either without bead co-stimulation (first two columns in each panel) or with 1:3 bead-to-T cell co-stimulation (third and fourth column in each panel); the results were compared to culture using the previous T-Rapa method (use of rapamycin and 3:1 bead-to-T cells; fifth and sixth column in each panel). Cytokine secretion analysis (IL-4 and IFN-g measurement) was performed at the end of culture, with results detailed at the end of T cell manufacturing (day 6) and after an additional 6 days in culture without inhibitors (day 12).

As detailed in FIGURE 23, the CD4⁺ and CD8⁺ T cells manufactured according to the methods described in this disclosure have a comparable degree of Th2 vs. Th1 cytokine polarization relative to T-Rapa cells, including in conditions where the methods involve no bead co-stimulation or co-stimulation at a reduced level, compared to the T-Rapa method. Specifically, there is a low level of secretion of the Th2-type cytokine IL-4 (with values at the end of manufacturing [day 6] and after an additional period of culture without inhibitors [day 12] in the range of 100 to 200 pg/ml at both day 6 and day 12) and a high level of secretion of the Th1-type cytokine IFN-γ at day 12 of culture (values between 1000 and 3000 pg/ml). The IFN-γ secretion in the new Rapa-T conditions at the end of manufacturing (day 6) was greatly reduced relative to the old T-Rapa condition, thereby indicating the favorable characteristic of T cell quiescence in the new Rapa-T manufacturing method, independent of whether the method utilized no bead co-stimulation or a low-level of bead co-stimulation (1:3 ratio of beads-to-T cells).

As detailed in FIGURE 24, the CD4⁺ and CD8⁺ T cells manufactured according to the methods described in this disclosure have a pattern of Th1 cytokine polarization relative to T-Rapa cells, including in conditions where the methods involve no bead co-stimulation or co-stimulation at a reduced level, compared to the T-Rapa method.

FIGURE 24 illustrates that the new Rapa-T method generates T cells with a favorable pattern of Th1 polarization relative to T-Rapa cells, independent of whether the new Rapa-T method uses no bead co-stimulation or a low level of bead co-stimulation (1:3 ratio of beads-to-T cells). In FIGURE 24, Rapa-T1 cells were generated by culture in IFN-α, temsirolimus, and basiliximab, as previously described, either without bead co-stimulation or with 1:3 bead-to-T cell co-stimulation; various control cultures were also evaluated, including the previous T-Rapa method (use of rapamycin and 3:1 bead-to-T cells). All cultures were at 9 x 10⁶ cells/ml concentration, did not have bead co-stimulation, did not have IL-2 addition, had a delay in addition of IFN-α, included temsirolimus at a concentration of 1 µM and basiliximab at a concentration of 10 µM, and used X-Vivo 20 media supplemented with 5% AB serum, unless otherwise specified. Specific culture conditions as per the legend in the figure are as follows: condition 1, Rapa-T method, as above; condition 2, Rapa-T method without serum; condition 3, Rapa-T method without basiliximab; condition 4, Rapa-T method without basiliximab and with reduced temsirolimus (0.1 µM); condition 5, Rapa-T method without temsirolimus or basiliximab; condition 6, Rapa-T method using input T cells contaminated with a high frequency of monocytes (79% of input cells were monocytes, increased from all other cultures, which had ~ 10% monocyte contamination); condition 7, Rapa-T method using no monocyte contamination (< 1%); condition 8, Rapa-T method using simultaneous addition of IFN-α (no overnight delay); condition 9, Rapa-T method with 1:3 bead co-stimulation; condition 10, control T cell condition (no inhibitors; 3:1 beads); and condition 11, old T-Rapa condition, rapamycin (1 µM), IL-2 addition at 20 IU/ml, 3:1 beads, no overnight pre-incubation. Cytokine secretion analysis (IL-2 and IFN-γ measurement) was performed at the end of culture, with results detailed at the end of T cell manufacturing (day 6) and after an additional 6 days in culture without inhibitors (day 12).

As detailed in FIGURE 24, the CD4⁺ and CD8⁺ T cells manufactured according to the methods described in this disclosure have a pattern of Th1 cytokine polarization relative to T-Rapa cells, including in conditions where the methods involve no bead co-stimulation or co-stimulation at a reduced level, compared to the T-Rapa method.

As depicted in FIGURE 24, Condition #1, the Rapa-T condition manufactured without beads, had a favorably high level of IL-2 secretion capacity, particularly at day 12 after T cell expansion in the absence of inhibitors. A similar pattern was observed in condition #2, which was performed in media that was not supplemented with serum, thereby indicating the ability to manufacture the Rapa-T cells with or without serum supplementation. The increased IL-2 secretion capacity, particularly in comparison to culture #11 (the old T-Rapa condition), indicates that the new Rapa-T manufacturing method generates T cells of a reduced differentiation precursor profile that can function in vivo in a helper-independent manner. Condition #1 was also advantageous in this regard relative to culture #9, which is the new Rapa-T condition manufactured with a 1:3 bead-to-T cell ratio of co-stimulation.

As depicted in FIGURE 24, Condition #1, the Rapa-T condition manufactured without beads, was also preferable in terms of IFN-γ secretion at day 6 end of manufacturing, as the levels were near the lower limit of detection, thereby indicating that the Rapa-T cell product was in a state of quiescence. By comparison, condition #5, which did not contain inhibitors, had nearly 4000 pg/ml secretion of IFN-γ at day 6; similarly, the old T-Rapa condition had a high level of IFN-γ secretion at day 6, approximately 6000 pg/ml. Of note, the new Rapa-T condition that utilized a 1:3 bead-to-T cell co-stimulation was less quiescent than condition #1 without beads, as there was approximately 200 pg/ml of IFN-γ secretion at the end of manufacturing, day 6. Finally, the new Rapa-T manufacturing method (either condition #1 without beads or condition #9 with beads) had a favorable increase in IFN-γ secretion capacity at day 12 of culture after a 6-day culture interval in the absence of inhibitors.

In summation, the new Rapa-T method can result in the manufacture of T cells with the following phenotypic characteristics: (a) relative to input normal T cells, a reduction in expression of regulatory T cells markers such as the transcription factor, FOXP3; and an increase in the Th1-type transcription factor, TBET; (b) relative to the old T-Rapa method, an increased state of quiescence, as indicated in part by reduced expression of the IL-2 receptor CD25 and by reduced secretion of the inflammatory cytokines IFN-γ and TNF-α at the end of manufacturing; (c) relative to the old T-Rapa method, a reduction in the expression of p-STAT5 and reduced levels of the mTOR pathway molecules p-RAPTOR, p-4EBP1 and p70S6K; (d) relative to input normal T cells, an increase in markers of autophagy, including but not limited to changes in expression of the molecule p62; (e) relative to input normal T cells, an increase in flow cytometry markers of naive or T central memory populations, including CD45RA, co-expression of CD62L/CCR7, concomitant expression of CD62L/CCR7/CD127; (f) relative to the old T-Rapa method, reduced expression of co-inhibitory molecules including but not limited to CTLA4; (g) relative to the old T-Rapa method, reduced expression of checkpoint inhibitory receptor expression, including but not limited to TIM3; and (h) relative to input normal T cells, an altered RNA expression pattern, including but not limited to an increase in markers of de-differentiation (including but not limited to Nanog, KLF4, and KLF10) and a reduction in markers of differentiation (including but not limited to perforin, granzyme B, IFN-γ).

The majority of the phenotype characterization of the T cell product manufactured according to the Rapa-T method detailed in this disclosure can be ascertained at the end of culture. However, it is important to note that the T cell product can be cryopreserved, and as such, phenotypic characterization of T cells in the post-thaw state reflect the actual product to be adoptively transferred to the subject. The Rapa-T cells in the post-thaw state can be characterized by the following: (a) maintenance of a state of quiescence, as indicated by low level expression of the IL-2 receptor CD25 that is comparable between day 6 end of manufacturing sample and the post-thaw sample; (b) relative to the day 6 end of manufacturing sample, the post-thaw sample will continue to have low level secretion of the inflammatory cytokines IFN-γ and TNF-α (not increased relative to the sample collected at the end of manufacturing); (c) relative to input normal T cells, the post-thaw sample will maintain an increase in flow cytometry markers of naive or T central memory populations, including CD45RA, co-expression of CD62L/CCR7, concomitant expression of CD62L/CCR7/CD127; (f) the post-thaw sample will continue to have reduced expression of co-inhibitory molecules including but not limited to CTLA4 (no increase in the post-thaw sample relative to the sample collected at the end of manufacturing); (g) the post-thaw sample will continue to have reduced expression of checkpoint inhibitory receptor expression, including but not limited to TIM3 (no increase in the post-thaw sample relative to the sample collected at the end of manufacturing); and (h) relative to input normal T cells, an altered RNA expression pattern, including but not limited to an increase in markers of de-differentiation (including but not limited to Nanog, KLF4, and KLF10) and a reduction in markers of differentiation (including but not limited to perforin, granzyme B, IFN-γ).

*Manufacturing from cryopreserved cell substrates.* In the case of previously collected PBSC products, such cryopreserved cells will be stored in the vapor phase of liquid nitrogen until thawing of cells and manufacture of Rapa-T cells. In the case of freshly isolated cells by apheresis or in the future by simple blood collection, the cells will undergo immediate processing, and then may either be placed directly into culture or may be cryopreserved by controlled rate freezing technology and stored in the vapor phase of liquid nitrogen for subsequent use later.

T cell culture from cryopreserved cell substrates for from freshly isolated cell populations requires some type of T cell enrichment, for example, by use of monoclonal antibody and column technology (positive or negative selection). Enrichment of the raw cellular material used in Rapa-T manufacturing does not require such antibody-based methodologies because T cells are efficiently enriched during the culture interval; as such, our method is consistent with recommendations for effective cell therapy on a global level. The initial processing steps for manufacture of Rapa-T cells focuses on removal of di-methyl sulfoxide (DMSO) used in the cryopreservation steps (when applicable), lysis of red blood cells (RBCs), and centrifugal removal of contaminating granulocytes and to some extent, monocytes. These steps are performed in a relatively automated method using primarily closed-system technology; this procedure is advantageous as it reduces human error, provides detailed manufacturing data for batch records, improves consistency across manufacturing runs, and reduces the risk for infectious agent contamination of the final product. Processing for the Rapa-T products can include the following steps: (1) thaw of cryopreserved products (when applicable) using solid-state, non-water based methods to reduce infectious agent contamination; (2) automated washing of the cellular product using the LOVO permeable membrane device; (3) integration of lysis of RBCs using ammonium-chloride-potassium (ACK) buffer during the LOVO washing steps; (4) volume reduction of cellular content using the LOVO method with subsequent plating of cells into the closed-system, counter-flow, centrifugal elutriation (CCE) device (Elutra; Terumo); and (5) pre-programmed operation of the Elutra device for efficient removal of granulocytes and monocytes by CCE.

After this lymphocyte enrichment and media purification, the cells can be plated into specialized chambers that possess an enriched capacity for oxygen exchange (G-Rex vessels; Wilson-Wolf). In addition to having enhanced gas permeability characteristics, the G-Rex vessels are closed system units and have the additional advantage of automated, closed system media volume reduction (GatheRex Liquid Handling Pump). The lymphocyte-enriched cells can be maintained in the G-Rex vessels for 6-days.

Several specific culture conditions can be utilized to promote the manufacture of a mixture of CD4⁺ and CD8⁺ T cells in the G-Rex vessels with functional attributes of a manufactured T cell. These specific conditions include: (1) use of enriched media (including but not limited to X-Vivo 20; Lonza) that is further supplemented with 5% human serum, or in some embodiments, no addition of serum; (2) incorporation of a 16-hour rest interval of cell plating into the G-Rex prior to co-stimulation (cells are plated at a relatively high density of 1.5 x 10⁶ T cells per ml); (3) during this initial rest interval, cells are optimally rested by addition of the monoclonal antibody basiliximab (which blocks the IL-2 receptor and thereby prevents autonomous T cell activation by endogenously produced IL-2) and temsirolimus (which is a pharmacologic inhibitor of mTORC1); (4) after this 16-hour rest interval, cells are co-stimulated with anti-CD3/anti-CD28 coated magnetic beads (3/28 beads) under sub-optimal conditions, as defined by a bead-to-T cell ratio of 1:3 (typically, most T cell expansion conditions utilize a 9-fold higher level of co-stimulation, a 3:1 bead-to-T cell ratio; in some cases, it is beneficial to avoid adding any co-stimulation reagent); (5) importantly, it is essential that the T cells are not washed after the initial rest interval; (6) after the rest interval, in addition to addition of 3/28 beads, it is essential to add the polarizing cytokine IFN-α at a high dose (10,000 IU/ml) to promote differentiation to CD4⁺ Th1 and CD8⁺ Tc1 phenotypes; (7) importantly, it is critical to avoid the addition of IL-2, which is a common additive to T cell cultures; and (8) after addition of the beads and IFN-α, it is important to leave the cells undisturbed until harvesting at day 6 of culture (no cell washing, no further culture additives).

*Cryopreservation of Manufactured T Cells.* 1) After the 6-day cell culture in the G-Rex vessels, the volume of the culture can be reduced in a closed system manner by the GatheRex instrument. Subsequently, the cells can be harvested, 3/28 beads can be removed by hand-held magnet, and cells can be placed into the LOVO device for serial washing of the cells to remove > 99% of culture additives (Temsirolimus, Basiliximab, IFN-α).

Washed cells can be reconstituted into cryopreservation media, which contains 5% DMSO and 5% pentastarch. The cryopreservation is performed in multiple single use aliquots in 50 ml freezer bags. Rapa-T cells are cryopreserved by GMP-compliant controlled rate freezing methodology and shipped in the vapor phase of liquid nitrogen by certified cryo-shipper after the Rapa-T cells have passed the specified release criteria testing.

Release criteria testing of the Rapa-T cells includes standard tests such as content of CD3⁺, CD4⁺ and CD8⁺ T cell purity (final product can be > 70% CD3⁺ T cell content by flow cytometry; CD4⁺ and CD8⁺ subsets can each be present at the 5% level). Cells can be > 70% viable, as determined by flow cytometry annexin and 7-AAD assays. Furthermore, cells should be free of bacterial and fungal contamination upon a minimum of a 7-day culture interval (ideally, a 14-day culture interval); furthermore, the cell product should be below detection limit for bacterial LPS endotoxin and mycoplasma.

In addition to these standard tests, specialized functional tests can constitute release criteria for the Rapa-T cell products. Prior to release of product and cell therapy, a Rapa-T cell can possess the following attributes relative to culture input T cells: (1) an enhanced T central memory phenotype, as defined by increased flow cytometric co-expression of CD62 ligand and CCR7; (2) low level expression of checkpoint inhibitory molecules, such as programmed death-1 (PD1); (3) a resting state, as defined by reduced levels of Th1/Tc1-type cytokine secretion upon maximal co-stimulation; (4) an autophagy signature, as evidenced by reduced mitochondrial mass by flow cytometry MitoTracker assay; (5) a resistant phenotype, as evidenced by at least 50% inhibition of mTORC1 and mTORC2 downstream targets; and (6) a multi-faceted differential gene expression profile of n=80 key transcription factor and differentiation molecules.

### Example 2

Steady-state apheresis was performed to obtain patient samples containing PBMCs. Lymphocytes in the samples were enriched by using an automated Ficoll procedure on a Sepax^{®} instrument by GE by >95% elimination of the undesirable contaminating neutrophil population. The lymphocyte-enriched cell populations were then plated in G-REX culture vessels at the initial cell densities in the culture media and serum supplementation conditions indicated in Table 1 below and incubated in culture for 6 days. Condition 1 represents the pre-culture control (enriched lymphocytes after Sepax^{®} automated Ficoll). Cultures were also initiated with variable inhibitor conditions by addition of temsirolimus, sirolimus and/or the anti-IL2 receptor monoclonal antibody basiliximab at the doses indicated in Table 1. Basiliximab was added at 10 µg/mL for Conditions 2-5 and 20 µg/mL for Conditions 6-8. Some culture conditions received anti-CD3, anti-CD28 co-stimulation using Dynal^{®} 3/28 beads at bead:T cell ratios of 0.88:1 (Conditions 2-3 and 5-6) or 3:1 (Conditions 8-9). Cytokines were added at the indicated times, which consisted of either IFN-α alone (10,000 IU/mL) or IFN-α (10,000 IU/mL) in combination with IL-2 (20 IU/mL). Cytokine addition was either at culture initiation (Conditions 8-9) or at one day after culture initiation (Conditions 2-7). Cultures 3-4 and 6-7 received additional media two days after culture initiation to dilute them to the final cell densities indicated.

**Table 1: Culture Conditions**

| **Condition #** | **Media** | **Serum (AB)** | **Initial Cell** | **TEM (µM)** | **Anti-IL2R** | **Co-stim.** | **Cytokine Addition** | **Final Cell** |
|---|---|---|---|---|---|---|---|---|
| | | | **Density (M/mL)** | | | | | **Density (M/mL)** |
| 1 | N/A | N/A | N/A | N/A | N/A | N/A | N/A | N/A |
| 2 | TM | No | 4.5 | 3 | Yes | Yes | IFN-α | 4.5 |
| 3 | TM | No | 15 | 3 | Yes | Yes | IFN-α | 4.5 |
| 4 | TM | No | 15 | 3 | Yes | No | IFN-α | 4.5 |
| 5 | TM | No | 9 | 4.5 | Yes | Yes | IFN-α | 9 |
| 6 | TM | No | 30 | 4.5 | Yes | Yes | IFN-α | 9 |
| 7 | TM | No | 30 | 4.5 | Yes | No | IFN-α | 9 |
| 8 | XV | 5% AB | 1.5 | None | No | Yes | IL-2 + IFN-α | 1.5 |
| 9 | XV | 5% AB | 1.5 | RAPA | No | Yes | IL-2 + IFN-α | 1.5 |

| | | | | | | | | |
|---|---|---|---|---|---|---|---|---|
| M/mL = millions of cells per milliliter; TEM = temsirolimus; RAPA = sirolimus oral solution at 1 µM; TM = TexMACS^{®} (Miltenyi^{®}); XV = X-Vivo 20^{®} (Lonza^{®}). TexMACS is a proprietary media formulation. | | | | | | | | |

Condition 9 represents the T-Rapa product. It was found that Condition 7 provided the optimal RAPA-T condition of those tested in Table 1. This condition had several key attributes: (1) serum-free media; (2) very high initial cell density (30 M/mL); (3) very high temsirolimus concentration (4.5 µM); (4) presence of anti-IL2 receptor monoclonal antibody; (5) absence of co-stimulation; (6) cytokine support with only IFN-α (no IL-2) added one day after culture initiation; and (7) cell dilution on day 2 of culture.

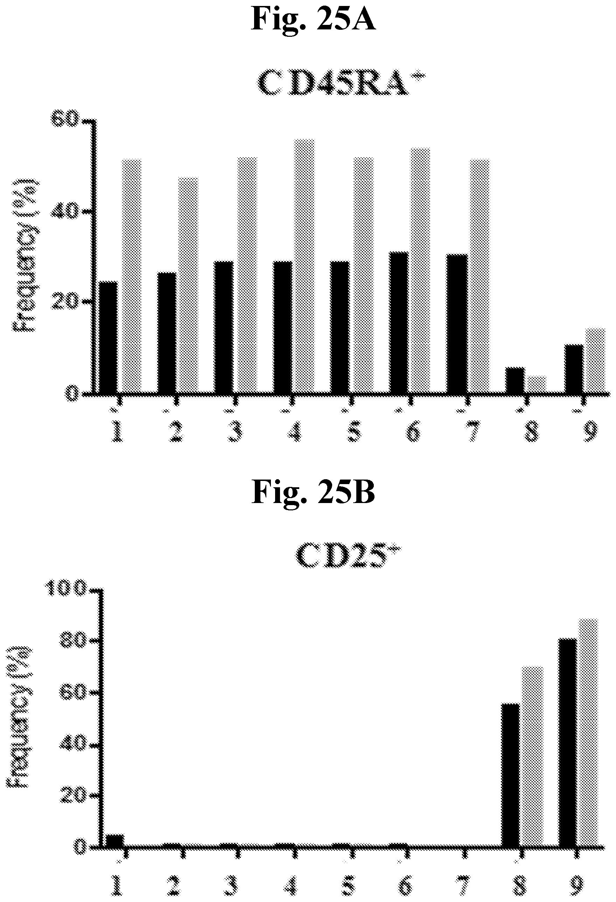

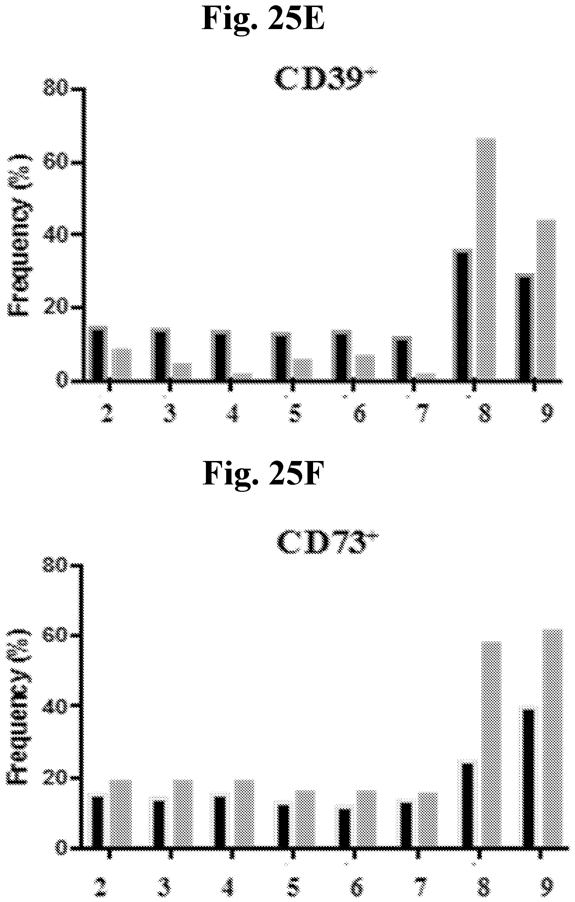

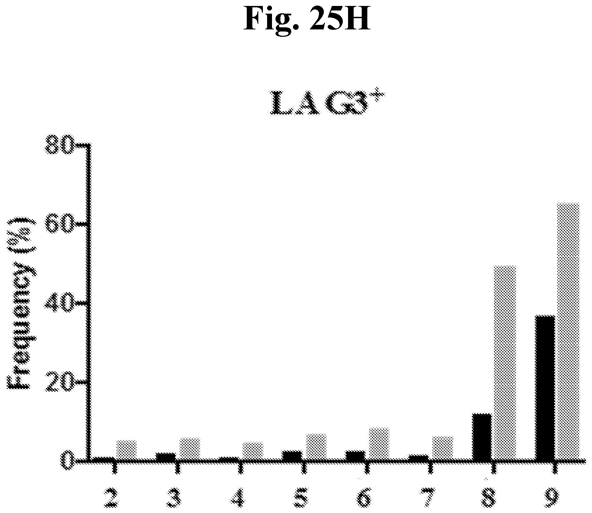

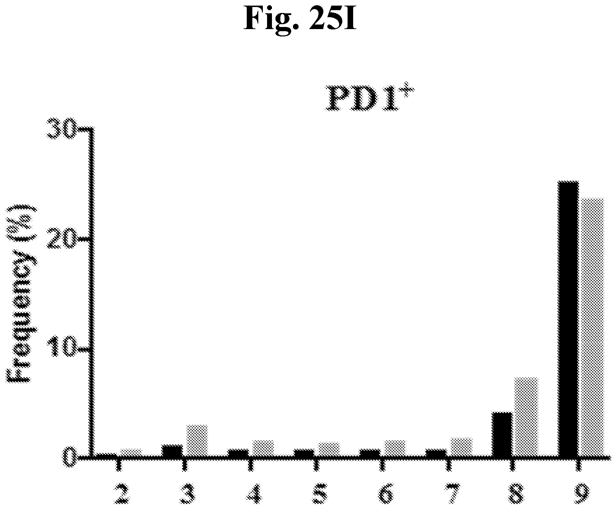

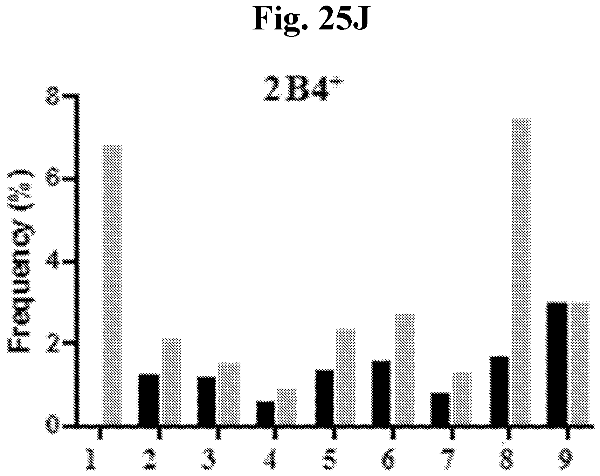

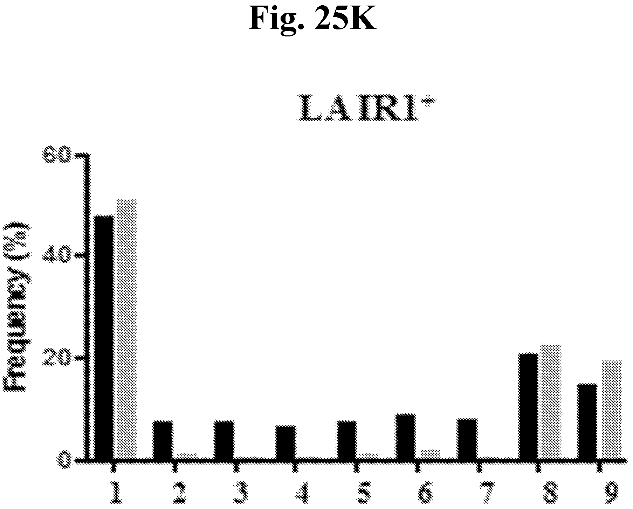

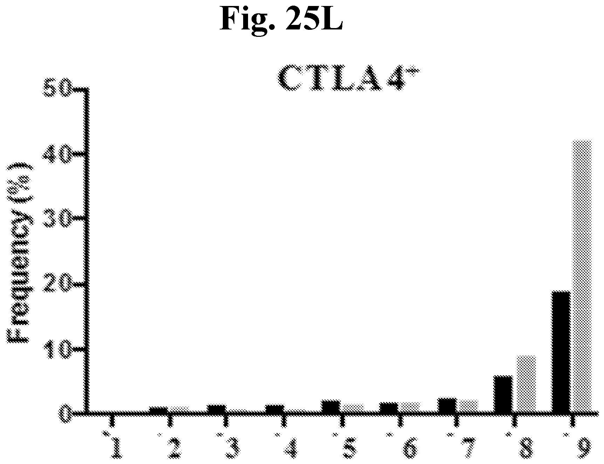

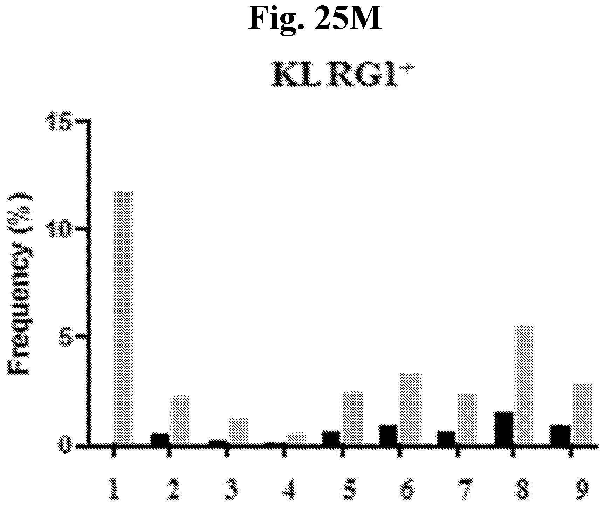

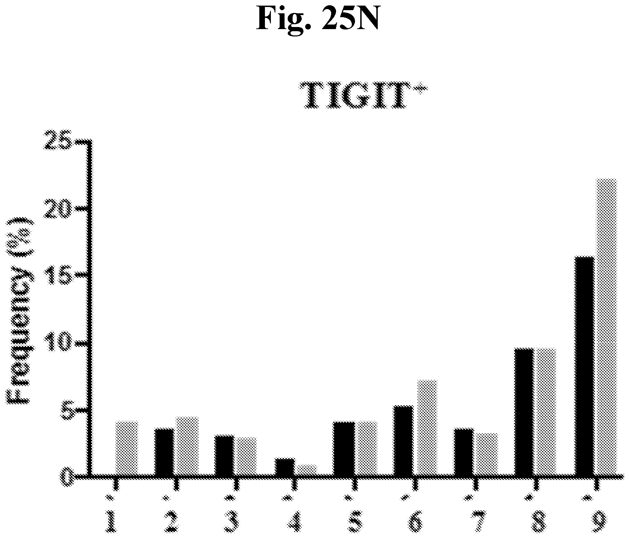

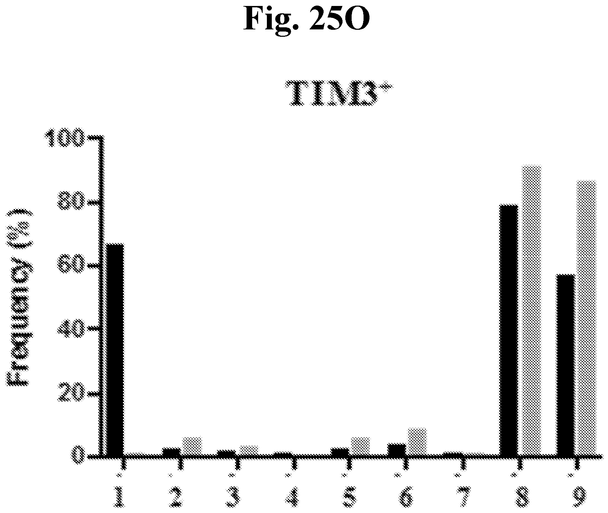

After 6 days in culture, the resultant T cells were harvested and evaluated by flow cytometry for specific molecule expression within the CD4+ (indicated in black columns) and CD8+ (indicated in gray columns) T cell subsets as shown in FIGURES 25A-25O.

FIGURE 25A (CD45RA+) demonstrates the importance of the RAPA-T culture conditions with regard to maintaining the naive T cell marker expression on both CD4+ and CD8+ T cells relative to culture input cells; in marked contrast, the previous T-RAPA condition resulted in a marked reduction in CD45RA+ cells.

FIGURE 25B (CD25+) demonstrates the importance of the RAPA-T culture conditions with respect to maintaining T cell quiescence in both CD4+ and CD8+ T cells relative to culture input cells; in marked contrast, the previous T-RAPA condition resulted in a markedly activated T cell state, as indicated by increased CD25 expression.

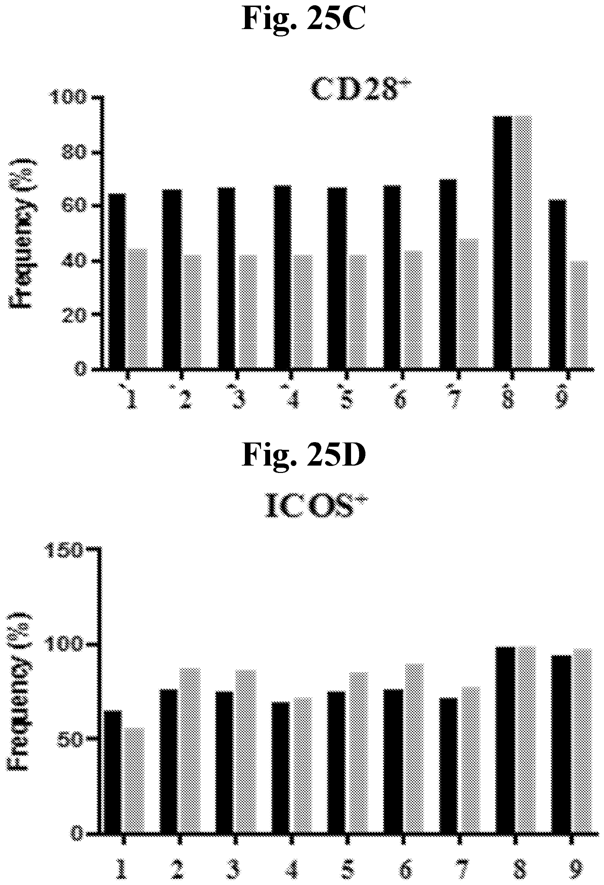

FIGURE 25C (CD28+) and FIGURE 25D (ICOS+) show that the RAPA-T and T-RAPA cell products had similar CD4+ and CD8+ T cell expression of these activating costimulatory molecules.

FIGURES 25E-25F (CD39+ and CD73+, respectively) show that the RAPA-T culture condition resulted in reduced expression of these ecto-nucleotidase molecules relative to the T-RAPA condition. These molecules exert an immune suppressive effect via metabolizing ATP into adenosine. Thus, the RAPA-T cell product is expected to be advantageous relative to the T-RAPA cell product in terms of therapeutic use.

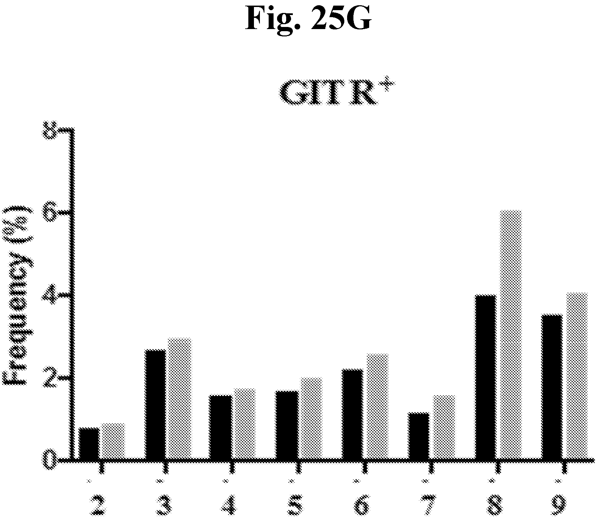

The remaining data in FIGURES 25G-25O indicate that the new RAPA-T method results in greatly reduced expression of molecules associated with immune senescence (KLRG1), associated with an immune suppressive regulatory T cell phenotype (GITR), or associated with checkpoint inhibitory function (LAG3, PD1, 2B4, LAIR1, CTLA4, TIGIT, and TIM3). Each of the variable culture conditions linked to the RAPA-T cell product were greatly reduced in each of these molecules relative to the T-RAPA cell products. Condition 7 demonstrated the most profound and consistent reduction in these molecules of the conditions tested.

### Example 3

T cells were prepared as in Example 2 with Culture Conditions 1-8 corresponding to Culture Conditions 2-9 of Example 2. At day 2 of the culture interval, the resultant T cells were harvested and evaluated by Western Blot Analysis (methods as per manufacturer's instruction; BioTechne Mr. Wes instrumentation)for molecules relevant to mTORC1, mTORC2, and STAT pathways.

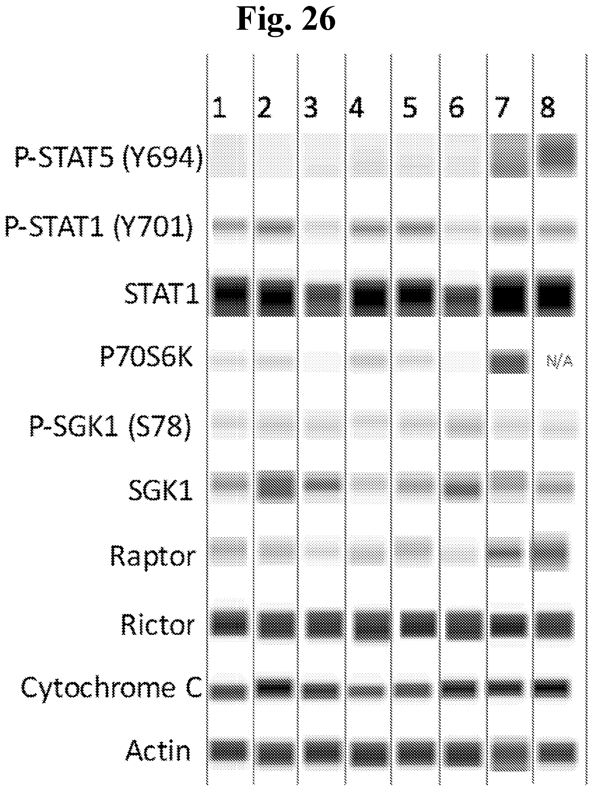

For optimal Th1/Tc1-type manufacturing, it is important to limit activation (phosphorylation) of STAT5, which can drive the regulatory T cell phenotype. As shown in Figure 26, each of the Rapa-T cell culture conditions (Conditions 1-6) were relatively devoid of phosphorylated STAT5; in marked contrast, the T-Rapa conditions showed a significant presence of phosphorylated STAT5 (Conditions 7-8). The reduction of STATS phosphorylation of the Rapa-T cells relative to the T-Rapa cells can be more than 75%.

For optimal Th1/Tc1-type manufacturing, it is important to have active signaling through specific STAT molecules that drive type I differentiation, including STAT1. As shown in Figure 26, each of the Rapa-T culture conditions showed a detectable level of STAT1 phosphorylation, albeit the level was somewhat reduced in Condition 6 (Example 2, Condition 7). However, the level of total STAT1 was also reduced in Condition 6.

The optimal phenotype of Rapa-T cells can also be characterized by a reduction in molecules associated with the mTORC1 pathway. Rapa-T Condition 6 (corresponding to Example 2, Condition 7) was essentially devoid in expression of the mTORC1-associated molecule, p70S6K. Provision of co-stimulation in other Rapa-T culture conditions (Conditions 1, 2, 4 and 5) increased p70S6K expression. Thus, during the Rapa-T manufacturing process, it may be beneficial to avoid co-stimulation.

The optimal phenotype of the Th1/Tc1-type RAPA-T cell manufacturing can also be contingent on the preservation of the mTORC2 signaling pathway. In this regard, it is beneficial that the optimal RAPA-T cell condition (Condition 6, corresponding to Example 2, Condition 7) has preservation of expression of mTORC2-associated molecules total SGK1 and phosphorylated SGK1. This characteristic of the optimal RAPA-T cell product regarding marked reduction in mTORC1 with relative preservation of mTORC2 is further exemplified by Condition 6, namely, marked reduction in the mTORC1-associated subunit molecule Raptor and relative preservation of the mTORC2-associated subunit molecule Rictor.

### Example 4

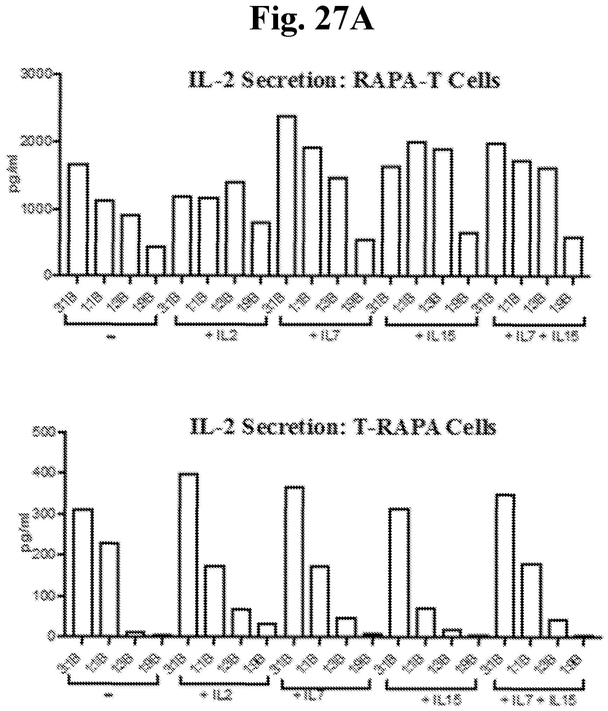

Steady-state apheresis was performed to obtain patient samples containing PBMCs. Lymphocytes in the samples were enriched by using an automated Ficoll procedure on a Sepax^{®} instrument by GE. The lymphocyte-enriched cell populations were then plated in G-REX culture vessels under two conditions, one corresponding to Condition 7 in Table 1 and one corresponding to Condition 9 in Table 1 (T-RAPA) and incubated in culture for 6 days as in Example 2. After 6 days of culture, the T cells were harvested and re-plated at a concentration of 1 × 106 cells/mL for generation of a 24-hour supernatant. At the time of re-plating, the T cells were co-stimulated with anti-CD3/anti-CD28 coated magnetic beads at bead:T cell ratios of 3:1, 1:1, 1:3 or 1:9. At each of these ratios, the 24-hour supernatant generation was performed with no cytokine addition (indicated by "-" symbol), addition of rhuIL-2 (100 IU/mL, indicated by "+IL-2"), addition of rhuIL-7 (10 ng/mL, indicated by "+IL-7"), addition of rhuIL-15 (10 ng/mL, indicated by "+IL-15"), or addition of both rhuIL-7 (10 ng/mL) and rhuIL-15 (10 ng/mL) (indicated by "+IL-7+IL-15"). IL-2 and TNF-α secretion was measured in the cells by known methods according to the manufacturer's instructions (Luminex). Results are shown in FIGURES 27A-27B.

CD4+ and CD8+ T cells at early states of differentiation, such as the naïve, central-memory, or stem central memory subsets may be beneficial for adoptive transfer. Such early differentiation T cells have been functionally characterized in part by their differential response to the key homeostatic cytokines, namely IL-7 and IL-15. As such, the ability of a given T cell product to respond to IL-7 and IL-15 is a desirable characteristic.

FIGURE 27A shows the IL-2 secretion profile of the optimal RAPA-T cell product whereas the lower panel shows the IL-2 secretion profile of the T-RAPA cell product. At maximal co-stimulation challenge and without exogenous cytokine support, the RAPA-T cell product secreted approximately a 5-fold higher amount of IL-2 relative to the T-RAPA cell product. Remarkably, even at very low levels of co-stimulation (1:9 bead:T cell ratio), the RAPA-T cell product secrets substantial IL-2; in marked contrast, this stimulation condition in the T-RAPA condition yielded undetectable levels of IL-2. IL-2 secretion capacity has been associated with the beneficial helper-independent T cell phenotype, which is a cytokine secretion characteristic observed in T cells at early states of differentiation. Finally, in the RAPA-T condition but not in the T-RAPA condition, addition of either IL-7 or IL-15 further augmented IL-2 secretion capacity. As such, the RAPA-T cells are uniquely responsive to the homeostatic cytokines IL-7 and IL-15.

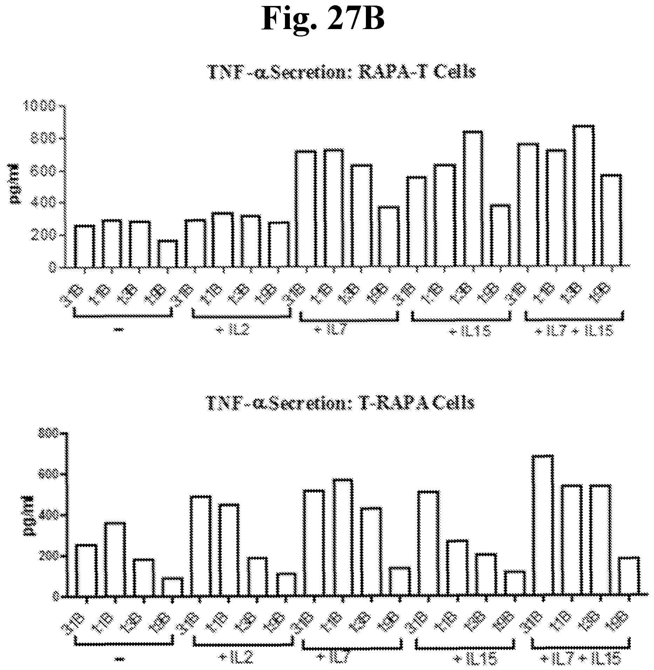

FIGURE 27B shows the TNF-α secretion profile of the optimal RAPA-T cell product whereas the lower panel shows the TNF-α secretion profile of the previous T-RAPA cell product. At maximal co-stimulation challenge and without exogenous cytokine support, the RAPA-T cell product secreted approximately equivalent TNF-α relative to the T-RAPA cell product. However, addition of IL-7 or IL-15 to the co-stimulation resulted in a higher capacity to secrete TNF-α in the RAPA-T condition relative to the T-RAPA condition. As such, the RAPA-T cells are uniquely responsive to the homeostatic cytokines IL-7 and IL-15 in terms of inducing secretion of the Th1/Tc1 effector cytokine TNF-α.

### Example 5

Human T cells were co-stimulated without any inhibitors ('Conventional"; addition of anti-CD23/anti-CD28 beads; 3:1 bead:T cell ratio). Alternatively, T cells were co-stimulated according to the RAPA-T cell conditions ("Rapamycin-Treated"), with the co-stimulation provided either by anti-CD3/anti-CD28 beads ("Dynabeads"; 1:3 bead:T cell ratio) or by Cloudz^{®} dissolvable co-stimulation micro-particles (Biot-Techne; use of Cloudz^{®} at 20% of manufacturer's recommended dose; 50 µL of Cloudz^{®} stock per 1 × 106 cells). After a 6-day culture, the T cells were harvested, adjusted to 1 × 10⁶ cells/mL, and co-stimulated using anti-CD3/anti-CD28 beads (3:1 bead:T cells ratio). The 24-hour supernatants were then collected and tested for the content of IL-2, TNF-α, and IL-13 by Luminex assay (results shown as pg/mL per 1 × 106 cells per 24 hours). Using the same protocol, the cells after 6-day culture were harvested and evaluated for flow cytometry for expression of cell surface markers, including CD4, CD8, CD25 and CTLA4.

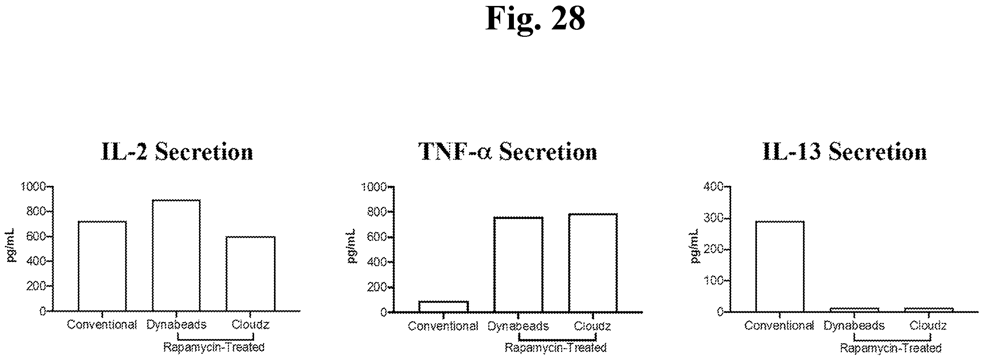

FIGURE 28 shows the IL-2, TNF-α and IL-13 secretion data. As shown in FIGURE 28, the results between RAPA-T cells co-stimulated by anti-CD3/anti-CD28 nanoparticles ("Dynabeads^{™}) and dissolvable anti-CD3/anti-CD28 microparticles (Cloudz^{®} reagent; BioTechne) are similar. These cells, as previously described, possess a Th1-type cytokine profile, as evidence by substantial secretion of IL-2 and TNF-α with minimal secretion of the Th2-type cytokine IL-13.

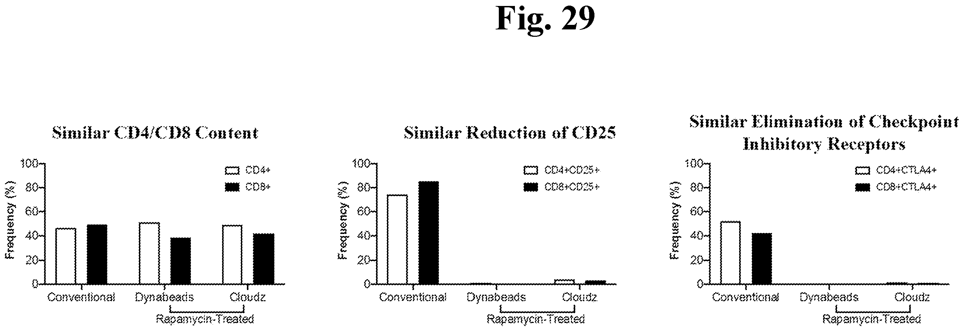

FIGURE 29 shows the frequency of cells expressing the cell surface markers, CD4, CD8, CD25 and CTLA4 as measured by flow cytometry. As shown in FIGURE 29, the results between RAPA-T cells co-stimulated by anti-CD3/anti-CD28 nanoparticles ("Dynabeads") and dissolvable anti-CD3/anti-CD28 microparticles (Cloudz^{®} reagent; Bio-Techne) are similar. These cells, as previously described, are quiescent (as indicated by reduced expression of CD25) and have reduced expression of checkpoint inhibitory receptors (as indicated by reduced expression of CTLA4)

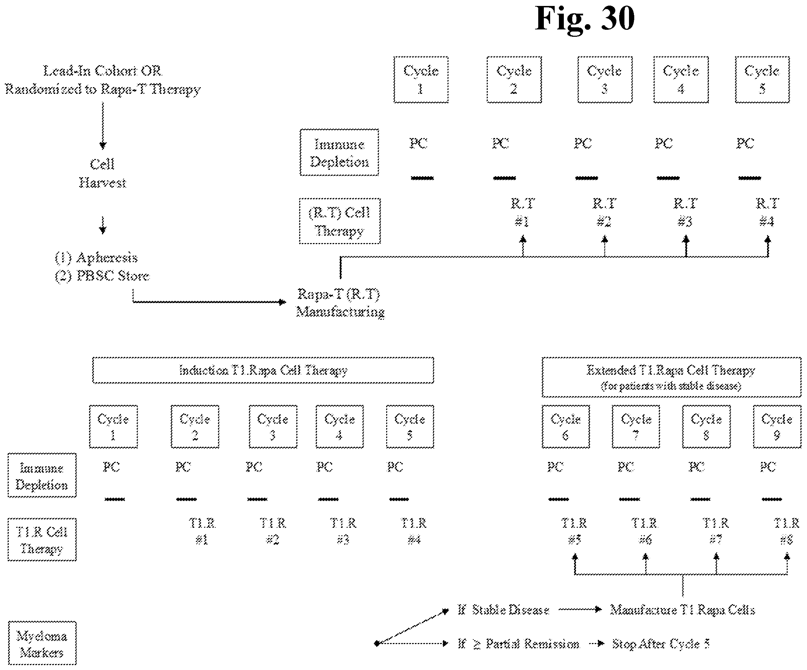

*Example 6: Phase III Randomized Clinical Trial of Rapa-T Cell Therapy* FIGURE 30 depicts the Randomized Phase 3 Protocol Schema. In the upper panel of FIGURE 30, for patients randomized to the Rapa-T therapy, autologous cells for Rapa-T cell manufacturing will be derived from a steady-state apheresis collected after randomization. The immune depleting regimen will be comprised of pentostatin and low-dose, dose-adjusted cyclophosphamide (PC regimen). The first PC cycle will be administered alone (without T cell therapy) at study entry during the interval of T1.Rapa manufacturing and will be 28 days in duration; PC cycles two, three, four, and five will be administered prior to each of the four T1.Rapa cell infusions and will be 35 days in duration. The lower panel of FIGURE 30 depicts extended T1.Rapa cell therapy for stable disease. For T1.Rapa cell recipients who have stable disease after cycle 4, an additional lot of T1.Rapa cells will be manufactured to allow up to four additional cycles of T1.Rapa cell therapy (cycles 6 through 9).

FIGURE 30 details manufactured T cell therapy, which will be administered to all patients randomized to the manufactured T cell Cohort. This therapy will involve: (1) collection of immune cells to be used as substrate for the manufactured T cell manufacturing, which will be obtained from either a previously harvested peripheral blood stem cell transplantation procedure or a fresh, steady-state apheresis procedure; (2) enrichment for mononuclear cell populations and subsequent incubation of mononuclear cells under the manufactured T cell culture conditions; (3) cryopreservation of single-use manufactured T cell therapeutic products, which undergo a verification step for identity and function; (4) patient treatment with a pentostatin plus cyclophosphamide drug regimen (PC regimen) will be initially in isolation and subsequently in combination with manufactured T cell therapy to both prepare the patient for manufactured T cell therapy and directly promote anti-tumor effects; and (5) specialized immune monitoring during and after therapy.

The immune depleting regimen will be comprised of pentostatin and low-dose, dose-adjusted cyclophosphamide (PC regimen). The first PC cycle will be administered alone (without T cell therapy) at study entry during the interval of manufactured T cell manufacturing and will be a minimum of 28 days in duration to allow blood count recovery; PC cycles two, three, four, and five will be administered prior to each of the four manufactured T cell infusions and will be a minimum of 35 days in duration to allow blood count recovery. There will be no maintenance therapy after completion of cycle 5 of manufactured T cell therapy. Treatment cycles can be longer than the indicated interval, extending to an indefinite interval, depending on the clinical situation. By way of example but not limitation, if a patient is in remission, cycles can be delayed until evidence of disease relapse. Moreover, additional maintenance cycles of the PC regimen plus adoptive manufactured T cell therapy is envisioned to maintain patients in a remission state, perhaps by administering 1 to 4 therapy cycles per year, or to treat disease relapse if it were to occur.

As indicated in FIGURE 30, for patients who have stable disease after 4 cycles of therapy, an additional manufacturing of Rapa-T cells can be performed, thereby facilitating potential therapy with additional cycles, up to 9 total Rapa-T cell therapy cycles.

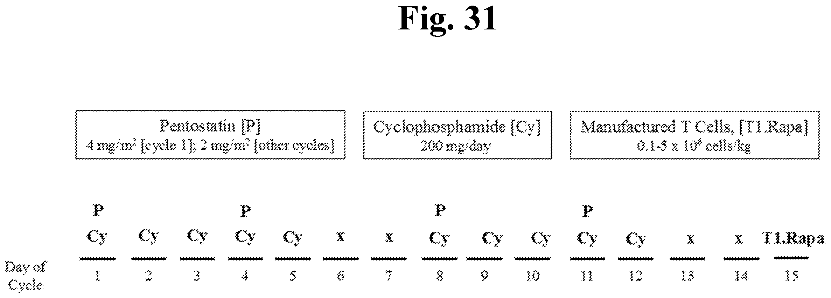

FIGURE 31 details the specifics of the PC chemotherapy regimen. Each cycle of PC therapy will consist of a 14-day course just prior to the T1.Rapa cell infusion on day 15 of the cycle (T1.Rapa dose: between 0.1 and 5 x 10⁶ cells/kg). For cycle 1, pentostatin (P) will be administered (i.v.) at a dose of 4 mg/m² on days 1, 4, 8, and 11; cyclophosphamide (Cy) will be administered at a dose of 200 mg per day on days 1 through 5 and days 8 through 12. For subsequent cycles, pentostatin will be reduced to a dose of 2 mg/m².

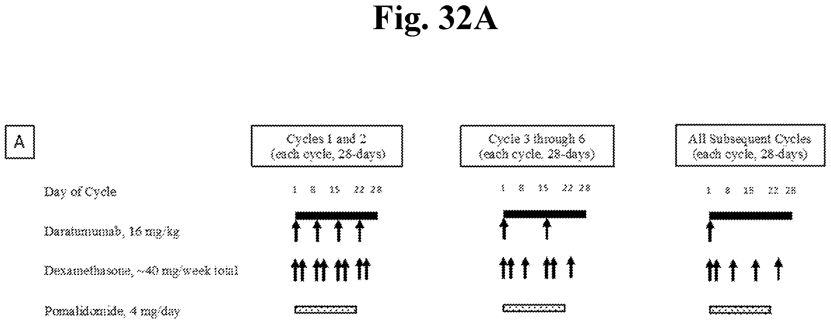

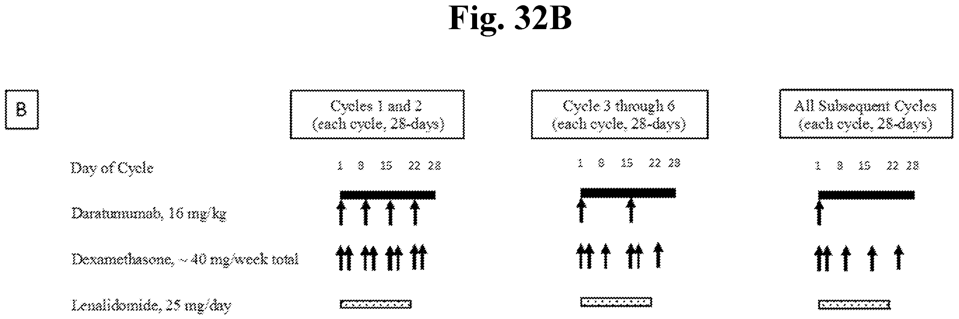

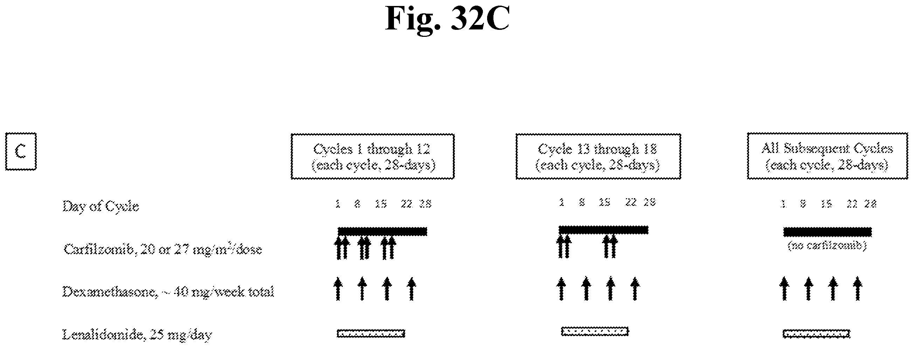

FIGURES 32A-32C detail the nature of the control arm, that is, subjects that are not randomized to the Rapa-T cell therapy will receive one of three FDA-approved triplet regimens suitable for subjects with MM in the second or third relapse, namely: the DPd regimen (FIGURE 32A); the DRd regimen (FIGURE 32B); or the KRd regimen (FIGURE 32C).

For the control cohort depicted in FIGURES 32A-32C, patients will be enrolled and subsequently randomized at the time of second or third relapse of multiple myeloma (MM). Patients must be candidates to receive one of three FDA-approved regimens for treating this patient population. Patients randomized to the control cohort will receive either the DPd, DRd, or KRd regimen using the published standard regimens. Specific aspects of these standard regimens are indicated above in: [FIGURE 32A] DPd Regimen; [FIGURE 32B] DRd Regimen; and [FIGURE 32C] KRd Regimen.

*Statistical Evaluation of Manufactured T cell Efficacy* The primary objective will be to compare the progression-free survival in recipients of the manufactured T cell therapy relative to recipients randomized to the standard-of-care therapy. By comparison, secondary objectives will be assessed in a preliminary manner using descriptive statistics. Eligible patients with MM in the second or third relapse will be randomized in a 1:1 manner to receive either standard-of-care therapy using an FDA-approved triplet regimen consisting of either DPd, DRd, or KRd or adoptive T cell therapy with ex vivo manufactured autologous, rapamycin-resistant Th1/Tc1 cells (manufactured T cell). N=65 evaluable patients will be accrued to each cohort. The primary study objective will be to determine whether patients treated on the manufactured T cell cohort have an increased progression-free-survival (PFS) relative to the patients treated on the standard-of-care cohort.

The progression-free survival (PFS) and overall survival of the patients will be estimated using the Kaplan-Meier method in both arms and presented with pointwise 95% confidence intervals. A non-parametric estimate of the median survival and its 95% confidence interval will be obtained by inverting the Kaplan-Meier estimate. The primary efficacy outcome (PFS) will be tested by a one-sided log-rank test. The final analysis will be performed when 130 PFS events have occurred in the study, or the recruitment goal has been reached and all patients have been followed for at least 12 months. Overall survival (OS) will be measured from the start of the treatment; death from any cause will be an event, and patients will be censored at the date of last contact.

Secondary endpoints will be evaluated in a descriptive manner such as mean, standard deviation, confidence interval, Kaplan-Meier analyses, or other methods of characterizing multiple myeloma remission status. Objective response rate (ORR) and minimum residual disease rate (MRD) will be estimated as the observed proportion of patients with the corresponding outcome, and presented with 95% confidence intervals.

Demographic and baseline data will be summarized in a descriptive manner. Categorical data will be presented as frequencies and percentages whereas continuous data will be presented using summary statistics such as mean, median, and standard deviation. Particular attention will be focused upon determination of prior therapies for multiple myeloma and degree of refractoriness to the individual agents utilized.

Two interim analyses with potential stopping for futility will be conducted when 28 and 55 PFS events have occurred overall (combining the two arms). A beta error-spending approach with a quadratic error-spending function will be used, which is a compromise between the O'Brien-Fleming and Pocock boundaries. The following table shows the null-hypothesis acceptance boundary for the two interim and one final analyses. These values would be modified using the error-spending function if the timing of the interim analyses is modified.

| Analysis | Number of PFS events | Expected number of patients randomized | Expected time from study start | P-value criterion for stopping for futility | P-value criterion for stopping for efficacy |
|---|---|---|---|---|---|
| 1^{st} interim | 28 | 80 | 19 months | p > 0.874 | - |
| 2^{nd} interim | 55 | 122 | 29 months | p > 0.425 | - |
| Final | 104 | 130 | 60 months | - | p < 0.026 |

The probability of stopping the trial early due to futility as a function of the true effect size is shown in the following table:

| True effect size as proportion of the design effect size | Probability of stopping for futility at 1^{st} interim analysis | Probability of stopping for futility at |
|---|---|---|
| | | 1^{st} or 2^{nd} interim analysis |
| 0 | 12.6 % | 58.3 % |
| 0.5 | 3.8 % | 22.6 % |
| 1 | 0.8 % | 4.4% |

### Collection of Immune Cells to be used as Substrate for Manufactured T Cell

### Manufacturing

Collection and shipping of cells for manufactured T cell manufacturing will be required for patients randomized to the manufactured T cell therapy cohort.

In addition, in cases where the subject has the necessary value for immune cells in the bloodstream as defined by absolute lymphocyte count (ALC; value of at least 300 lymphocytes per microliter), steady-state apheresis will be performed and will consist of a 10- to 15-liter collection. The apheresis should be performed within 10 days of study entry. The apheresis product will be immediately shipped (without cryopreservation) to Rapa Therapeutics.

The first cycle of the PC regimen should be started within 10 days after study entry.

Subsequent iterations of manufactured T cell therapy may become more efficient, and as such, smaller numbers of input cells might be used to initiate manufacturing. Such improved methods will involve at least in part improved host preparation and improved manufacturing processes. In such methods, it will be possible to manufacture T cells with starting material obtained from a simple blood draw of 500 mL or less.

### Manufactured T cell Manufacturing

In the case of previously collected cellular products, such cryopreserved cells will be stored in the vapor phase of liquid nitrogen until thawing of cells and manufacture of manufactured T cells. In the case of freshly isolated cells by apheresis or in the future by simple blood collection, the cells will undergo immediate processing, and then may either be placed directly into culture or may be cryopreserved by controlled rate freezing technology and stored in the vapor phase of liquid nitrogen for subsequent use later.

T cell culture from cryopreserved cell substrates from freshly isolated cell populations requires some type of T cell enrichment. By way of example but not limitation, T cell enrichment can be achieved by use of monoclonal antibody and column technology (positive or negative selection). Enrichment of the raw cellular material used in manufactured T cell manufacturing does not require such antibody-based methodologies because T cells are efficiently enriched during the culture interval; as such, our method is consistent with recommendations for effective cell therapy on a global level. The initial processing steps for manufacture of manufactured T cells focuses on removal of di-methyl sulfoxide (DMSO) used in the cryopreservation steps (when applicable), lysis of red blood cells (RBCs), and centrifugal removal of contaminating granulocytes and to some extent, monocytes. These steps are performed in a relatively automated method using primarily closed-system technology. This procedure is advantageous as it reduces human error, provides detailed manufacturing data for batch records, improves consistency across manufacturing runs, and reduces the risk for infectious agent contamination of the final product. Processing for the manufactured T cell products incorporates the following steps: (1) thaw of cryopreserved products (when applicable) using solid-state, non-water based methods to reduce infectious agent contamination as in Triana E, Ortega S, Azqueta C, et al. Thawing of cryopreserved hematopoietic progenitor cells from apheresis will be with using a new drywarming device. Transfusion. 2013;53(1):85-90; (2) automated washing of the cellular product using the LOVO permeable membrane device as in Mfarrej B, Bouchet G, Couquiaud J, et al. Pre-clinical assessment of the Lovo device for dimethyl sulfoxide removal and cell concentration in thawed hematopoietic progenitor cell grafts. Cytotherapy. 2017;19(12):1501-1508; (3) integration of lysis of RBCs using ammonium-chloride-potassium (ACK) buffer, described in Brown WE, Hu JC, Athanasiou KA. Ammonium-Chloride-Potassium Lysing Buffer Treatment of Fully Differentiated Cells Increases Cell Purity and Resulting Neotissue Functional Properties. Tissue engineering Part C, Methods. 2016;22(9):895-903, during the LOVO washing steps; (4) volume reduction of cellular content using the LOVO method with subsequent plating of cells into the closed-system, counter-flow, centrifugal elutriation (CCE) device (Elutra; Terumo) as previously described in Stroncek DF, Fellowes V, Pham C, et al. Counter-flow elutriation of clinical peripheral blood mononuclear cell concentrates for the production of dendritic and T cell therapies. J Transl Med. 2014;12:241.(doi):10.1186/s12967-12014-10241-y; and (5) pre-programmed operation of the Elutra device for efficient removal of granulocytes and monocytes by CCE.

After this lymphocyte enrichment and media purification, the cells are plated into specialized chambers that possess an enriched capacity for oxygen exchange (G-Rex vessels; Wilson-Wolf), described in Bajgain P, Mucharla R, Wilson J, et al. Optimizing the production of suspension cells using the G-Rex "M" series. Molecular Therapy Methods & Clinical Development. 2014;1:14015. In addition to having enhanced gas permeability characteristics, the G-Rex vessels are closed system units and have the additional advantage of automated, closed system media volume reduction (GatheRex Liquid Handling Pump). The lymphocyte-enriched cells are maintained in the G-Rex vessels for 6-days.

Several specific culture conditions are utilized to promote the manufacture of a mixture of CD4⁺ and CD8⁺ T cells in the G-Rex vessels with functional attributes of a manufactured T cell. These specific conditions include: (1) use of enriched media (including but not limited to X-Vivo 20; Lonza; mediate may also be supplemented with 5% AB serum), Zhang HD, Song ZL, Li WP. [In vitro cultivation of dendritic cells with serum-free medium]. Zhongguo shi yan xue ye xue za zhi. 2006;14(5):985-989; Lonza) that is further supplemented with 5% human serum; (2) incorporation of a 16-hour rest interval of cell plating into the G-Rex prior to co-stimulation (cells are plated at a relatively high density of 1.5 x 10⁶ T cells per ml); (3) during this initial rest interval, cells are optimally rested by addition of the monoclonal antibody basiliximab (which blocks the IL-2 receptor and thereby prevents autonomous T cell activation by endogenously produced IL-2) and temsirolimus (which is a pharmacologic inhibitor of mTORC1); (4) after this 16-hour rest interval, cells are either not co-stimulated or co-stimulated with anti-CD3/anti-CD28 coated magnetic beads (3/28 beads) under sub-optimal conditions, as defined by a bead-to-T cell ratio of 1:3 (typically, most T cell expansion conditions utilize a 9-fold higher level of co-stimulation, a 3:1 bead-to-T cell ratio); (5) importantly, it is essential that the T cells are not washed after the initial rest interval; (6) after the rest interval, in addition to addition of 3/28 beads, it is essential to add the polarizing cytokine IFN-α at a high dose (10,000 IU/ml) to promote differentiation to CD4⁺ Th1 and CD8⁺ Tc1 phenotypes; (7) importantly, it is critical to avoid the addition of IL-2, which is a common additive to T cell cultures; and (8) after addition of the beads and IFN-α, it is important to leave the cells undisturbed until harvesting at day 6 of culture (no cell washing, no further culture additives).

### Cryopreservation of Manufactured T cell Products and Verification of Identity and Function

After the 6-day cell culture in the G-Rex vessels, the volume of the culture will be reduced in a closed system manner by the GatheRex instrument. Subsequently, the cells will be harvested, 3/28 beads removed by hand-held magnet, and cells placed into the LOVO device for serial washing of the cells to remove > 99% of culture additives (Temsirolimus, Basiliximab, IFN-α).

Washed cells will be reconstituted into cryopreservation media, which contains 5% DMSO and 5% pentastarch. The cryopreservation will be performed in multiple single use aliquots in 50 ml freezer bags. The manufactured T cell dose will be between 0.1 and 5 x 10⁶ T cells per kg of recipient body weight. Four single-use aliquots will be cryopreserved to allow four consecutive, approximate monthly infusions of manufactured T cells. Manufactured T cells will be cryopreserved by GMP-compliant controlled rate freezing methodology, as previously described in Hunt CJ. Cryopreservation of Human Stem Cells for Clinical Application: A Review. Transfusion medicine and hemotherapy : offizielles Organ der Deutschen Gesellschaft fur Transfusionsmedizin und Immunhamatologie. 2011;38(2):107-123, and; cells will be shipped in the vapor phase of liquid nitrogen by certified cryo-shipper after the manufactured T cells have passed the specified release criteria testing.

Release criteria testing of the manufactured T cells will include standard tests such as content of CD3⁺, CD4⁺ and CD8⁺ T cell purity (final product must be > 70% CD3⁺ T cell content by flow cytometry; CD4⁺ and CD8⁺ subsets must each be present at the 5% level). Cells must be > 70% viable, as determined by flow cytometry annexin and 7-AAD assays. Furthermore, cells must be free of bacterial and fungal contamination upon a minimum of a 3-day culture interval (ideally, a 14-day culture interval); furthermore, the cell product must be below detection limit for bacterial LPS endotoxin and mycoplasma.

In addition to these standard tests, specialized functional tests will constitute release criteria for the manufactured T cell products. Prior to release of product and cell therapy, a manufactured T cell can possess the following attributes relative to culture input T cells: (1) an enhanced T central memory phenotype, as defined by increased flow cytometric co-expression of CD62 ligand and CCR7; (2) low level expression of checkpoint inhibitory molecules, such as programmed death-1 (PD1); (3) a resting state, as defined by reduced levels of Th1/Tc1-type cytokine secretion upon maximal co-stimulation; (4) an autophagy signature, as evidenced by reduced mitochondrial mass by flow cytometry MitoTracker assay, see Xiao B, Deng X, Zhou W, Tan EK. Flow Cytometry-Based Assessment of Mitophagy Using MitoTracker. Frontiers in cellular neuroscience. 2016;10:76; (5) a resistant phenotype, as evidenced by at least 50% inhibition of mTORC1 and mTORC2 downstream targets; and (6) a multi-faceted differential gene expression profile of n=80 key transcription factor and differentiation molecules.

### Host Preparation Using PC Regimen

Patients on the manufactured T cell treatment receive a pentostatin plus cyclophosphamide (PC) regimen. Cycle 1 of the PC regimen will be administered during the interval of manufactured T cell manufacturing and will be thus administered without accompanying T cell infusion. Cycle 1 is advantageous on two levels: first, it will reduce the number and function of host regulatory T cells and end-stage senescent effector T cells, thereby potentiating futures cycles of manufactured T cell therapy; and second, it will directly mediate anti-tumor effects against the multiple myeloma, thereby controlling disease during the manufacturing interval. After cycle #1, subsequent cycles of the PC regimen will be followed by the adoptive transfer of manufactured T cells on the day after the two-week PC regimen interval (day 15). These cycles of the PC regimen are additionally advantageous because they will further modulate the host biology, including increasing T cell homeostatic cytokines such as IL-7 and IL-15, which will allow improved manufactured T cell expansion after adoptive transfer.

The PC regimen is followed by manufactured T cell infusion. Each cycle of PC therapy will consist of a 14-day course just prior to the manufactured T cell infusion on day 15 of the cycle. The manufactured T cell dose will be between 1 and 5 x 10⁶ cells/kg, including T cell doses between 1 × 10⁵ and 5 x 10⁶ cells/kg. Pentostatin (P) will be administered (i.v.) at a dose of 4 mg/m2 on days 1, 4, 8, and 11; cyclophosphamide (Cy) will be administered at a dose of 200 mg per day on days 1 through 5 and days 8 through 12.

Premedications and prehydration will be required prior to pentostatin administration. Prehydration with 1 liter of 0.9% sodium chloride will be given 60 minutes prior to pentostatin. Premedications with an anti-emetic will be required. Anti-emetic regimen recommendations are as follows: (1) Dexamethasone, 12 mg by IV infusion 60 minutes prior to each pentostatin dose (that is, Days 1, 4, 8, and 11 of the cycle); (2) In addition, oral dexamethasone may be administered on other days on an as-needed basis at a dose of 4 mg per day; (3) Ondansetron will be administered at a dose of 8 mg by IV infusion 60 minutes prior to each dose of pentostatin; (4) For the remainder of treatment, ondansetron may be administered on an as-needed basis at an oral dose of 8 mg (tablets) every 12 hours on Days 1 through 14; and (5) Aprepitant may be added as-needed to the anti-emetic regimen in patients with uncontrolled nausea and vomiting. The pentostatin dose will be 4 mg/m²; each dose of pentostatin will be administered intravenously over 30-60 minutes.

Pentostatin will be dose modified, with dosage of pentostatin administered to a patient being between 1-4 mg/m². Pentostatin will be dose modified based on creatine clearence (CrCl), which will be obtained either by 24-hour urine or calculated by the Cockcroft-Gault formula. If a subject experiences an increase in creatinine level during the pentostatin and cyclophosphamide therapy, subsequent dosing will be modified as follows: CrCl ≥ 60 mL/min/1.73 m² → administer 4 mg/m² of pentostatin (full-dose); CrCl < 60 mL but ≥ 30 mL/min/1.73 m² → administer pentostatin at a 50% dose reduction, to 2 mg/m²; and CrCl < 30 mL → hold pentostatin. Pentostatin is rarely associated with organ toxicity such as neurologic toxicity (seizure, coma) or cardiac toxicity (decreased ejection fraction). As such, special attention should be paid towards evaluating any organ toxicity that arises during PC therapy. In the event that pentostatin is associated with any organ toxicity of grade 2 or greater severity, the institutional PI should be contacted to discuss whether further pentostatin therapy and further protocol therapy is warranted.

Oral cyclophosphamide (Cy) will be used as a component of the PC regimen, wherein the dose of cyclophosphamide will be between 50-400mg. The dose of Cy will be 200 mg daily on days 1-5 and 8-12 during cycles number one through five of the PC regimen. Cyclophosphamide 200 mg by intravenous infusion will be also permitted for tolerability issues or financial considerations. Due to cyclophosphamide bladder toxicity, adequate hydration must be maintained during the PC regimen. Patients should drink at least 2 to 4 liters of fluid per day to maintain a clear urine color.

Cyclophosphamide dosing will be adjusted, as necessary, according to the table below based on the complete blood count (CBC) and differential cell values (absolute lymphocyte count [ALC] and absolute neutrophil count [ANC]) obtained on days 1, 4, 8, and 11 of the cycle. The stated goal of the PC regimen is to attain immune depletion and immune suppression while minimizing myeloid cell suppression. To help ensure this goal, the cyclophosphamide dosing will be adjusted, as necessary, according to the following table based on the ALC and ANC values obtained on the days of pentostatin administration (that is, days 1, 4, 8, and 11 of the cycle). Notations in the table are as follows: ¹ Pentostatin will not be dose-adjusted based on ALC/ANC values; ² For ANC values < 500, in addition to decrease in cyclophosphamide dosing, patient will receive G-CSF therapy until next ANC measurement; ³ Cyclophosphamide dose indicated will be continued daily until the next ALC/ANC measurements (performed on days 1, 4, 8, and 11 of the cycle).

Variations on the PC regimen are envisioned. First, pentostatin and cyclophosphamide are synergistic in terms of their immune depletion and immune suppression actions; such synergy also likely exists in terms of anti-tumor effects, although there is little information regarding this possibility. As such, we envision that the PC regimen might be used as a standalone therapy for cancer therapy, including solid tumors; in one prior example, patients with refractory mesothelioma who received a combination regimen that was comprised in part by the PC regimen had unprecedented anti-tumor benefits. Second, because of this synergy, we propose that it will be advantageous to administer the two drugs simultaneously by intravenous infusion, preferably by mixing the pentostatin and cyclophosphamide into the same intravenous infusion bag for ease of administration and to reduce pharmacy errors. In such an application, it would be important to offer options of the PC mixtures that would encompass the various clinically-relevant pentostatin-to-cyclophosphamide ratios.

| **Cyclophosphamide Dose Adjustment Based on ALC and ANC Values** | | | | | | |
|---|---|---|---|---|---|---|
| Day of Cycle¹ | | ALC Value at time of Evaluation | | ANC Value at time of Evaluation² | | Cyclophosphamide Dose³ |
| 1 | | Any | | > 1000 | | 200 |
| | | | | | | |
| 4 | | ≥ 400 | | > 1000 | | 200 |
| | | 200-399 | | 500-999 | | 100 |
| | | < 200 | | < 500 | | 0 |
| | | | | | | |
| 8 | | ≥ 200 | | > 1000 | | 200 |
| | | 101-199 | | 500-999 | | 100 |
| | | < 100 | | < 500 | | 0 |
| | | | | | | |
| 11 | | ≥ 100 | | > 1000 | | 200 |
| | | 50-99 | | 500-999 | | 100 |
| | | < 50 | | < 500 | | 0 |
| | | | | | | |

### Manufactured T cell Infusion

Premedication will be required prior to all manufactured T cell administrations. Diphenhydramine (25 to 50 mg IV or PO) and acetaminophen (650 mg, PO) will be administered 30-60 minutes prior to manufactured T cell infusions.

Manufactured T cell infusions will occur on Day 15 of cycles two through five; however, up to a 3-day delay in manufactured T cell infusion may occur for logistic reasons. In addition, up to a 4-week delay between cycles will be allowed for logistic reasons and to allow for recovery of toxicities. The manufactured T cell dose will be 5 x 10⁶ cells/kg; however, if sub-optimal cell yield occurs during manufacturing, doses of as low as 0.1 × 10⁶ cells/kg will be allowed. The cryopreserved manufactured T cells will be thawed and immediately and rapidly administered intravenously (within 30 minutes) by gravity following the appropriate institutional SOP for blood product administration. This T cell infusion will be performed in the outpatient setting unless there is an unforeseen circumstance that requires inpatient administration. No steroids will be allowed in the management of DMSO-related toxicities (chills, muscle aches) that may occur immediately after cellular infusion unless the toxicity is deemed life threatening.

It is envisioned that smaller quantities of manufactured T cell infusion below 0. 1 × 10⁶ cells/kg may also be clinically-relevant (by way of example but not limitation, one-log lower, at 1 × 10⁵ cells/kg). First, manufacturing will be optimized to yield manufactured T cells that mediate further heightened in vivo effects, thereby reducing the required T cell dose; this will be advantageous in part because T cell collection can occur by simple blood draw and in part because of an improved manufacturing feasibility. Second, with further improvements in the PC regimen, as detailed above, the adoptively transferred manufactured T cells will have a further improved in vivo selective advantage relative to host cells, thereby effectively reducing the required manufactured T cell dose.

### Specialized Immune Monitoring During and After Therapy

Peripheral blood mononuclear cells and bone marrow cells will be sent to Rapa Therapeutics such that immune monitoring tests can be performed; the purpose of these tests will be to explore the mechanism of action of the manufactured T cell therapy and to develop biomarkers that will predict manufactured T cell efficacy. In one effort, we will evaluate manufactured T cell recipients for their capacity to produce various Th1- and Th2-type cytokines in response to various stimulations, including autologous multiple myeloma tumor cells or known or suspected tumor antigens, such as molecules in the cancer-testis-antigen (CTA) family. The CTA family of genes is extensive in number, and has been shown to be relevant in multiple myeloma; because the sequences of CTA genes are known and the relevance of specific CTA genes has been characterized in multiple myeloma, manufactured T cell therapy can be demonstrated to specifically induce T cell-mediated immunity against a range of CTA antigens. Measurement of such cytokine responses can be performed using RNA expression analysis, secretion analysis by ELISA or Luminex multi-analyte method, flow cytometry, or ELISPOT assay. Antigen-specific immunity can also be quantified by use of antibody production assays or cytolytic assays.

We will evaluate whether T cells obtained after manufactured T cell therapy have increased reactivity to autologous multiple myeloma cells relative to T cells obtained before manufactured T cell therapy. One obstacle to this effort is that propagation of patient-specific multiple myeloma cell lines is typically not successful. To overcome this obstacle, we will propagate myeloma cells using: specialized vessels, as in Zhang W, Gu Y, Sun Q, et al. Ex Vivo Maintenance of Primary Human Multiple Myeloma Cells through the Optimization of the Osteoblastic Niche. PLoS One. 2015;10(5), and media supplemented with combinations of factors known to enhance multiple myeloma proliferation and survival, including IL-6, CD40 ligand, and acquisition of resistance to carfilzomib. Patient-specific multiple myeloma cells can be used alone as stimulators in the assessment of immune T cell anti-tumor reactivity; alternatively, such tumor cells can be rendered into the apoptotic state, with the contents loaded into professional antigen-presenting-cells, which can be manufactured from patient specific monocytes collected from the elutriation procedure during manufactured T cell manufacturing.

In addition, we envision that T cell receptor (TCR) immune repertoire analysis will be useful as a biomarker for manufactured T cell therapy. Preferably, such repertoire analysis will be performed by RNA sequencing rather than the more commonly employed DNA sequencing. Unlike the majority of T cell therapies, which are targeted, manufactured T cell therapy is a polyclonal method as the manufacturing process does not preferentially shift T cell reactivity towards any particular tumor antigen. As such, any beneficial anti-tumor effect after manufactured T cell therapy is expected to be derived from in vivo clonal expansion to a diversity of tumor antigens; given this biology, successful manufactured T cell therapy will result in a differential TCR repertoire when comparing the patient pre-treatment repertoire to the posttreatment repertoire. In other cancer therapy settings, such as monoclonal antibody therapy to disengage checkpoint inhibition, successful therapy is associated with emergence of new TCR clonal specificities, known as skewing of the TCR repertoire that can be ascertained by quantification of the Morisito Index, Robert L, Harview C, Emerson R, et al. Distinct immunological mechanisms of CTLA-4 and PD-1 blockade revealed by analyzing TCR usage in blood lymphocytes. Oncoimmunology. 2014;3:e29244. In a similar manner, with successful manufactured T cell therapy, there will be skewing of the TCR repertoire; persistence of the TCR skewing beyond the interval of manufactured T cell therapy will be consistent with long-term T cell immunity to malignancy. With manufacturing advances, improved forms of manufactured T cells will be generated; in such a case, the TCR skewing will be more extensive, will occur at reduced numbers of treatment cycles, and will be more durable in the post-therapy interval.

### Protocol Inclusion Criteria for Therapy of Multiple Myeloma

Male or female patients ≥ 18 years of age are potentially eligible for manufactured T cell therapy. There will be no formal upper age limit. However, for patients over the age of 65 years who have a history of cardio-vascular pathology or symptoms (even if not clearly fitting the exclusion criteria detailed below), there should be evaluation by a cardiologist at the multicenter site. Such a subject will then be considered on a case-by-case basis. Overall patient performance status should be at least of moderately good health, as quantified by an ECOG Performance Status of ≤ 2.

Patients must have a confirmed diagnosis of multiple myeloma by histology or cytology studies. In addition, the disease must be symptomatic and the patient must be in the second or third relapse of disease after having received drugs from the following classes of agents: proteasome inhibitors, immune modulatory drugs, alkylators, CD38 monoclonal antibodies, and glucocorticoids.

Patients in the second or third relapse of disease are in a relatively advanced stage of disease. However, with demonstration of the safety and efficacy of manufactured T cell therapy, we envision that multiple myeloma patients earlier in the treatment algorithm will benefit from manufactured T cell therapy. By way of example but not limitation, manufactured T cell therapy may be used as an alternative to high-dose chemotherapy combined with autologous hematopoietic cell transplantation or may be used for the substantial number of patients who are transplant ineligible. Furthermore, manufactured T cell therapy can be envisioned at the earliest point in multiple myeloma progression prior to clinical symptoms, that is, during early detection at the phase of smoldering disease.

On the other hand, it is envisioned that the Rapa-T cell therapy described here will be applicable to the treatment of patients in more advanced stages of multiple myeloma and in patients with highly refractory disease. Specifically, Rapa-T cell therapy can be utilized to treat penta-refractory MM, which is defined as patients who have relapsed MM and resistance to five of the top drugs used to treat MM, namely: lenalidomide, pomalidomide, bortezomib, carfilzomib, and daratumumab.

Because patients with penta-refractory MM do not have a standard-of-care option for therapy, the clinical protocol to assess Rapa-T cell therapy in this setting will be a single-arm phase II study similar to that previously performed in Chen C, Siegel D, Gutierrez M, et al. Safety and efficacy of selinexor in relapsed or refractory multiple myeloma and Waldenstrom macroglobulinemia. Blood. 2018;131(8):855-863 to evaluate a novel anti-cancer drug. For this phase II study, the Rapa-T cell therapy will be administered, as described in FIGURES 30, 31, and 32A-32C. The statistical goal of the study will be to determine whether the Rapa-T cell therapy can induce a significant rate of at least partial remission of the penta-refractory MM, as defined by a rate that is at least consistent with 30%.

Must have a potential source of autologous T cells potentially sufficient to manufacture manufactured T cells. Specifically, the patient must have either a sufficient number of previously cryopreserved PBSC units available for manufacturing (defined by total CD34⁺ content of > 2 million cells/kg) or a sufficient number of circulating T cells that can be harvested by steady-state apheresis (defined by an ALC of greater than 300 cells per microliter).

Patients must be at least two weeks from myeloma therapy, major surgery, radiation therapy, participation in other investigational trials and have recovered from clinically significant toxicities of these prior treatments (resolution of CTCAE toxicity to value of 2 or less). Cardiac ejection fraction (EF) by MUGA or 2-D echocardiogram must be within institution normal limits, with an EF level of at least 40%. Kidney function as measured by serum creatinine must be less than or equal to 2.5 mg/dl. Liver function must be adequate, as measured by AST and ALT less than or equal to 3 x upper limit of normal and Total Bilirubin less than or equal to 1.5 (except if due to Gilbert's disease). Lung function must be adequate, as defined by corrected DLCO greater than or equal to 50% of expected on Pulmonary Function Tests. There must be no history of abnormal bleeding tendency. Voluntary written consent must be given before performance of any study related procedure not part of standard medical care, with the understanding that consent may be withdrawn by the patient at any time without prejudice to future medical care.

### Randomized Phase III Trial: Standard of Care Therapy

To prove the benefit of the manufactured T cell therapy, a randomized study will be performed to formally compare the results of manufactured T cell therapy with standard-of-care therapy, which will consist of either the DPd, DRd, or KRd regimens; the regimens will be administered as prescribed in the literature, according to their FDA approved status for MM patients in the second or third relapse.

### Example 7

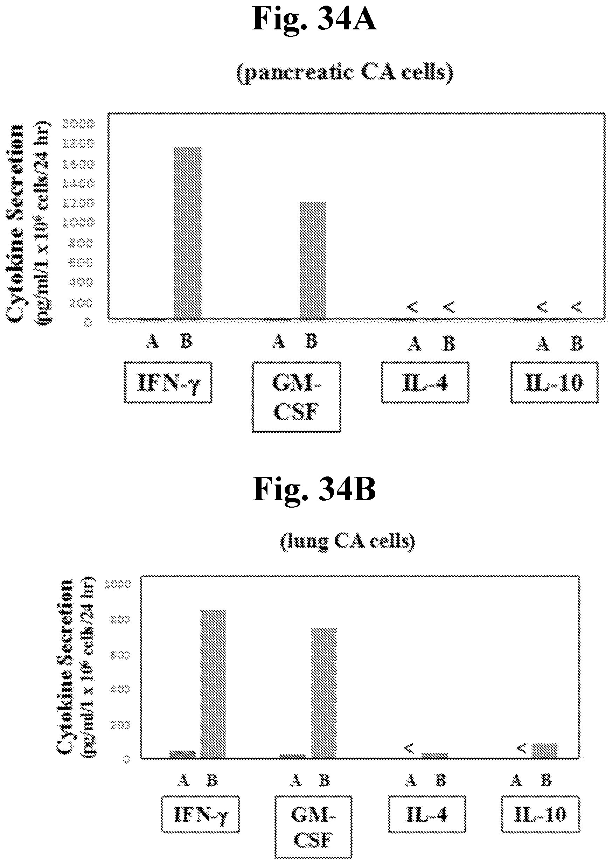

Steady-state apheresis was performed to obtain patient samples containing PBMCs Lymphocytes in the samples were enriched by using an automated Ficoll procedure on a Sepax^{®} instrument by GE The lymphocyte-enriched cell populations were then plated in G-REX culture vessels and cultured for 6 days in TexMACS media (without serum supplementation; no IL-2 supplementation) and containing IFN-α, temsirolimus and basiliximab. At the end of manufacturing, the resulting Th1/Tc1 cells were exposed to either pancreatic cancer cells (MIA-Paca2 cell line) or lung cancer cells (H23 cell line) that were rendered apoptotic by exposure to etoposide. Pulsing with this tumor lysate was followed by 7 days in culture in IL-2 (200 IU/mL), at which time a secondary exposure to tumor lysate was performed. After an additional 7 day culture interval in IL-2 containing media, a tertiary tumor lysate exposure was performed and the resultant 24-hour supernatant was tested for cytokine content by Luminex assay (results shown are in pg/mL per 1 × 10⁶ cells per 24 hours). Results for the cytokine assay are shown in FIGURES 34A-B. Condition A indicates pulsing with a sub-optimal formulation of the tumor lysate; condition B represents the optimal formulation of the tumor lysate. "<" indicates that the value was below the detection limit.

As shown in FIGURES 34A-B, RAPA-T cells can be further characterized by their capacity to respond to tumor cells, including solid tumors such as pancreatic cancer cells and lung cancer cells. As further shown in FIGURES 34A-B, the RAPA-T cells can maintain the characteristic Th1 cytokine phenotypes, as evidenced by high level secretion of IFN-γ and GM-CSF, and a reduced secretion of Th2 cytokines IL-4 and IL-10.

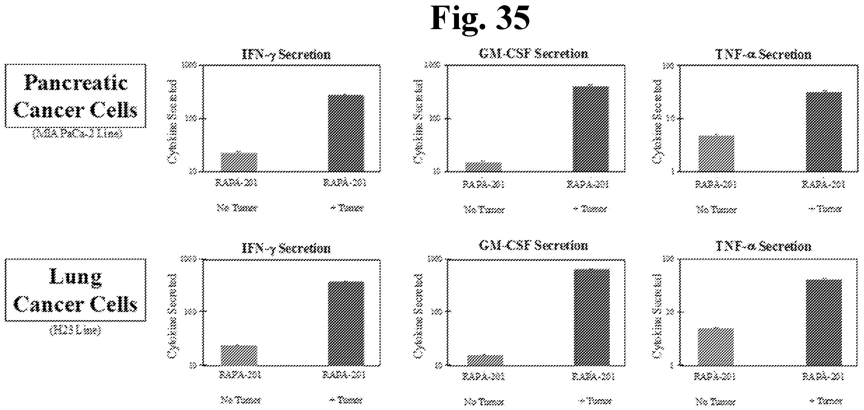

In another experiment, at the end of manufacturing, the resulting Th1/Tc1 cells were exposed to either pancreatic cancer cells (MIA-Paca2 cell line) or lung cancer cells (H23 cell line) that were rendered apoptotic by exposure to etoposide. Pulsing with this tumor lysate was followed by 7 days in culture in IL-7 (20 ng/mL) and IL-15 (10 ng/mL), at which time a secondary exposure to tumor lysate was performed. After an additional 7 day culture interval in IL-7 and IL-15 containing media, a tertiary tumor lysate exposure was performed and the resultant 24-hour supernatant was tested for cytokine content by Luminex assay (results shown are in pg/mL per 1 × 10⁶ cells per 24 hours). A control culture ("RAPA-201, No Tumor") consisted of the manufactured resistant Th1/Tc1 cells that were propagated in IL-7 and IL-15 containing media but did not receive pulsing with the tumor lysate. Results for the cytokine assay are shown in FIGURE 35.

As shown in FIGURE 35, RAPA-T cells can be further characterized by their capacity to respond to tumor cells, including solid tumors such as pancreatic cancer cells and lung cancer cells. This in vitro sensitization to solid tumor cell lines can be readily demonstrated by culture expansion in media supplemented with IL-7 and IL-15, which are two homeostatic cytokines that have been shown to selectively drive the effector function of RAPA-T cells. RAPA-T cell cytokine seretion to the tumor cells can maintain the characteristic Th1 cytokine phenotype, as evidenced by high level secretion of IFN-γ, GM-CSF and TNF-α.

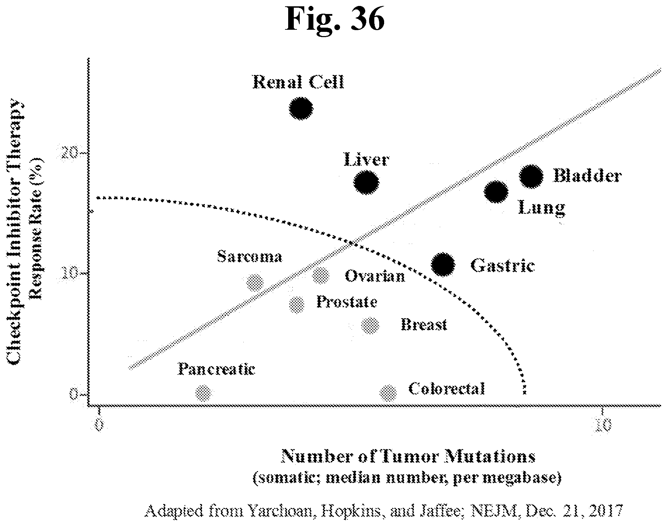

As shown in FIGURE 36, numerous cancers, such as renal cell carcinoma, liver cancer, lung cancer, bladder cancer and gastric cancer have been shown to be responsive to checkpoint inhibitor therapy such as monoclonal therapy against checkpoint inhibitor molecules such as PD-1 and CTLA4 which can induce remission in solid tumors. In a further experiment, under a Simon's 2-stage Design, patients (n = 7) with renal cancer, lung cancer, liver cancer, gastric cancer, bladder cancer and low mutation PDL1-negative or low mutation rate cancers will be administered manufactured T cell therapy. If any cohort has at least one responsive patient, the cohort will be expanded to a 20 patient cohort.

Without being bound to theory, it is expected that the Rapa-T cells can provide therapeutic benefit in cancer because the cells have reduced or no checkpoint inhibitor receptors. It is suspected that certain cancers may be non-responsive to certain treatment because of checkpoint inhibitor receptors other than PD1 and CTLA4. Thus, it is expected, without being bound to theory that the Rapa-T cells, due to the lack of additional checkpoint inhibitor receptors may be effective in treating other cancers.

### Example 8

Rapa-T cells were manufactured by a 6-day culture, with the post-Ficoll cell population cultured in media containing temsirolimus (2 µM) and the anti-IL-2 receptor monoclonal antibody, basiliximab (30 µg/mL) . After 24 hours, the culture was supplemented with IFN-α (20,000 IU/mL) there was no IL-2 supplementation of culture. There was no form of anti-CD3/anti-CD28 co-stimulation used in the culture.

In contrast, for the "control": the post-Ficoll cell population was cultured in media without temsirolimus and without basiliximab. The cells were co-stimulated on the day of culture initiation with anti-CD3/anti-CD28 coated beads at a bead-to-T cell ratio of 3:1. The media was supplemented on the day of culture initiation with IL-2 (20 IU/mL) and IFN-α (20,000 IU/mL). Mean fluorescence intensity (MFI) for BTLA, CTLA4, PD1 and TIM3 was measured by flow cytometry for the CD4+ and CD8+ T cells subsets for the culture input population, the Rapa-T cells and the control cells. Data are shown in Table 2 below. A reduction in checkpoint inhibitor receptor expression was seen as between the Rapa-T and control cells with the MFI for each checkpoint being approximately the same for the Rapa-T cells and the culture input cells.

**Table 2: Mean Fluorescence Intensities**

| **Checkpoint** | **Day 0 Culture Input** | **Day 6 Rapa-T** | **Day 6 T-Rapa** | **Percent Reduction (Rapa-T v. T-Rapa)** |
|---|---|---|---|---|
| BTLA | CD4⁺ | 3.59 | 3.70 | 92% |
| | CD8⁺ | 3.02 | 3.89 | 79% |
| CTLA4 | CD4⁺ | 3.13 | 2.93 | 100% |
| | CD8⁺ | 3.10 | 3.04 | 100% |
| PD1 | CD4⁺ | 2.92 | 3.10 | 90% |
| | CD8⁺ | 3.13 | 3.03 | 100% |
| TIM3 | CD4⁺ | 3.80 | 3.57 | 100% |
| | CD8⁺ | 3.03 | 3.10 | 96% |

## Claims

1. A method for producing manufactured T cells, comprising:
inoculating a culture input population of cells comprising T cells from a subject in a culture medium comprising temsirolimus and an IL-2 signaling inhibitor, wherein said culture medium does not contain IL-2 and no IL-2 is added to said culture medium and wherein
said temsirolimus is present in said culture medium at a concentration of at least 1 µM, and wherein said IL-2 signaling inhibitor is an anti-IL-2 receptor antibody or fragment thereof;
adding IFN-α to said culture medium;
incubating said T cells and culture medium to yield manufactured T cells; and
harvesting said manufactured T cells.

2. The method of claim 1, wherein the culture input population of cells comprising T cells are inoculated at a cell density of at least 1.5 x 10⁶ cells per mL.

3. The method of any of the preceding claims, wherein said temsirolimus is present in said culture medium at a concentration of about 4.5 µM.

4. The method of any of the preceding claims, wherein said IL-2 signaling inhibitor is basiliximab or daclizumab.

5. The method of any of the preceding claims, wherein said IL-2 signaling inhibitor is present in said culture medium at a concentration of 5 to 50 µg/mL.

6. The method of any of the preceding claims, wherein said IFN-α is added to said culture medium at a concentration of 1,000 to 10,000 IU/mL.

7. The method of any of the preceding claims, wherein said T cells and culture medium are incubated for a period of 4 days to about 8 days, preferably wherein said T cells and culture medium are incubated for a period of 6 days.

8. The method of any of the preceding claims, wherein said culture medium further comprises 5% human serum.

9. The method of any of the preceding claims, wherein said culture input population of cells comprises no more than 66% T cells out of the total number of cells in the culture input population of cells.

10. The method of any of the preceding claims, wherein said culture input population of cells comprises about 50% to about 95% T cells out of the total number of cells in the culture input population of cells.

11. The method of any of the preceding claims, wherein said culture input population of cells further comprises monocytes.

12. The method of claim 1, wherein no anti-CD3/anti-CD28 co-stimulation is performed.

## Patentansprüche

1. Verfahren zur Produktion hergestellter T-Zellen, umfassend:
Beimpfen Kultur-Eingangspopulation von Zellen, die T-Zellen von einem Patienten umfasst, in ein Kulturmedium, das Temsirolimus, und einen IL-2-Signalinhibitor umfasst, wobei das Kulturmedium kein IL-2 enthält und dem Kulturmedium kein IL-2 hinzugefügt wird und wobei
Temsirolimus in dem Kulturmedium in einer Konzentration von mindestens 1 µM vorhanden ist und wobei der IL-2-Signalinhibitor ein Anti-IL-2-Rezeptor-Antikörper oder ein Fragment davon ist;
Hinzufügen von IFN-α zu dem Kulturmedium;
Inkubation der T-Zellen und des Kulturmediums, um hergestellte T-Zellen zu gewinnen; und
Ernten der hergestellten T-Zellen.

2. Verfahren nach Anspruch 1, wobei die Kultur-Eingangspopulation von Zellen, die T-Zellen umfassen, mit einer Zelldichte von mindestens 1,5 x 10⁶ Zellen pro ml beimpft wird.

3. Verfahren nach einem der vorstehenden Ansprüche, wobei Temsirolimus in dem Kulturmedium in einer Konzentration von etwa 4,5 µM vorhanden ist.

4. Verfahren nach einem der vorstehenden Ansprüche, wobei der IL-2-Signalinhibitor aus Basiliximab oder Daclizumab besteht.

5. Verfahren nach einem der vorstehenden Ansprüche, wobei der IL-2-Signalinhibitor in dem Kulturmedium in einer Konzentration von 5 bis 50 µg/ml vorhanden ist.

6. Verfahren nach einem der vorstehenden Ansprüche, wobei das IFN-α dem Kulturmedium in einer Konzentration von 1.000 bis 10.000 IU/ml hinzugefügt wird.

7. Verfahren nach einem der vorstehenden Ansprüche, wobei die T-Zellen und das Kulturmedium für einen Zeitraum von 4 bis etwa 8 Tagen inkubiert werden, bevorzugt wobei die T-Zellen und das Kulturmedium für einen Zeitraum von 6 Tagen inkubiert werden.

8. Verfahren nach einem der vorstehenden Ansprüche, wobei das Kulturmedium weiter 5 % menschliches Serum umfasst.

9. Verfahren nach einem der vorstehenden Ansprüche, wobei die Kultur-Eingangspopulation von Zellen nicht mehr als 66 % T-Zellen aus der Gesamtzahl der Zellen in der Kultur-Eingangspopulation von Zellen umfasst.

10. Verfahren nach einem der vorstehenden Ansprüche, wobei die Kultur-Eingangspopulation von Zellen etwa 50 % bis etwa 95 % T-Zellen aus der Gesamtzahl von Zellen in der Kultur-Eingangspopulation von Zellen umfasst.

11. Verfahren nach einem der vorstehenden Ansprüche, wobei die Kultur-Eingangspopulation von Zellen weiter Monozyten umfasst.

12. Verfahren nach Anspruch 1, wobei keine Co-Stimulation von Anti-CD3/Anti-CD28 durchgeführt wird.

## Revendications

1. Méthode de production de lymphocytes T fabriqués, comprenant :
une inoculation d'une population d'intrant de culture formée de cellules comprenant des lymphocytes T provenant d'un sujet dans un milieu de culture comprenant du temsirolimus et un inhibiteur de signalisation IL-2, dans laquelle ledit milieu de culture ne contient pas d'IL-2 et aucune IL-2 n'est ajoutée audit milieu de culture et dans laquelle
ledit temsirolimus est présent dans ledit milieu de culture à une concentration d'au moins 1 µM, et dans laquelle ledit inhibiteur de signalisation IL-2 est un anticorps anti-récepteur IL-2 ou un fragment de celui-ci ;
un ajout d'IFN-α audit milieu de culture ;
une incubation desdits lymphocytes T et milieu de culture pour donner des lymphocytes T fabriqués ; et
une récolte desdits lymphocytes T fabriqués.

2. Méthode selon la revendication 1, dans laquelle la population d'intrant de culture formée de cellules comprenant des lymphocytes T est inoculée à une densité cellulaire d'au moins 1,5 x 10⁶ cellules par mL.

3. Méthode selon l'une quelconque des revendications précédentes, dans laquelle ledit temsirolimus est présent dans ledit milieu de culture à une concentration d'environ 4,5 µM.

4. Méthode selon l'une quelconque des revendications précédentes, dans laquelle ledit inhibiteur de signalisation IL-2 est basiliximab ou daclizumab.

5. Méthode selon l'une quelconque des revendications précédentes, dans laquelle ledit inhibiteur de signalisation IL-2 est présent dans ledit milieu de culture à une concentration de 5 à 50 µg/mL.

6. Méthode selon l'une quelconque des revendications précédentes, dans laquelle ledit IFN-α est ajouté audit milieu de culture à une concentration de 1000 à 10 000 Ul/mL.

7. Méthode selon l'une quelconque des revendications précédentes, dans laquelle lesdits lymphocytes T et milieu de culture sont incubés pendant une période de 4 jours à environ 8 jours, de préférence dans laquelle lesdits lymphocytes T et milieu de culture sont incubés pendant une période de 6 jours.

8. Méthode selon l'une quelconque des revendications précédentes, dans laquelle ledit milieu de culture comprend en outre un sérum humain à 5 %.

9. Méthode selon l'une quelconque des revendications précédentes, dans laquelle ladite population d'intrant de culture formée de cellules ne comprend pas plus de 66 % de lymphocytes T sur le nombre total de cellules dans la population d'intrant de culture formée de cellules.

10. Méthode selon l'une quelconque des revendications précédentes, dans laquelle ladite population d'intrant de culture formée de cellules comprend environ 50 % à environ 95 % de lymphocytes T sur le nombre total de cellules dans la population d'intrant de culture formée de cellules.

11. Méthode selon l'une quelconque des revendications précédentes, dans laquelle ladite population d'intrant de culture formée de cellules comprend en outre des monocytes.

12. Méthode selon la revendication 1, dans laquelle aucune co-stimulation anti-CD3/anti-CD28 n'est effectuée.
